# EUROPÄISCHE PATENTSCHRIFT

(11) **EP 1 446 375 B1**
(45) Veröffentlichungstag und Bekanntmachung des Hinweises auf die Patenterteilung: **14.05.2008**
(21) Anmeldenummer: 02787515.2
(22) Anmeldetag: 26.10.2002
(51) Int. Cl.: C07C 43/225, C07C 205/34, C07C 255/54, C07D 213/643, C07D 231/20, C07D 239/34, A01N 39/00, A01N 37/34, A01N 43/40, A01N 43/50, A01N 43/56

(54) **DIHALOGENPROPEN-VERBINDUNGEN, VERFAHREN ZU IHRER HERSTELLUNG, SIE ENTHALTENDE MITTEL UND IHRE VERWENDUNG ALS SCHAEDLINGSBEKAEMPFUNGSMITTEL**
DIHALOGENPROPENE COMPOUNDS, METHOD FOR THE PRODUCTION THEREOF, AGENTS CONTAINING SAID COMPOUNDS AND THE USE THEREOF AS PEST CONTROL AGENTS
COMPOSES DIHALOGENOPROPENE, LEUR PROCEDE DE PRODUCTION, AGENTS CONTENANT LESDITS COMPOSES ET LEUR UTILISATION COMME PARASITICIDES

(30) Priorität: 10.11.2001 DE 10155385
(43) Veröffentlichungstag der Anmeldung: 18.08.2004
(73) Patentinhaber: Bayer CropScience S.A., 69009 Lyon (FR)
(72) Erfinder: TIEBES, Jörg, 60431 Frankfurt (DE); BRAUN, Ralf, 76857 Ramberg (DE); DICKHAUT, Joachim, 69121 Heidelberg (DE); JAKOBI, Harald, 60598 Frankfurt (DE); LINDELL, Stephen, 65779 Kelkheim-Fischbach (DE); SALGADO, Vincent, L., 51371 Leverkusen (DE); Auler, Eva, 65812 Bad Soden (DE); JANS, Daniela, 61348 Bad Homburg v. d. H. (DE); WAIBEL, Jutta, Maria, 60529 Frankfurt (DE); HEMPEL, Waltraud, 65835 Liederbach (DE); WILHELM, Ronald, 65719 Hofheim, Ts. (DE)
(74) Vertreter: Gambert, Ulrike
(86) Internationale Anmeldenummer: PCT/EP2002/011980
(87) Internationale Veröffentlichungsnummer: WO 2003/042147

(56) Entgegenhaltungen:
- EP-A- 0 787 710
- US-A- 5 922 880
- US-A- 6 140 274

## Beschreibung

Die Erfindung betrifft Dihalogenpropen-Verbindungen, Verfahren zu ihrer Herstellung, sie enthaltende Mittel sowie ihre Verwendung zur Bekämpfung von tierischen Schädlingen, insbesondere Arthropoden, wie Insekten und Acarina, und Helminthen.

Wegen des enormen Schadens, den Insekten beispielsweise durch Fraß an Nutzpflanzen, Lebensmittelvorräten, Holz und Textilien oder auch durch Krankheitsübertragung auf Mensch, Haustiere und Nutzpflanzen verursachen, ist die Verwendung von Insektiziden oder Repellentien nach wie vor unverzichtbar. Insektizide sind ein wichtiger Bestandteil der integrierten Schädlingskontrolle und tragen entscheidend zu Ernteertrag und Kontinuität der Ernten in aller Welt bei.

Die insektizide bzw. akarizide Wirkung von Verbindungen aus der Klasse der Dihalogenpropen-Verbindungen ist an sich bekannt.

Verbindungen aus dieser Substanzklasse und deren insektizide bzw. akarizide Wirkung werden in den EP-A-787,710, WO-A-96/11,909, WO-A-97/27,173 und WO-A-97/28,112 beschrieben.

Da sich aber die ökologischen und ökonomischen Anforderungen an moderne Insektizide laufend erhöhen, beispielsweise was Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Insektizide zu entwickeln, die zumindest in Teilbereichen Vorteile gegenüber den bekannten aufweisen.

Es wurde gefunden, daß Verbindungen der allgemeinen Formel (I) ein gutes Wirkungsspektrum gegenüber tierischen Schädlingen bei gleichzeitig guter Pflanzenverträglichkeit und günstigen toxikologischen Eigenschaften gegenüber Säugetieren und aquatischen Lebewesen aufweisen.

Gegenstand der Erfindung sind daher Verbindungen der Formel (I), 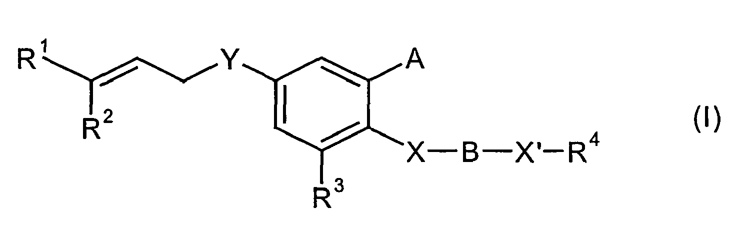 worin
R¹ und R² unabhängig voneinander Halogen bedeuten,
Y -O-, -S- oder -NH- ist,
X -O-, -S(O)ᵣ- oder -NR⁵- ist, worin r = 0, 1 oder 2 und R⁵ Wasserstoff oder C₁-C₈Alkyl bedeuten,
X' eine direkte C-C-Bindung, -O-, -S(O)ᵣ- oder -NR⁵- sind, worin r und R⁵ die oben definierte Bedeutung besitzen,
R³ Wasserstoff, Halogen, Nitro, Cyano, C₁-C₈Alkyl, C₁-C₈Haloalkyl, C₁-C₈Alkoxy, C₁-C₈Hatoalkoxy oder C₃-C₁₀Cycloalkyl ist oder einer der für A definierten Bedeutungen besitzt,
A eine der Gruppen -OR⁶, -SR⁶, -NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -C(=Z)-O-N=C(NH₂)-R⁶, -O-C(=Z)-R⁶, -O-C(=Z)-OR⁶, -O-C(=Z)-SR⁶, -O-C(=Z)- NR⁷R⁸, -S-C(=Z)-R⁶, -S-C(=Z)-OR⁶, -S-C(=Z)-SR⁶, -S-C(=Z)- NR⁷R⁸, -NR⁹-C(=Z)-R⁶, -NR⁹-C(=Z)-OR⁶, -NR⁹-C(=Z)-SR⁶ oder -NR⁹-C(=Z)- NR⁷R⁸ bedeutet, oder
A C₂-C₈Alkenyl ist, das gegebenenfalls mit ein oder mehreren Resten substituiert ist, wobei diese Reste Halogen, Cyano, Nitro, Hydroxy, C₁-C₈Alkyl, C₁-C₈Haloalkyl, C₁-C₈Alkoxy, C₁-C₈Haloalkoxy, Tri-(C₁-C₈-alkyl)silyl, Aryl-(C₁-C₈)-Dialkylsilyl, Diaryl-(C₁-C₈)-Alkylsilyl, Triarylsilyl, -COOR⁶, -CO-NR⁷R⁸, C₆-C₁₄Aryl und/oder Heteroaryl mit ein bis drei Ringheteroatomen sind, wobei diese Reste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A C₂-C₈Alkinyl ist, das gegebenenfalls mit ein oder mehreren Resten substituiert ist, wobei diese Reste Halogen, Cyano, Nitro, Hydroxy, C₁-C₈Alkyl, C₁-C₈Haloalkyl, C₁-C₈Alkoxy, C₁-C₈Haloalkoxy, Tri-(C₁-C₈-alkyl)silyl, Aryl-(C₁-C₈)-Dialkylsilyl, Diaryl-(C₁-C₈)-Alkylsilyl, Triarylsilyl, -COOR⁶, -CO-NR⁷R⁸, C6-C₁₄Aryl und/oder Heteroaryl mit ein bis drei Ringheteroatomen sind, wobei diese Reste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A C₆-C₁₄Aryl ist, das gegebenenfalls mit ein oder mehreren Resten substituiert ist, wobei diese Reste Halogen, Cyano, Hydroxy, Nitro, C₁-C₈Alkyl, C₁-C₈Haloalkyl, C₁-C₈Alkoxy, C₁-C₈Haloalkoxy, C₁-C₈Alkylthio, C₁-C₈Haloalkylthio, C₂-C₈Alkenyl, C₂-C₈Haloalkenyl, C₂-C₈Alkinyl, C₂-C₈Haloalkinyl, C₂-C₈-Alkyloxyalkylen, C₂-C₈-Alkylthioalkylen, C₃-C₈-Alkanoyloxyalkylen, C₁-C₈-Aminoalkylen, Phenyloxyalkylen, Phenylthioalkylen, C₃-C₈Cycloalkyl, C₆-C₁₄Aryl, Heteroaryl mit ein bis drei Ringheteroatomen, -COOR⁶, -CO-NR⁷R⁸, und/oder -S(O)ᵣ-R³ sind, worin r und R³ die weiter oben definierte Bedeutung besitzen, wobei die Aryl- und/oder die Heteroarylreste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A ein heterocyclischer Rest mit ein bis drei Ringheteroatomen und vorzugsweise zwei bis acht Ringkohlenstoffatomen ist, der gegebenenfalls mit ein oder mehreren Resten substituiert ist, wobei diese Reste Halogen, Cyano, Hydroxy, Nitro, C₁-C₈Alkyl, C₁-C₈Haloalkyl, C₁-C₈Alkoxy, C₁-C₈Haloalkoxy, C₁-C₈Alkylthio, C₁-C₈Haloalkylthio, C₂-C₈Alkenyl, C₂-C₈Haloalkenyl, C₂-C₈Alkinyl, C₂-C₈Haloalkinyl, C₂-C₈-Alkyloxyalkylen, C₂-C₈-Alkylthioalkylen, C₃-C₈-Alkanoyloxyalkylen, C₁-C₈-Aminoalkylen, Phenyloxyalkylen, Phenylthioalkylen, C₃-C₈Cycloalkyl, C₆-C₁₄Aryl, Heteroaryl mit ein bis drei Ringheteroatomen, -COOR⁶, -CO-NR⁷R⁸ und/oder -S(O)ᵣ-R³ sind, worin r und R³ die weiter oben definierte Bedeutung besitzen, wobei die Aryl- und/oder die Heteroarylreste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A ein durch ein bis sechs Gruppen substituiertes C₁-C₈Alkyl ist, dessen Substituenten ausgewählt werden aus der Gruppe bestehend aus Hydroxy, Amino, N-(C₁-C₈-Alkyl)amino, N,N-Bis-(C₁-C₈-Alkyl)amino, C₁-C₈Alkoxy, C₁-C₈Haloalkoxy, C₁-C₈Acyloxy und C₁-C₈Haloacyloxy, oder wobei zwei Substituenten zusammen auch eine gegebenenfalls ein oder mehrere Sauerstoff-, Stickstoff- und/oder Schwefelatome enthaltende Alkylenkette ausbilden können, beispielsweise ein Acetal-, Lacton- oder Lactam-Ringsystem bilden,
Z =O, =S, =N-R³³, =N-O-R³³ oder =N-NR³³R³⁴ darstellt,
R⁶, R⁷, R⁸, R⁹, R³³ und R³⁴ unabhängig voneinander Wasserstoff, C₁-C₈Alkyl, C₂-C₈Alkenyl, C₂-C₈Alkinyl, C₃-C₁₀Cycloalkyl, C₄-C₁₀Cycloalkenyl, C₄-C₁₀Cycloalkinyl, C₆-C₁₄Aryl oder Heteroaryl mit ein bis drei Ringheteroatomen bedeuten, die ihrerseits durch Halogen, Hydroxy, Cyano, Nitro, C₁-C₈Alkyl, C₂-C₈Alkenyl, C₂-C₈Alkinyl, C₃-C₁₀Cycloalkyl, C₄-C₁₀Cyctoalkenyl, C₄-C₁₀Cycloalkinyl, C₆-C₁₄Aryl, halogensubstituiertes C₆-C₁₄Aryl, Heteroaryl mit ein bis drei Ringheteroatomen oder halogensubstituiertes Heteroaryl mit ein bis drei Ringheteroatomen, Amino, N-(C₁-C₈-Alkyl)amino, N,N-Bis-(C₁-C₈-Alkyl)amino, Tri-(C₁-C₈-alkyl)silyl, Aryl-(C₁-C₈)-Dialkylsilyl, Diaryl-(C₁-C₈)-Alkylsilyl, Triarylsilyl, C₁-C₈Alkoxy und/oder C₁-C₈Haloalkoxy substituiert sein können,
B eine zweiwertige Brückengruppe darstellt und Alkylen mit ein bis zwölf Kohlenstoffatomen, Cycloalkylen mit drei bis vierzehn Kohlenstoffatomen, Alkylen-Cycloalkylen mit vier bis sechsundzwanzig Kohlenstoffatomen, Alkylen-Cycloalkylen-Alkylen mit fünf bis achtunddreizig Kohlenstoffatomen darstellt, wobei diese Brückengruppen ein bis drei ethylenisch-ungesättigte Bindungen aufweisen können und/oder durch -O-, -S-, -C(=O)O- oder -NR⁵- Gruppen unterbrochen sein können, wobei R⁵ die oben definierte Bedeutung aufweist, und wobei die Brückengruppe gegebenenfalls ein bis zehn Substituenten aufweist, die ausgewählt werden aus der Gruppe bestehend aus C₁-C₃Alkyl, Trifluormethyl oder Trichlormethyl, und
R⁴ einen einwertigen C₆-C₁₄Arylrest oder stickstoffhaltigen Heteroarylrest mit mindestens einem, vorzugsweise ein bis drei, Ringheteroatomen darstellt, der gegebenenfalls mit ein bis vier Resten substituiert ist, die ausgewählt werden aus der Gruppe bestehend aus Halogen, Cyano, Nitro, Thiocyanato, Isocyanato, C₁-C₈Alkyl, C₂-C₈Alkenyl, C₂-C₈Alkinyl, C₃-C₁₀Cycloalkyl, C₄-C₁₀Cycloalkenyl, C₄-C₁₀Cycloalkinyl, C₆-C₁₄Aryl, stickstoffhaltigem Heteroaryl mit ein bis drei Ringheteroatomen, wobei diese Substituenten wiederum substituiert sein können mit Resten ausgewählt aus der Gruppe bestehend aus Halogen, Cyano, Nitro, C₁-C₈Alkyl, C₁-C₈Haloalkyl, C₁-C₈Alkoxy und C₁-C₈Haloalkoxy, -C(=Z)-R⁶, -C(=Z)-OR⁶, -C(=Z)-SR⁶, -C(=Z)-NR⁷R⁸, -C(=Z)-O-N=C(NH₂)-R⁶, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)- NR⁷R⁸, -NR⁹-C(=Z)-R⁶, -NR⁹-C(=Z)-OR⁶, -NR⁹-C(=Z)-SR⁶, -NR⁹-C(=Z)-NR⁷R⁸, -OR⁶, -SR⁶, -S(=O)R⁶ ,-S(=O)₂R⁶, -NR⁷R⁸, worin Z, R⁶, R⁷, R⁸ und R⁹ die oben definierten Bedeutungen besitzen.

Unter dem Ausdruck "Halogen" sind Fluor, Chlor, Brom und/oder Jod zu verstehen. Bevorzugt werden Fluor, Chlor und/oder Brom.

Unter dem Ausdruck "(C₁-C₈)-Alkyl" ist ein unverzweigter oder verzweigter aliphatischer und gesättigter Kohlenwasserstoffrest mit ein bis acht Kohlenstoffatomen zu verstehen, wie z. B. der Methyl-, Ethyl-, Propyl-, Isopropyl-, 1-Butyl-, 2-Butyl-, Isobutyl-, tert-Butyl-, n-Pentyl, 2-Methylbutyl-, 1,1-Dimethylpropyl-, n-Hexyl, n-Heptyl, n-Octyl oder 2-Ethylhexyl-Rest. Bevorzugt wird Methyl.

Unter "(C₁-C₈)-Haloalkyl" ist eine unter dem Ausdruck "(C₁-C₈)-Alkyl" genannte Alkylgruppe zu verstehen, in der ein oder mehrere Wasserstoffatome durch die gleiche Anzahl gleicher oder verschiedener Halogenatome, bevorzugt Chlor oder Fluor, ersetzt sind, wie die Trifluormethyl-, die 1- oder 2-Fluorethyl-, die 2,2,2-Trifluorethyl-, die Chlormethyl-, Fluormethyl-, die Difluormethyl- oder die 1,1,2,2-Tetrafluorethylgruppe.

Unter "(C₁-C₈)-Alkoxy" sind Alkoxygruppen zu verstehen, deren Kohlenwasserstoffreste die unter den Ausdrücken "(C₁-C₈)-Alkyl " angegebenen Bedeutungen haben.

Unter "(C₁-C₈)-Haloalkoxy" "ist eine unter dem Ausdruck "(C₁-C₈)-Alkoxy" genannte Alkoxygruppe zu verstehen, in der ein oder mehrere Wasserstoffatome durch die gleiche Anzahl gleicher oder verschiedener Halogenatome, bevorzugt Chlor oder Fluor, ersetzt sind, wie die Trifluormethyloxy-, die 1- oder 2-Fluorethyloxy-, die 2,2,2-Trifluorethyloxy-, die Chlormethyloxy-, Fluormethyloxy-, die Difluormethyloxy- oder die 1,1,2,2-Tetrafluorethyloxygruppe.

Unter "(C₁-C₈)-Alkylthio" sind Alkylthiogruppen zu verstehen, deren Kohlenwasserstoffreste die unter den Ausdrücken "(C₁-C₈)-Alkyl "angegebenen Bedeutungen haben.

Unter "(C₁-C₈)-Haloalkylthio" ist eine unter dem Ausdruck "(C₁-C₈)-Alkylthio" genannte Alkylthiogruppe zu verstehen, in der ein oder mehrere Wasserstoffatome durch die gleiche Anzahl gleicher oder verschiedener Halogenatome, bevorzugt Chlor oder Fluor, ersetzt sind, wie die Trifluormethylthio-, die 1- oder 2-Fluorethylthio-, die 2,2,2-Trifluorethylthio-, die Chlormethylthio-, Fluormethylthio-, die Difluormethylthio- oder die 1,1,2,2-Tetrafluorethylthiogruppe.

Unter "(C₃-C₁₀)-Cycloalkyl" sind monocyclische und gesättigte Alkylreste mit drei bis zehn Ringkohlenstoffatomen zu verstehen. Beispiele dafür sind Cyclopropyl, Cyclobutyl, Cyclopentyl, Cyclohexyl, Cycloheptyl oder Cyclooctyl. Ferner sind darunter bicyclische und gesättigte Alkylreste zu verstehen, wie der Norbornyl- oder Bicyclo[2.2.2]octyl-Rest oder auch kondensierte und gesättigte Systeme wie z. B. der Dekahydronaphthyl-Rest.

Der Ausdruck "Tri-(C₁-C₈-alkyl)-silyl" bedeutet ein Siliciumatom, das drei gleiche oder verschiedene Alkylreste gemäß der obigen Definition trägt. Analog stehen "Aryl-(C₁-C₈)-Dialkylsilyl" für ein Siliciumatom, das einen Arylrest und zwei gleiche oder verschiedene Alkylreste gemäß der obigen Definition trägt, "Diaryl-(C₁-C₈)-Alkylsilyl" für ein Siliciumatom, das einen Alkylrest und zwei gleiche oder verschiedene Arylreste gemäß der obigen Definition trägt, und "Triarylsilyl" für ein Siliciumatom, das drei gleiche oder verschiedene Arylreste gemäß der obigen Definition trägt.

Beispiele für Reste -COOR⁶ sind die Carboxylgruppe oder deren Ester mit einwertigen aliphatischen, cycloaliphatischen, aromatischen oder heterocyclischen Alkoholen gemäß den weiter oben für R⁶ gegebenen Definitionen. Bevorzugt werden die Carboxylgruppe, die Methyl-, Ethyl- oder Propylester, die Cyclohexylester und die Phenylester, wobei in den aliphatischen, cycloaliphatischen, aromatischen oder heterocyclischen Resten ein oder mehrere Wasserstoffatome durch Halogen, insbesondere durch Fluor oder Chlor ersetzt sein können. Ein Beispiel dafür ist die Trifluormethylgruppe.

Beispiele für Reste -CO-NR⁷R⁸ sind die Carboxylamidgruppen, deren Stickstoffatom ein- oder zweifach mit einwertigen aliphatischen, cycloaliphatischen, aromatischen oder heterocyclischen Resten gemäß den weiter oben für R⁷ und R⁸ gegebenen Definitionen substituiert sein kann. Bevorzugt werden die Carboxylamidgruppe, die N-Methyl-, N-Ethyl- oder N-Propylcarboxamidgruppe sowie die entsprechend disubstituierten Derivate, wie N,N-Dimethyl-, N,N-Diethyl- oder N,N-Dipropylcarboxamidgruppe sowie die entsprechend unsymmetrisch substituierten Gruppen, wobei in den aliphatischen, cycloaliphatischen, aromatischen oder heterocyclischen Resten ein oder mehrere Wasserstoffatome durch Halogen, insbesondere durch Fluor oder Chlor ersetzt sein können. Ein Beispiel dafür ist die Trifluormethylgruppe.

Unter dem Ausdruck "(C₆-C₁₄)-Aryl" ist ein carbocyclischer, d.h. aus Kohlenstoffatomen aufgebauter, aromatischer Rest mit 6 bis 14, insbesondere 6 bis 12 Ringkohlenstoffatomen zu verstehen. Beispiele dafür sind monocyclische aromatische Reste, wie Phenyl, polycyclische anellierte aromatische Reste, wie Naphthyl oder Anthracyl, oder polycyclische, über C-C-Bindungen oder Brückengruppen, wie -O- oder-S-, verbundene aromatische Reste, wie Biphenylyl.

Bevorzugt wird Phenyl.

Der Ausdruck " heterocyclischer Rest mit ein bis drei Ringheteroatomen" steht für einen cyclischen Rest, der vollständig gesättigt, teilweise ungesättigt oder vollständig ungesättigt bzw. aromatisch sein kann und der durch mindestens ein bis drei gleiche oder verschiedene Heteroatome, vorzugsweise aus der Gruppe Stickstoff, Schwefel oder Sauerstoff, unterbrochen ist, wobei jedoch nicht zwei Sauerstoffatome direkt benachbart sein dürfen und noch mindestens ein Kohlenstoffatom im Ring vorhanden sein muß. Vorzugsweise handelt es sich um Reste mit vier, fünf, sechs oder sieben Ringatomen, insbesondere fünf oder sechs Ringatomen.

Die Bezeichnungen "C₂-C₈Alkenyl" bzw. "C₃-C₈Alkenyl" und "C₂-C₈Alkinyl" bzw. "C₃-C₈Alkinyl" bedeuten einen geradkettigen oder verzweigten Kohlenwasserstoffrest mit zwei bis acht bzw. drei bis acht Kohlenstoffatomen, der mindestens eine Mehrfachbindung beinhaltet, wobei sich diese an beliebiger Position des betreffenden ungesättigten Restes befinden kann.

"(C₂-C₈)-Alkenyl" steht demnach beispielsweise für die Vinyl-, Allyl-, 2-Methylpropenyl-, 1- oder 2-Butenyl-, Pentenyl-, 2-Methylpentenyl-, Hexenyl-, Heptenyl- oder Octenyl-Gruppe.

"(C₂-C₈)-Alkinyl" steht demnach beispielsweise für die Ethinyl-, Propargyl-, 2-Methylpropinyl-, 2-Butinyl-, Pentinyl-, 2-Methylpentinyl-, Hexinyl-, Heptinyl- oder Octinyl-Gruppe.
Unter "(C₂-C₈)-Haloalkenyl" bzw. "(C₂-C₈)-Haloalkinyl" sind unter dem Ausdruck "(C₂-C₈)-Alkenyl" bzw. "(C₂-C₈)-Alkinyl" genannte Alkenyl- bzw. Alkinylgruppen zu verstehen, in denen ein oder mehrere Wasserstoffatome durch die gleiche Anzahl gleicher oder verschiedener Halogenatome, bevorzugt Chlor oder Fluor, ersetzt sind, wie die 1- oder 2-Fluorethenyl- oder-ethinylgruppe oder die 1- oder 2-Chloroethenyl- oder -ethinylgruppe.

Unter "C₂-C₈-Alkyloxyalkylen" sind Alkylreste mit zwei bis acht Kohlenstoffatomen zu verstehen, die ein oder mehrere Sauerstoffatome in der Alkylkette enthalten. Beispiele dafür sind Methoxymethylen, Methoxyethylen, Methoxypropylen, Methoxybutylen, Ethoxymethylen, Ethoxyethylen, Ethoxypropylen und Ethoxybutylen.

Unter "C₂-C₈-Alkylthioalkylen" sind Alkylreste mit zwei bis acht Kohlenstoffatomen zu verstehen, die ein oder mehrere Schwefelatome in der Alkylkette enthalten. Beispiele dafür sind Methylthiomethylen, Methylthioethylen, Methylthiopropylen, Methylthiobutylen, Ethylthiomethylen, Ethylthioethylen, Ethylthiopropylen und Ethylthiobutylen.

Unter "C₃-C₈-Alkanoyloxyalkylen" sind Alkylreste mit drei bis acht Kohlenstoffatomen zu verstehen, die ein oder mehrere Carbonyloxygruppen, vorzugsweise eine Carbonyloxygruppe, in der Alkylkette enthalten. Beispiele dafür sind Methanoyloxymethylen, Methanoyloxyethylen, Methanoyloxypropylen, Methanoyloxybutylen, Ethanoyloxymethylen, Ethanoyloxyethylen, Ethanoyloxypropylen und Ethanoyloxybutylen.

Unter "C₁-C₈-Aminoalkylen" sind Alkylreste mit ein bis acht Kohlenstoffatomen zu verstehen, die ein oder mehrere Aminogruppen in der Alkylkette oder als Substituenten der Alkylkette enthalten. Die Aminogruppen können ihrerseits ein- oder zweifach alkylsubstituiert sein. Die Aminogruppen können endständig sein oder sich in der Kette befinden. Beispiele dafür sind Aminomethylen, 2-Aminoethylen, 3-Aminopropylen, 4-Aminobutylen, Methylaminomethylen, 2-Methylaminoethylen, 3-Methylaminopropylen und 4-Methylaminobutylen.

Unter "Phenyloxyalkylen" sind Alkylreste zu verstehen, die mit einer oder mehreren Phenyloxygruppen substituiert sind. Ein Beispiel dafür ist Phenyloxymethylen.

Unter "Phenylthioalkylen" sind Alkylreste zu verstehen, die mit einer oder mehreren Phenylthiogruppen substituiert sind. Ein Beispiel dafür ist Phenylthiomethylen.

Der Ausdruck "durch ein bis sechs Gruppen substituiertes C₁-C₈Alkyl" steht für einen C₁-C₈Alkylrest beim dem ein bis sechs Wasserstoffatome durch Hydroxylgruppen, Aminogruppen, die gegebenenfalls am Stickstoff durch ein oder zwei C₁-C₈Alkylgruppen substituiert sein können, C₁-C₈Alkoxygruppen, C₁-C₈Haloalkoxygruppen, C₁-C₈Acyloxygruppen, C₁-C₈Haloacyloxygruppen oder Kombinationen dieser Gruppen substituiert sind.

Dabei besitzen die Begriffe "C₁-C₈Alkylgruppen", "C₁-C₈Alkoxygruppen" und "C₁-C₈Haloalkoxygruppen" die oben angegebene Bedeutung.

Unter "C₁-C₈Acyloxygruppen" sind einwertige Reste von gesättigten Carbonsäuren mit ein bis acht Kohlenstoffatomen zu verstehen. Beispiele dafür sind die Formyl-, Acetyl-, Propionyl-, Butyryl-, 2-Methylbutyryl-, Pivaloyl- oder Octanoyl-Gruppe.

Unter "C₁-C₈Haloacyloxygruppen" sind einwertige Reste von gesättigten Carbonsäuren mit ein bis acht Kohlenstoffatomen zu verstehen, bei denen ein oder mehrere Wasserstoffatome durch Halogenatome substituiert sind. Beispiele dafür sind die Chloroformyl-, Monochloroacetyl-, Dichloroacetyl-, Trichloroacetyl, Monochloropropionyl-, Dichloropropionyl- Trichloropropionyl, Tetrachloropropionyl-, Pentachloropropionyl-, Monochlorobutyryl-, Dichlorobutyryl-, Trichlorobutyryl-, Tetrachlorobutyryl-, Pentachlorobutyryl-, Hexachlorobutyryl-, Heptachlorobutyryl-Gruppe oder die entsprechenden bromierten Derivate bzw. bromierte und chlorierte Derivate.

Unter "(C₄-C₁₀)-Cycloalkenyl" sind monocyclische, mindestens eine Mehrfachbindung enthaltende Alkylreste mit vier bis zehn Ringkohlenstoffatomen zu verstehen. Beispiele dafür sind Cyclobutenyl, Cyclopentenyl, Cyclohexenyl, Cycloheptenyl, Cyclooctenyl oder Cyclodecenyl. Ferner sind unter solchen Resten bicyclische, mindestens eine Mehrfachbindung enthaltende Alkylreste zu verstehen, wie der Norbornenyl- oder Bicyclo[2.2.2]octenyl-Rest oder auch kondensierte, mindestens eine Mehrfachbindung enthaltende Systeme wie z. B. der Tetra-, Hexa- oder Oktahydronaphthyl-Rest.

"(C₄-C₁₀)-Cycloalkinyl" steht z.B. für den Cyclooctinyl-, Cyclononinyl- oder Cyclodecinyl-Rest.

Unter dem Ausdruck "zweiwertige Brückengruppe" sind zweiwertige Alkylenreste mit ein bis zwölf Kohlenstoffatomen, zweiwertige Cycloalkylenreste mit drei bis vierzehn Kohlenstoffatomen, sowie deren Kombinationen, wie Alkylen-Cycloalkylen mit vier bis sechsundzwanzig Kohlenstoffatomen, Alkylen-Cycloalkylen-Alkylen mit fünf bis achtunddreizig Kohlenstoffatomen, zu verstehen. Diese Alkylen- und/oder Cycloalkylenreste können dabei ein bis drei ethylenisch-ungesättigte Bindungen aufweisen und/oder sie können durch ein oder mehrere Sauerstoffatome, Schwefelatome, Stickstoffatome oder Sauerstoffatome aufweisende Gruppen unterbrochen sein. Die Alkylenreste können dabei geradkettig oder verzweigt sein.

Diese Alkylen- und/oder Cycloalkylenreste können ferner ein bis zehn Substituenten aufweisen. Dabei handelt es sich um C₁-C₃Alkylgruppen und/oder um Trichlormethyl und/oder um Trifluormethyl. Beispiele für C₁-C₃Alkylgruppen sind Methyl, Ethyl, n-Propyl und Isopropyl. Bevorzugt werden Methyl, Trichlormethyl und Trifluormethyl.

Der Ausdruck "stickstoffhaltiger Heteroarylrest mit mindestens einem, vorzugsweise ein bis drei, Ringheteroatomen" steht für einen heteroaromatischen Rest, der durch mindestens ein oder mehrere gleiche oder verschiedene Heteroatome, insbesondere Atome aus der Gruppe Stickstoff, Schwefel oder Sauerstoff, unterbrochen sein kann, von denen mindestens ein Heteroatom ein Stickstoffatom sein muß, wobei jedoch nicht zwei Sauerstoffatome direkt benachbart sein dürfen und noch mindestens ein Kohlenstoffatom im Ring vorhanden sein muß. Vorzugsweise handelt es sich um Reste mit fünf oder sechs Ringatomen, von denen eines, zwei oder drei Stickstoffatome sind.

Bevorzugt werden Verbindungen der Formel I, worin R¹ und R² jeweils Chlor oder Brom bedeuten.

Bevorzugt werden ebenfalls Verbindungen der Formel I, worin Y -O- ist.

Ganz besonders bevorzugt werden Verbindungen der Formel I, worin R¹ und R² jeweils Chlor sind und Y -O- ist.

Ferner werden Verbindungen der Formel I bevorzugt, worin X -O-, -S- oder -NH- ist und X' eine direkte C-C-Bindung, -O-, -S- oder -NH- ist sind, insbesondere Verbindungen der Formel I, worin X und X' jeweils -O- oder X -O- und X' eine direkte C-C-Bindung bedeuten.

Bevorzugt werden Verbindungen der Formel I, worin R³ Wasserstoff, Fluor, Chlor, Brom, Methyl, Trifluormethyl, Monochlormethyl, Dichlormethyl, Trichlormethyl, Methoxy, Trifluormethoxy, Monochlormethoxy, Dichlormethoxy, Trichlormethoxy, Nitro, Cyano, Cyclohexyl, Phenyl oder ein einwertiger Rest abgeleitet von Thiophen, Furan, Pyrrol, Thiazol, Oxazol, Imidazol, Isothiazol, Isoxazol, Pyrazol, 1,3,4-Oxadiazol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Tetrazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin, 4H-Chinolizin, Piperidin, Pyrrolidin, Oxazolin, Tetrahydrofuran, Tetrahydropyran, Isoxazolidin oder Thiazolidin bedeutet.

Ganz besonders bevorzugt werden Verbindungen der Formel 1, worin R³ Methyl, Trifluormethyl, Cyano, Chlor oder Brom bedeutet.

Bevorzugt werden Verbindungen der Formel I, worin Z =O, =N-OH, =N-OCH₃ oder =N-CH₂-CH=CH₂ bedeutet.

Besonders bevorzugte Reste A sind die Gruppen -C(=O)-CH₃, -C(=N-OCH₃)-CH₃, -C(=N-OH)-CH₃, -C(=O)-OCH₃, -C(=O)-CH=CH-N(CH₃)₂, -C(=O)-NH-C₂H₅, -C(=O)-NH₂, -C(=O)-N(CH₃)₂, -C(=O)-NH-CH₂-CH₂-OH, -C(=O)-NH-CH(CH₃)-CH₂-OH, -C(=O)-CHBr₂, -C(=O)-N(CH₃)-O-CH₃, -C(=N-O-CH₂-CH=CH₂)-CH₃, -C(=S)-NH₂, -C(=O)-O-C₆H₅, -C(=O)-O-C₃H₇, -C(=O)-imidazolyl, -C(=O)-3-ethyl-1,2,4-oxadiazol-5-yl, -C(=O)-2Δ-oxazolin-2-yl, -C(=O)-1-methyl-pyrazol-3-yl, -C(=O)-ethin-2-yl, -C(=O)-1-hydroxy-ethin-2-yl, -C(=O)-1-trimethylsilyl-ethin-2-yl, -C(=O)-1-hexyl-ethin-2-yl, -C(=O)-1-(2-chlorphenyl)-ethin-2-yl, -C(=O)-1-methoxy-ethin-2-yl und -C(=O)-ethen-2-yl.

Bevorzugt werden Verbindungen der Formel I, worin A C₂-C₈Alkenyl, C₂-C₈Alkinyl, Phenyl, Pyridyl oder Pyrimidyl bedeutet, die gegebenenfalls substituiert sind durch ein oder mehrere Halogenatome, Cyanogruppen, Nitrogruppen, Hydroxylgruppen, C₁-C₃-Alkoxyreste oder C₁-C₃Haloalkoxyreste, C₁-C₃Alkoxyreste, C₁-C₃Haloalkoxyreste, die ihrerseits gegebenenfalls substituiert sind durch ein oder mehrere Halogenatome, Cyanogruppen, Nitrogruppen, Hydroxylgruppen, C₁-C₃Alkoxyreste oder C₁-C₃Haloalkoxyreste,
oder die -OR⁶, -SR⁶, -NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)- NR⁷R⁸, -S-C(=Z)-R⁶, -S-C(=Z)-OR⁶, -S-C(=Z)-SR⁶, -S-C(=Z)- NR⁷R⁸, -NR⁹C(=Z)-R⁶, -NR⁹-C(=Z)-OR⁶, -NR⁹-C(=Z)-SR⁶ oder -NR⁹-C(=Z)-NR⁷R⁸, bedeutet, wobei Z, R⁶, R⁷, R⁸ und R⁹ die weiter oben definierten Bedeutungen besitzen,
oder die durch ein oder mehrere Hydroxylgruppen, C₁-C₃-Alkoxyreste, C₁-C₃Haloalkoxyreste, C₁-C₃Acyloxyreste oder C₁-C₃Haloacyloxyreste substituiertes C₁-C₈-Alkyl bedeutet,
oder die eine Gruppe der Formeln Q¹ - Q¹⁰ bedeuten 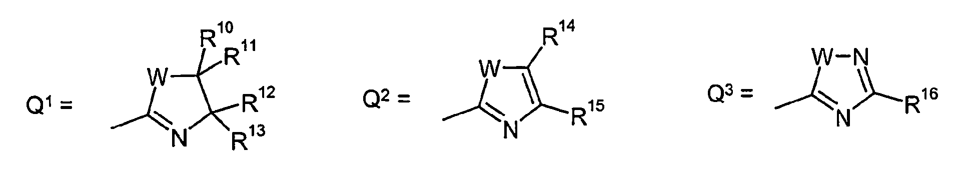 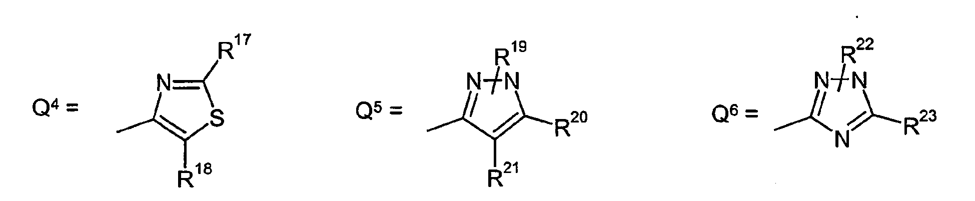 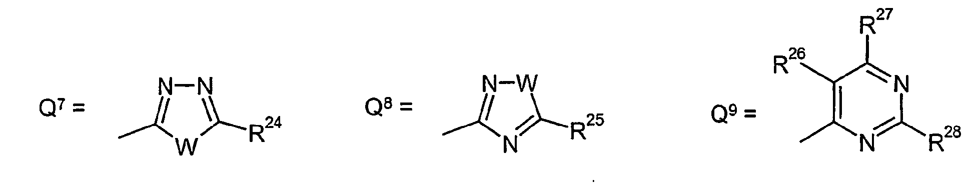 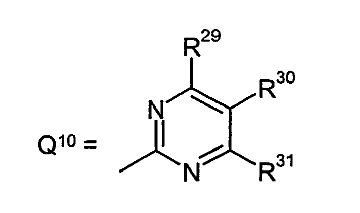 worin
W Sauerstoff oder Schwefel ist, und
R¹⁰ bis R¹⁸, R²⁰ bis R²¹, R²³ bis R³¹ Wasserstoff, C₁-C₆Alkyl, C₃-C₇Cycloalkyl, C₂-C₆Alkenyl, C₄-C₇Cycloalkenyl, Pyridyl oder Phenyl bedeuten, wobei die C₁-C₆Alkyl-, C₃-C₇Cycloalkyl-, C₂-C₆Alkenyl- oder die C₄-C₇Cycloalkenylreste gegebenenfalls ein- oder mehrfach, vorzugsweise ein-, zwei- oder dreifach substitiert sind durch Halogen, Cyano, -OR⁶, -SR⁶, -S(O)R⁶, -S(O)₂R⁶ und/oder-NR⁷R⁸, wobei die Reste R⁶, R⁷ und R⁸ die oben definierte Bedeutung aufweisen, und wobei Phenyl oder Pyridyl gegebenenfalls ein- oder mehrfach, vorzugsweise ein-, zwei- oder dreifach substitiert sind durch Halogen, Cyano, Nitro, -OR⁶, -SR⁶, -S(O)R⁶, -S(O)₂R⁶,-NR⁷R⁸, C₁-C₄Alkyl, C₁-C₄Haloalkyl, C₂-C₄Alkenyl und/oder C₂-C₄Haloalkyl.

Bevorzugt werden Verbindungen der Formel I, worin B eine Gruppe der Formeln P¹ bis P⁶ bedeutet 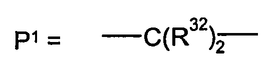 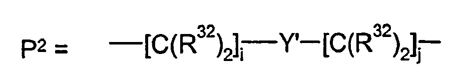 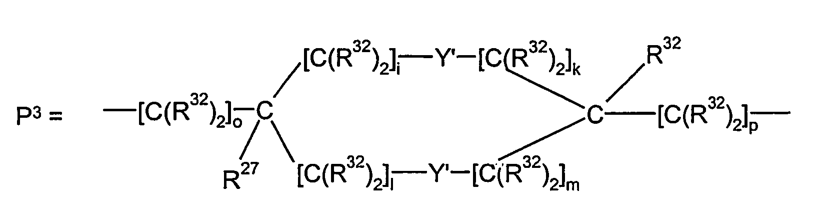 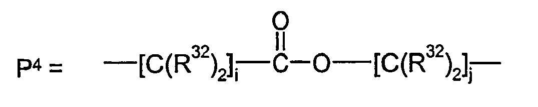 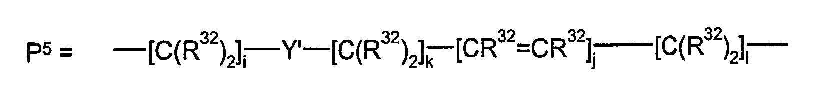 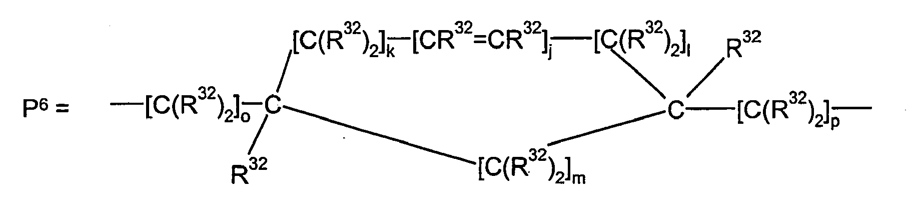 worin R³² jeweils unabhängig voneinander Wasserstoff, C₁-C₃Alkyl oder Trifluormethyl bedeuten,
Y' jeweils unabhängig voneinander eine direkte C-C-Bindung, -O-, -S- oder -NHbedeuten,
i eine ganze Zahl von 1 bis 6 ist,
j eine ganze Zahl von 1 bis 6 ist,
k eine ganze Zahl von 0 bis 2 ist,
I eine ganze Zahl von 0 bis 2 ist,
m eine ganze Zahl von 0 bis 2 ist,
o eine ganze Zahl von 0 bis 1 ist, und
p eine ganze Zahl von 0 bis 1 ist.

Ganz besonders bevorzugt werden Verbindungen der Formel I, worin B eine Gruppe der Formel -C_{q}H_{2q-} ist, und q eine ganze Zahl von 2 bis 4 ist, insbesondere -(CH₂)₂-, -(CH₂)₃- und -(CH₂)₄-.

Bevorzugt werden ferner Verbindungen der Formel I, worin R⁴ C₆-C₁₄Aryl bedeutet, das gegebenenfalls substituiert ist durch ein oder mehrere, vorzugsweise ein bis drei Reste ausgewählt aus der Gruppe bestehend aus Halogen, Cyano, Nitro, Thiocyanato, Isocyanato, C₁-C₆-Alkyl, C₃-C₈-Cycloalkyl, C₂-C₈-Alkenyl, C₄-C₈-Cycloalkenyl und/oder C₂-C₈-Alkinyl, wobei ein oder mehrere Wasserstoffatome der C₁-C₆-Alkyl-, C₃-C₈-Cycloalkyl-, C₂-C₈-Alkenyl-, C₄-C₈-Cycloalkenyl- und/oder C₂-C₈-Alkinylreste durch Halogen und/oder Cyano substituiert sein können.

Besonders bevorzugt werden Verbindungen der Formel I, worin R⁴ ausgewählt wird aus der Gruppe bestehend aus Phenyl, Pyridyl, Pyrimidyl, Pyrazinyl, Pyrazolyl oder Naphthyl, die gegebenenfalls substituiert sind durch einen oder mehrere Reste ausgewählt aus der Gruppe bestehend aus Halogen, Cyano, Nitro, C₁-C₃-Alkyl, C₁-C₃-Haloalkyl, C₁-C₃-Alkoxy, C₁-C₃-Haloalkoxy, -C(=Z)-R⁶, -C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)-NR⁷R⁸, -NR⁹-C(=Z)-R⁶, ,-NR-C(=Z)-OR⁶ ,-NR⁹-C(=Z)-SR⁶, -NR⁹-C(=Z)- NR⁷R⁸, -OR⁶, -SR⁶, -S(=O)R⁶ und -S(=O)₂R⁶ bedeuten, wobei Z, R⁶, R⁷, R⁸ und R⁹ die weiter oben definierten Bedeutungen besitzen.

Ganz besonders bevorzugt werden Verbindungen der Formel I, worin R⁴ Phenyl oder Pyridyl ist, das ein bis drei Substituenten trägt aus der Gruppe Halogen, Cyano, Nitro, C₁-C₃-Alkyl und C₁-C₃-Haloalkyl oder von Kombinationen dieser Substituenten.

Ganz besonders bevorzugte Reste R⁴ sind Trifluoromethylphenyl, Chlorophenyl, Nitrophenyl, Nitropyridyl, Trifluoromethylpyridyl, Di-trifluoromethyl-pyridyl, Chloropyridyl, Dichloropyridyl, Chloro-trifluoromethyl-pyridyl, Trifluoromethylpyrimidyl, Di-trifluoromethyl-pyrimidyl, Methyl-trifluoromethyl-pyrimidyl und Trifluoromethyl-pyrazol.

Je nach Art der oben definierten Substituenten weisen die Verbindungen der allgemeinen Formel (I) saure oder basische Eigenschaften auf und können Salze bilden. Tragen die Verbindungen der allgemeinen Formel (I) beispielsweise Gruppen wie Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄)-Alkylresten sowie Mono-, Di- und Trialkanolamine von (C₁-C₄)-Alkanolen. Tragen die Verbindungen der allgemeinen Formel (I) beispielsweise Gruppen wie Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls insektizide, akarizide und mitizide Eigenschaften auf.

Die Verbindungen der allgemeinen Formel (I) können ein oder mehrere asymmetrische Kohlenstoffatome oder Stereoisomere an Doppelbindungen aufweisen. Es können daher Enantiomere oder Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Isomeren aufgetrennt werden. Racemate können nach üblichen Methoden in die Enantiomeren aufgetrennt werden.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Vertag, Stuttgart, beschrieben werden.

Die im Nachfolgenden beschriebenen Synthesewege benutzen Reaktionsdurchführungen sowie Aufarbeitungs- und Reinigungsmethoden, wie sie üblicherweise in der organisch-chemischen Literatur anzutreffen sind. Reaktionen finden im allgemeinen in Lösung statt, wobei beispielsweise alkoholische Lösungsmittel wie Methanol oder Ethanol; Kohlenwasserstoffe, wie Hexan, Benzol oder Toluol; Ether, wie Diethylether oder Tetrahydrofuran; Chlorkohlenwasserstoffe, wie Dichlormethan, Chloroform oder Tetrachlorethan; amidhaltige Lösungsmittel, wie N,N-Dimethylformamid; oder schwefelhaltige Lösungsmittel, wie Dimethylsulfoxid oder Sulfolan zum Einsatz kommen können.

Im Einzelfall können Reaktionen auch in einer Suspension, Emulsion oder in fester Phase stattfinden.

Als Reaktionstemperatur kann ein Bereich zwischen -200°C und 250°C in Frage kommen. Im allgemeinen liegt die Reaktionstemperatur jedoch zwischen -78°C und 150°C.

Zur Aufarbeitung wird üblicherweise eine Extraktion mit zwei oder mehreren nicht miteinander mischbaren Lösungsmitteln vorgenommen. Alternativ können unter anderem Festphasenextraktionen (H.G. Kicinski, Chemie in Labor und Biotechnik, 1996, 47(12), 542-8) oder das Ausfällen der Produkte in einem geeigneten Lösungsmittel bzw. Lösungsmittelgemisch zur Anwendung kommen.

Die Reinigung der Rohprodukte erfolgt üblicherweise durch chromatographische Verfahren, wie Säulenchromatographie oder HPLC; durch Destillation oder Kristallisation aus geeigneten Lösungsmitteln. Andere Verfahren sind jedoch nicht ausgeschlossen.

Die vorliegende Erfindung betrifft auch Verfahren zur Herstellung von Verbindungen der allgemeinen Formel (I) umfassend die Schritte: 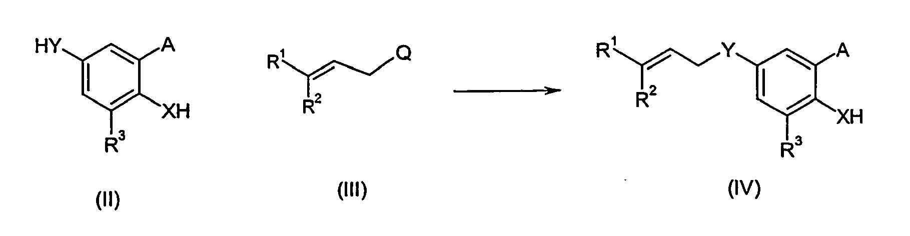 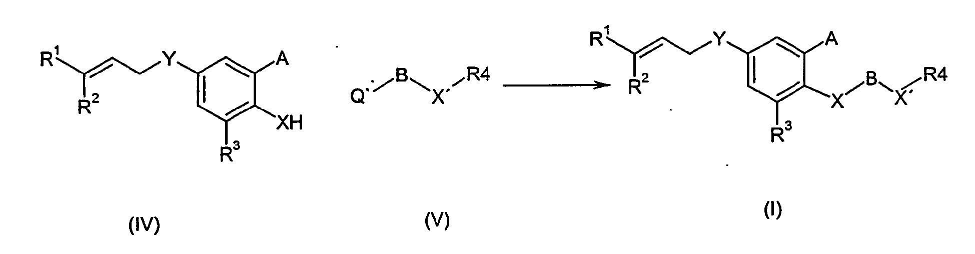
a) Umsetzung der Verbindungen der Formeln II und III zur Verbindung der Formel IV und
b) Umsetzung der Verbindung der Formeln IV und V zur Verbindung der Formel I,
worin R¹, R², R³, R⁴, A, Y, X und X' die weiter oben definierten Bedeutungen besitzen und Q und Q' Abgangsgruppen darstellen.

Zur Herstellung von Verbindungen der allgemeinen Formel (I) in welchen R¹, R², R³, R⁴, R⁵ und A die zur Formel (I) angegebene Bedeutungen haben und worin Y, X und X' jeweils O bedeuten kann beispielsweise gemäß den nachfolgenden Schemata 1 und 2 verfahren werden.

Eine allgemeine Herstellungsweise von Dihalogenpropenether-Verbindungen ist im Schema 1 dargestellt. Dihalogenpropen-Thioether bzw. -Amine sind in Analogie dazu durch Einsatz entsprechender Thioether- bzw. Amin-Ausgangsverbindungen darstellbar. 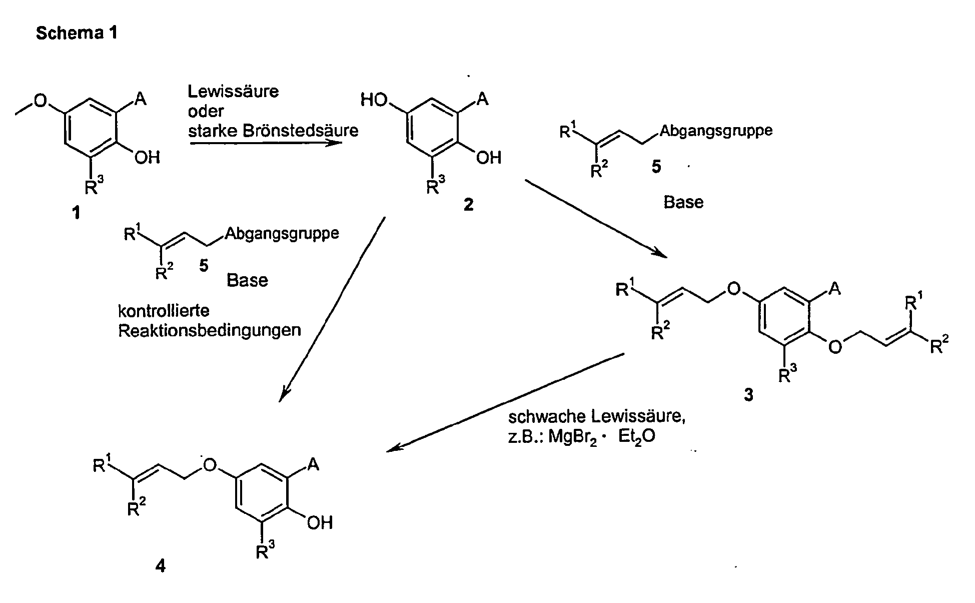 Eine große Zahl der hier beanspruchten Verbindungen ist über einen Baustein vom Typ 4 in Schema 1 als gemeinsames Intermediat zugänglich. Die Synthese von 4 geht in der Regel von käuflichen oder literaturbeschriebenen Verbindungen 1 aus. Zunächst wird der Methylether in 1 gespalten durch Umsetzung mit Lewissäuren wie zum Beispiel Bortribromid oder mit starken Brönstedsäuren, wie zum Beispiel Bromwasserstoffsäure. Die daraus entstandene Verbindung 2 wird mit 5 alkyliert. Dies kann unter vorsichtig kontrollierten Reaktionbedingungen (zum Beispiel Einhaltung niedriger Temperaturen und Unterschüssen an 5) geschehen, so dass der Baustein 4 in einem Schritt entsteht. Alternativ hierzu kann die Alkylierung auch zunächst 3 ergeben, welches anschließend unter Verwendung einer schwachen Lewissäure wie zum Beispiel Magnesiumdibromid-Diethylether-Komplex weiter zu 4 umgesetzt wird.

Die Phenole 4 können wie in Schema 2 ausgeführt im Sinne einer Alkylierungsreaktion mit einem Baustein vom Typ 11 oder im Sinne einer Kondensationsreaktion mit einem Baustein vom Typ 12 zu den Verbindungen 9 umgesetzt werden.

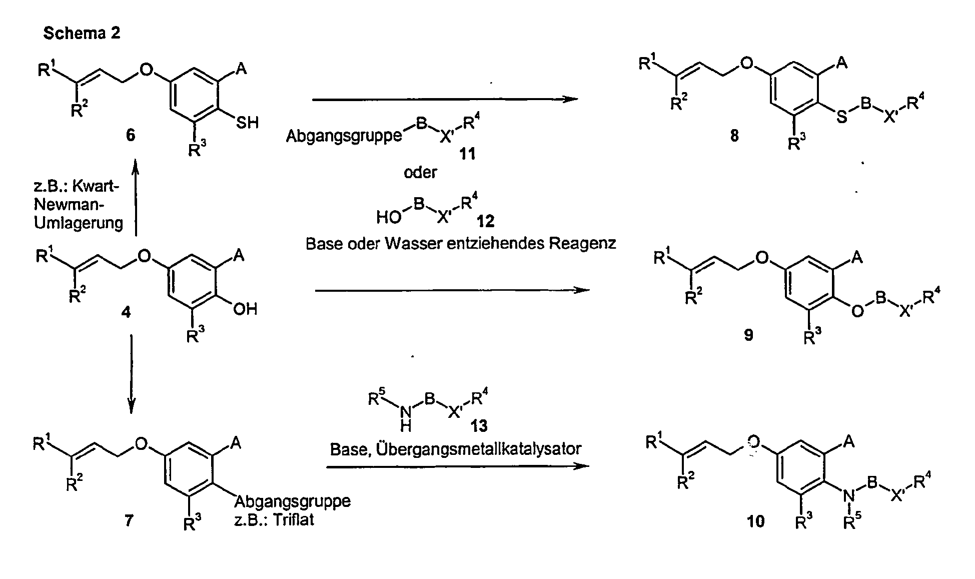

Im Falle einer Alkylierungsreaktion ist gegebenenfalls die Anwesenheit einer Base erforderlich, wobei organische Base, wie beispielsweise Triethylamin oder Dimethylaminopyridin, als auch anorganische Basen, wie beispielswise Kaliumcarbonat, in Betracht kommen. Zur Durchführung einer Kondensation ist die Anwesenheit eines wasserentziehenden Reagenzes oder einer wasserentziehenden Reagenzkombination erforderlich.

Die Reaktion erfolgt unter Mitsunobu-Bedingungen, also durch Umsetzung des OH-Derivats mit einem Alkohol in Gegenwart eines Azodicarbonsäurediesters sowie eines Phosphins. Beispielhaft sei hier Diethylazodicarboxylat in Kombination mit Triphenylphosphin genannt.

Die Darstellung der Bausteine 11 und 12 folgt in der Literatur beschriebenen Vorgehensweisen (z.B. WO-A-96/11,909). Die Phenole 4 können aber auch zunächst in die entsprechenden Thiophenole 6 übergeführt werden. Hierzu bietet sich eine zum Beispiel eine Kwart-Newman-Umlagerung über ein geeignetes N,N-Dialkyl-thiocarbamoyl-Derivat von 4 an (siehe K.-D. Gundermann, K. Hümke in Houben-Weyl "Methoden der Organischen Chemie", Georg-Thieme-Verlag, 4. Auflage, 1985, Band E11 "Organische Schwefelverbindungen Teil I", Seite 54 - 55). Die weitere Umsetzung zu den beanspruchten Verbindungen 8 erfolgt analog zur Überführung von 4 in 9.

Desweiteren kann die phenolische Gruppe in 4 in eine für übergangsmetallkatalysierte Reaktionen geeignete Abgangsgruppe umgewandelt werden (zum Beispiel einer Trifluormethansulfonat-Gruppe), um anschließend gemäß literaturbekannten Verfahren durch ein Amin vom Typ 13 substituiert zu werden (siehe zum Beispiel Wolfe, John P. et al. J. Org. Chem. 2000, 65(4), 1158 -1174).

Als Übergangsmetallkatalysatoren kommen insbesondere Palladiumkomplexe in Betracht. Diese Vorgehensweise führt zu den beanspruchten Verbindungen 10.

Diejenigen der beanspruchten Verbindungen, bei denen die Gruppe A der allgemeinen Formel I eine Alkinyl oder Alkenylgruppe gemäß den Strukturformeln 19 und 20 ist, sind - wie in Schema 3 gezeigt - ausgehend von den literaturbekannten, monobenzoylierten Hydrochinonen 14 zugänglich.

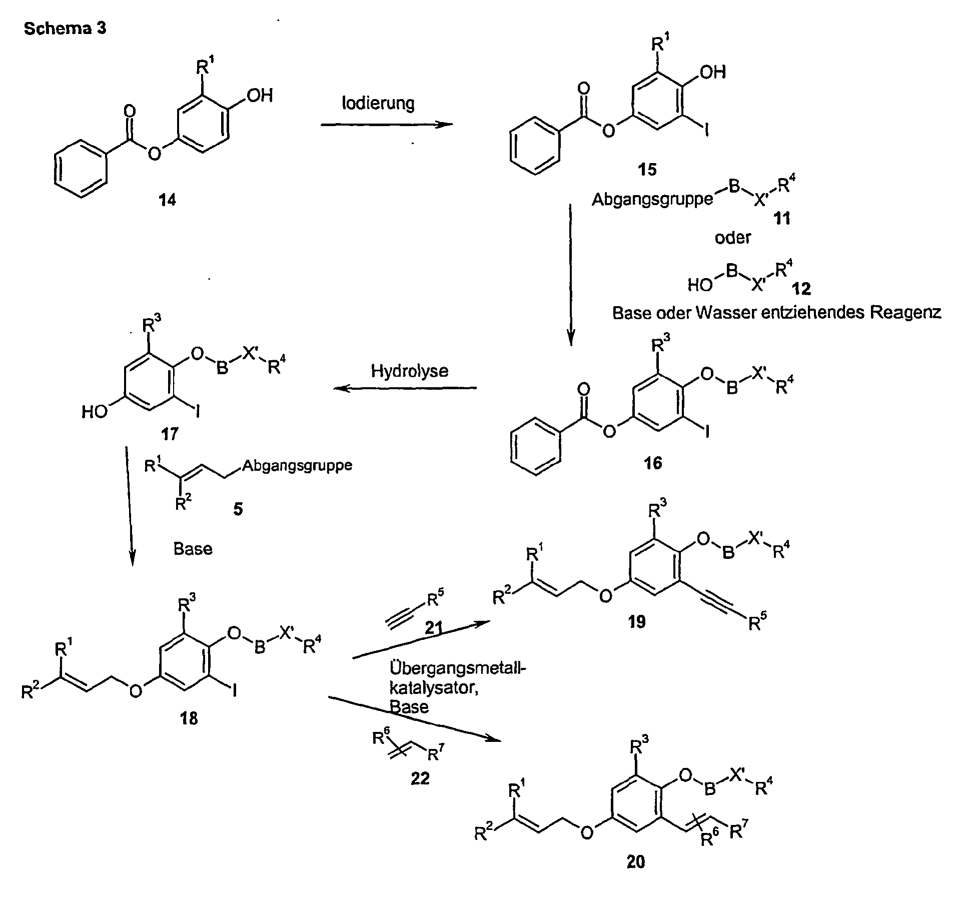

Die monobenzoylierten Hydrochinone 14 werden zunächst unter Einwirkung eines geeigneten Reagenzes selektiv in ortho-Stellung zur freien Phenolgruppe bromiert oder iodiert. Eine lodierung gelingt unter anderem mit Hilfe eines Gemisches aus Chloramin T und Natriumiodid, wobei die Strukturen 15 erhalten werden. 15 wird über eine Alkylierung oder eine Kondensation mit den Bausteinen 11 oder 12 (analog der Umsetzung von 4 zu 9) zu den Verbindungen 16 umgesetzt. Die Benzoylgruppe in 16 wird in einem basischen Reaktionsmedium hydrolytisch abgespalten. Basische Reaktionsbedingungen können dabei insbesondere durch anorganische Basen, wie zum Beispiel Natriumhydroxid oder Kaliumcarbonat, eingestellt werden. Die dadurch entstandenen Phenole 17 werden mit einem Baustein 5 und einer beliebigen Base alkyliert. Die letztendliche Einführung einer Alkinylgruppe zu den beanspruchten Verbindungen 19 oder einer Alkenylgruppe zu den beanspruchten Verbindungen 20 erfolgt durch Reaktion mit geeigneten Alkinen 21 beziehungsweise geeigneten Alkenen 22, einem Übergangsmetallkatalysator und einer beliebigen Base. Als Übergangsmetallkatalysator kommen insbesondere Palladiumkomplexe oder Gemische aus Palladiumkomplexen und anorganischen Kupferverbindungen zum Einsatz.

Diejenigen der beanspruchten Verbindungen oder Intermediate, bei denen die Gruppe A der allgemeinen Formel I beziehungsweise der Strukturen 4, 6 oder 7 eine Acetylgruppe oder eine Alkoxycarbonylgruppe ist, können Ausgangspunkt für weitere Abwandlungen der Gruppe A unter Verwendung von Standardreaktionen der Organischen Chemie sein. Dies ist beispielhaft in Schema 4 dargestellt.

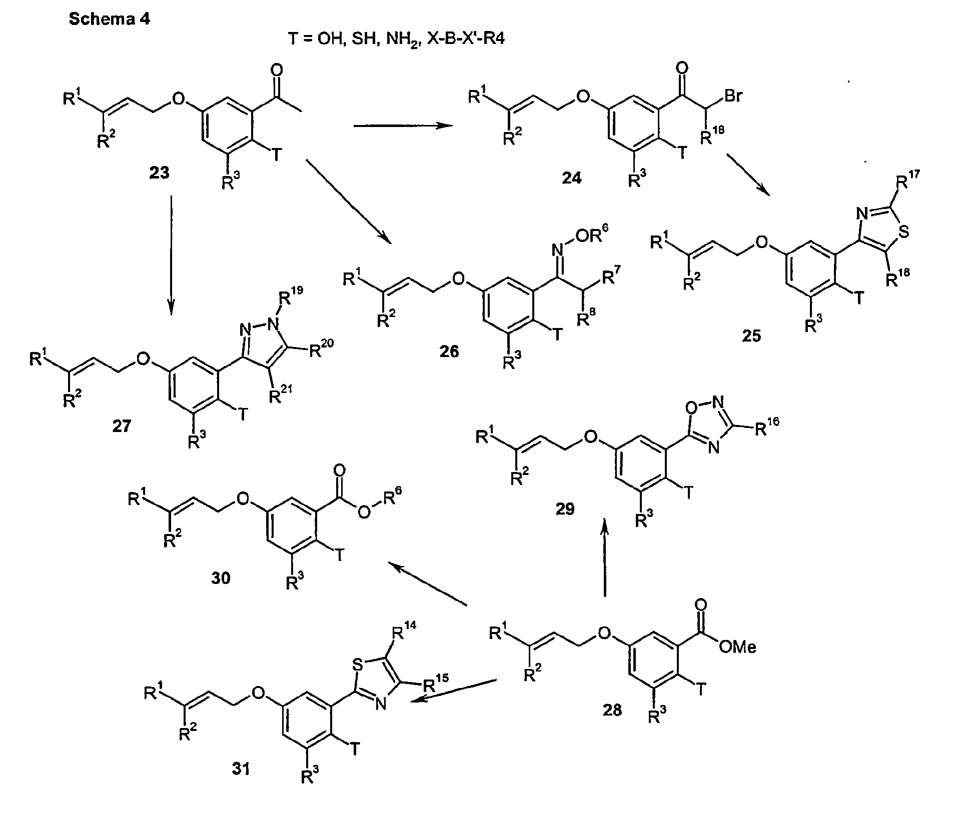

Beispielsweise kann eine Verbindung der Struktur 23 in α-Stellung zur Carbonylgruppe alkyliert werden und / oder bromiert werden. Zur Alkylierung stehen in der Literatur eine Reihe von Verfahren zur Verfügung, von denen hier exemplarisch die Alkylierung von metallierten Hydrazonen gemäß Chem. Ber. 1978, 111, 1337 - 1361 genannt sei. Eine Bromierung gelingt durch Reaktion mit Brom oder einer Vielzahl möglicher Bromierungsreagenzien meist in einem inerten Lösungsmittel. Die so erhaltenen Bromketone können mit primären Thioamiden umgesetzt werden, wobei Thiazole der Formel 25 erhalten werden. Die Umsetzung von Verbindungen der Formel 23 mit Hydroxylaminen oder ihrer Salze liefert Oxime, die beliebig in α-Stellung zum Beispiel durch Alkylierung weiter abgewandelt werden können zu den Strukturen vom Typ 26. Die Reaktion der Acylgruppe in 23 mit geeigneten Amidacetalen wie zum Beispiel N,N-Dimethylformamid-O,O-dimethylacetal liefert ein Enamino-Keton als Intermediat, dessen weitere Umsetzung mit beliebigen Hydrazinen zu Strukturen vom Typ 27 führt.

Verbindungen der allgemeinen Struktur 28 können mit beliebigen Amidoximen zur Reaktion gebracht werden, wodurch 1,2,4-Oxadiazole der Formel 28 erhalten werden. Diese Umsetzung kann in einem Schritt unter Verwendung einer starken Base meist in einem protischen Lösungsmittel erfolgen oder in zwei Schritten über die entsprechenden, offenkettigen O-Acylamidoxime als Zwischenstufe. Die Umesterung von 28 mit beliebigen Alkoholen nach einem Standardverfahren wie es zum Beispiel in Org. Syn. Coll. Vol. 1955, 3,146 beschrieben ist liefert die entsprechenden Ester 30. Gemäß dem in Org. Coll. Vol. 1955, 3, 516, 536 beschriebenen Verfahrens oder eines beliebigen äquivalenten Verfahrens kann der Ester der Verbindungen 28 in ein primäres Amid überführt werden. Mit Hilfe eines geeigneten Thiolierungsreagenzes wie zum Beispiel Lawesson's Reagenz (Tetrahedron 1985, 41, 5061) werden die entsprechenden Thioamide erhalten, deren weitere Umsetzung mit beliebigen α-Haloketonen oder α-Haloaldehyden zu den Thiazolen vom Typ 31 führt. Die Hydrolyse der Esterfunktionalität in 28 unter basischen oder sauren, protischen Bedingungen liefert die entsprechenden Säuren, die beispielsweise im Sinne einer Curtius-Umlagerung abgebaut werden. Hierzu eignet sich unter anderem die Einwirkung von Diphenylphosphorylazid (DPPA) wie in J. Am. Chem. Soc. 1972, 94, 6203 beschrieben. Erfolgt diese Umlagerung in Gegenwart eines geeigneten Alkohols R³-OH werden die Carbamate 32 erhalten. Die Gegenwart eines primären oder sekundären Amins ergibt die entsprechenden Harnstoffe als Produkte.

Kollektionen aus Verbindungen der Formel 1, die nach oben genanntem Schema synthetisiert werden können, können zusätzlich in parallelisierter Weise hergestellt werden , wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisiert. Insgesamt wird hierunter eine Vorgehensweise verstanden, wie sie beispielsweise durch S. H. DeWitt in "Annual Reports in Combinatorial Chemistry and Molecular Diversity: Automated synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen der allgemeinen Formel I beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise Roboter bedient werden. Derartige Automationssysteme können zum Beispiel von der Firma Zymark Corporation, Zymark Center, Hopkinton, MA 01748, USA bezogen werden.

Neben dem hier beschriebenen kann die Herstellung von Verbindungen der allgemeinen Formel I vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.

Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel l in Form von Substanzkollektionen, die Bibliotheken genannt werden.

Gegenstand der vorliegenden Erfindung sind auch Bibliotheken, die mindestens 2 Verbindungen der Formel I.

Die Verbindungen der Formel (I) eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.. Aus der Ordnung der Isopoda z.B. Oniscus aselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung des Isoptera z.B. Reticulitermes spp..
Aus der Ordnung der Anoplura z.B. Phylloera vastatrix, Pemphigus spp., Pediculus humanus corporis, Haematopinus spp., Linognathus spp..
Aus der Ordnung der Mallophaga z.B. Trichodectes pp., Damalinea spp..
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp..
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopsis, Ceratophyllus spp..
Aus der Ordnung der Arachnida z.B. Scorpio maurus, Latrodectus mactans.
Aus der Klasse der Helminthen z.B. Haemonchus, Trichostrongulus, Ostertagia, Cooperia, Chabertia, Strongyloides, Oesophagostomum, Hyostrongulus, Ancylostoma, Ascaris und Heterakis sowie Fasciola.
Aus der Klasse der Gastropoda z.B. Deroceras spp., Arion spp., Lymnaea spp., Galba spp., Succinea spp., Biomphalaria spp., Bulinus spp., Oncomelania spp..
Aus der Klasse der Bivalva z.B. Dreissena spp..

Zu den pflanzenparasitären Nematoden, die erfindungsgemäß bekämpft werden können, gehören beispielsweise die wurzelparasitären Bodennematoden wie z.B. solche der Gattungen Meloidogyne (Wurzelgallennematoden, wie Meloidogyne incognita, Meloidogyne hapla und Meloidogyne javanica), Heterodera und Globodera (zystenbildende Nematoden, wie Globodera rostochiensis, Globodera pallida, Heterodera trifolii) sowie der Gattungen Radopholus wie Radopholus similis, Pratylenchus wie Pratyglenchus neglectus, Pratylenchus penetrans und Pratylenchus curvitatus;
Tylenchulus wie Tylenchulus semipenetrans, Tylenchorhynchus, wie Tylenchorhynchus dubius und Tylenchorhynchus claytoni, Rotylenchus wie Rotylenchus robustus, Heliocotylenchus wie Haliocotylenchus multicinctus, Belonoaimus wie Belonoaimus longicaudatus, Longidorus wie Longidorus elongatus, Trichodorus wie Trichodorus primitivus und Xiphinema wie Xiphinema index.

Ferner lassen sich mit den erfindungsgemäßen Verbindungen die Nematodengattungen Ditylenchus (Stengelparasiten, wie Ditylenchus dipsaci und Ditylenchus destructor), Aphelenchoides (Blattnematoden, wie Aphelenchoides ritzemabosi) und Anguina (Blütennematoden, wie Anguina tritici) bekämpfen.

Die Erfindung betrifft auch Mittel, beispielsweise Pflanzenschutzmittel, vorzugsweise insektizide, akarizide, ixodizide, nematizide, molluskizide oder fungizide, besonders bevorzugt insektizide und akarizide Mittel, die eine oder mehrere Verbindungen der Formel (I) neben geeigneten Formulierungshilfsmitteln enthalten.

Zur Herstellung der erfindungsgemäßen Mittel gibt man den Wirkstoff und die weiteren Zusätze zusammen und bringt sie in eine geeignete Anwendungsform.

Die Erfindung betrifft auch Mittel, insbesondere insektizide und akarizide Mittel, die die Verbindungen der Formel (I) neben geeigneten Formulierungshilfsmitteln enthalten.

Die erfindungsgemäßen Mittel enthalten die Wirkstoffe der Formeln (I) im allgemeinen zu 1 bis 95 Gew.-%. Sie können auf verschiedene Art formuliert werden, je nachdem wie es durch die biologischen und/oder chemischphysikalischen Parameter vorgegeben ist. Als Formulierungsmöglichkeiten kommen daher beispielsweise in Frage:
Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und beispielsweise beschrieben in: Winnacker-Küchler, "Chemische Technologie", Band 7, C. Hanser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel, d.h. Träger- und/oder oberflächenaktive Stoffe, wie Inertmaterialien, Tenside, Lösungsmittel und weitere Zusatzstoffe sind ebenfalls bekannt und beispielsweise beschrieben in: Watkins, "Handbook of Insecticide Dust Diluents and Garriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., lnterscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Cadodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise gewünschtenfalls in Mischung mit Düngemitteln - granuliert werden.

In Spritzpulvern beträgt die Wirkstoffkonzentration üblicherweise etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,0005 und 10,0 kg/ha oder mehr Aktivsubstanz, vorzugsweise liegt sie jedoch zwischen 0,001 und 5 kg/ha Wirkstoff.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:
1. aus der Gruppe der Phosphorverbindungen
   Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Fonofos, Formothion, Fosthiazate (ASC-66824) Heptenophos, lsazophos, lsothioate, lsoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos,Tetrachlorvinphos, Thiometon, Triazophos, Trichlorphon, Vamidothion;
2. aus der Gruppe der Carbamate
   Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneamino)-N-methyl-N-(morpholinothio)carbamate (UC 51717), Triazamate;
3. aus der Gruppe der Carbonsäureester
   Acrinathrin, Allethrin, Alphametrin, 5-Benzyl-3-furylmethyl-(E)-(1 R)-cis-2,2-dimethyl-3-(2-oxothiolan-3-ylidenemethyl)cyclopropanecarboxylate, Beta-Cyfluthrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1 RS)-trans-3-(4-tert.butylphenyl)-2,2-dimethylcyclopropanecarboxylate (NCI 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, ABG-9008, Acetamiprid, Anagrapha falcitera, AKD-1022, AKD-3059, ANS-118, Bacillus thuringiensis, Beauveria bassianea, Bensultap, Bifenazate (D-2341), Binapacryl, BJL-932, Bromopropylate, BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden (Thiamethoxam), Diafenthiuron, N-(3,5-Dichlor-4-(1,1,2,3,3,3-hexafluor-1-propyloxy) phenyl)carbamoyl)-2-chlorbenzcarboximidsäureethylester, DDT, Dicofol, Diflubenzuron, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Endosulfan, Ethiprole (Sulfethiprole), Ethofenprox, Etoxazole (YI-5301), Fenazaquin, Fenoxycarb, Fipronil, Fluazuron, Flumite (Flufenzine, SZI-121), 2-Fluro-5-(4-(4-ethoxyphenyl)-4-methyl-1-pentyl)diphenylether (MTI 800), Granulose- und Kernpolyederviren, Fenpyroximate, Fenthiocarb, Flubenzimine, Flucycloxuron, Flufenoxuron, Flufenprox (ICI-A5683), Fluproxyfen, Gamma-HCH, Halofenozide (RH-0345), Halofenprox (MTI-732), Hexaflumuron (DE 473), Hexythiazox, HOI-9004, Hydramethylnon (AC 217300), Lufenuron, Imidacloprid, Indoxacarb (DPX-MP062), Kanemite (AKD-2023), M-020, MTI-446, Ivermectin, M-020, Methoxyfenozide (Intrepid, RH-2485), Milbemectin, NC-196, Neemgard, Nitenpyram (Tl-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-1111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242, SI-8601, Silafluofen, Silbmadine (CG-177), Spinosad, SU-9118, Tebufenozide, Tebufenpyrad (MK-239), Teflubenzuron, Tetradifon, Tetrasul, Thiacloprid, Thiocyclam, TI-435, Tolfenpyrad (OMI-88), Triazamate (RH-7988), Triflumuron, Verbutin, Vertalec (Mykotal), YI-5301,

Die oben genannten Kombinationspartner stellen bekannte Wirkstoffe dar, die zum großen Teil in Ch.R Worthing, S.B. Walker, The Pesticide Manual, 11. Auflage, British Crop Protection Council Farnham, 1997 beschrieben sind.

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Gegenstand der Erfindung sind daher auch die Verwendung der Verbindungen der Formel (I) oder ihrer Salze zur Bekämpfung von tierischen Schädlingen und ein Verfahren zur Bekämpfung von tierischen Schädlingen, wobei man die tierischen Schädlinge direkt oder indirekt in Kontakt mit einer Verbindung der Formel (I) oder deren Salze liegt.

Die erfindungsgemäßen Wirkstoffe eignen sich auch zur Bekämpfung von Endo- und Ektoparasiten auf dem human- und veterinärmedizinischen Gebiet bzw. auf dem Gebiet der Tierhaltung. Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen Verbindungen der Formel (I) können demgemäß auch besonders vorteilhaft zur Behandlung von Warmblütlem, insbesondere in der Viehhaltung (z.B. Rinder, Schafe, Schweine und Geflügel wie Hühner, Gänse usw.) eingesetzt werden. In einer bevorzugten Ausführungsform der Erfindung werden den Tieren die Verbindungen, gegebenenfalls in geeigneten Formulierungen und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die Verbindungen können bei Rindern z.B. in Dosierungen von 0,01 bis 1 mg/kg Körpergewicht eingesetzt werden.

Neben lethaler Wirkung auf Schädlinge zeichnen sich die Verbindungen der Formel (I) oder deren Salze auch durch einen ausgeprägten Repellenteffekt aus.

Repellent im Sinne der Beschreibung ist ein Stoff oder Stoffgemisch, das abwehrend oder vertreibend auf andere Lebewesen, insbesondere Schädlinge und Lästlinge wirkt. Der Begriff umfaßt dabei auch Effekte wie den Antifeeding-Effekt, wobei die Nahrungsaufnahme gestört oder verhindert wird (fraßabweisender Effekt), Unterdrückung der Eiablage oder eine Beeinflussung der Populationsentwicklung.

Gegenstand der Erfindung ist daher auch die Verwendung von Verbindungen der Formel (I) oder deren Salze zur Erzielung der genannten Effekte, insbesondere bei den in den biologischen Beispielen benannten Schädlingen.

Gegenstand der Erfindung ist auch ein Verfahren zur Abwehr oder zur Vertreibung von Schadorganismen, wobei man eine oder mehrere Verbindungen der Formel (I) oder deren Salze an dem Ort ausbringt, von dem die Schadorganismen ferngehalten oder vertrieben werden sollen.

Ausbringen kann im Falle einer Pflanze beispielsweise eine Behandlung der Pflanze oder auch des Saatguts bedeuten.

Es ist, was die Beeinflussung von Populationen angeht, von Intereesse, dass die Effekte auch hintereinander bei der Entwicklung einer Population beobachtet werden, wobei sie sich auffaddieren können. Hierbei kann der Einzeleffekt selbst nur einen Wirkungsgrad von deutlich unter 100% haben und insgesamt am Ende kann doch eine 100%ige Wirkung erreicht werden.

Außerdem zeichnen sich die Verbindungen der Formel (I) oder ihre Saize dadurch aus, dass man - will man die oben angeführten Effekte ausnutzen, - zu einem früheren Zeitpunkt als bei einer direkten Bekämpfung üblich das Mittel appliziert. Der Effekt hält häufig lange Zeit an, so dass eine Wirkungsdauer von mehr als 2 Monaten erreicht wird.

Die Effekte treten bei Insekten, Spinnentieren und den anderen der oben erwähnten Schädlinge auf.

Neben den bisher genannten Applikationsverfahren zeigen die erfindungsgemässen Wirkstoffe der Formel (I) eine hervorragende systemische Wirkung. Die Wirkstoffe können daher auch über Pflanzenteile, unterirdische wie oberirdische (Wurzel, Stengel, Blatt), in die Pflanzen eingebracht werden, wenn die Wirkstoffe in flüssiger oder fester Form in die direkte Umgebung der Pflanze appliziert werden (z.B. Granulate in der Erdapplikation, Applikation in gefluteten Reisfeldern).

Daneben sind die erfindungsgemäßen Wirkstoffe in besonderer Weise zu Behandlung von vegetativen und generativen Vermehrungsmaterial einsetzbar, wie z.B. von Saatgut von beispielsweise Getreide, Gemüse, Baumwolle, Reis, Zuckerrübe und anderen Kultur- und Zierpflanzen, von Zwiebeln, Stecklingen und Knollen weiterer vegetativ vermehrter Kultur- und Zierpflanzen. Die Behandlung hierfür kann vor der Saat bzw. dem Pflanzvorgang erfolgen (z.B. durch spezielle Techniken des Seedcoatings, durch Beizung in flüssiger oder fester Form oder Seedboxtreatment), während des Saatvorgangs bzw. des Pflanzens oder nach dem Saat- bzw. Pflanzvorgang durch spezielle Applikationstechniken (z.B. Saatreihenbehandlung). Die angewandte Wirkstoffmenge kann entsprechend der Anwendung in einem größerem Bereich schwanken. Im allgemeinen liegen die Aufwandmengen zwischen 1 g und 10 kg Wirkstoff pro Hektar Bodenfläche.

Die Verbindungen der Formel (I) können auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten, wie bestimmten Insekten oder Mikroorganismen, wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung in wirtschaftlich bedeutenden transgenen Kulturen von Nutz- und Zierpflanzen, z.B. von Getreide, wie Weizen, Gerste, Roggen, Hafer, Hirse, Reis, Maniok und Mais, oder auch Kulturen von Zuckerrübe, Baumwolle, Soja, Raps, Kartoffel, Tomate, Erbse und anderen Gemüsesorten.

Bei der Anwendung in transgenen Kulturen, insbesondere mit Insektenresistenzen treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Schädlingsspektrum, das bekämpft werden kann oder veränderte Aufwandmengen, die für die Applikation eingesetzt werden können.

Gegenstand der Erfindung ist deshalb auch die Verwendung von Verbindungen der Formel (I) zur Bekämpfung von Schadorganismen in transgenen Kulturpflanzen und ein Verfahren zur Bekämpfung von Schadorganismen in transgenen Kulturpflanzen, wobei man die Schadorganismen direkt oder indirekt in Kontakt mit einer Verbindung der Formel (I) bringt.

Die Anwendung der erfindungsgemäßen Verbindungen beinhaltet neben direkter Applikation auf die Schädlinge jede andere Applikation, bei der Verbindungen der Formel (I) auf die Schädlinge wirken. Solche indirekten Applikationen können beispielsweise in der Anwendung von Verbindungen liegen, die, beispielsweise im Boden, der Pflanze oder dem Schädling, zu Verbindungen der Formel (I) zerfallen oder abgebaut werden.
Nachfolgende Beispiele dienen zur Erläuterung der Erfindung.

### A. Chemische Beispiele

### Beispiel A: 3-Acetyl-1-chlor-5-(3,3-dichlorprop-2-enyloxy)-2-[3-(5-trifluormethylpyrid-2-yloxy)-propyloxy]benzol (Nr. 27)

### 1. Stufe: 3-Acetyl-1-chlor-2,5-dihydroxybenzol

8,7 g 3-Acetyl-1-chlor-2-hydroxy-5-methoxybenzol wurden in 150 ml Bromwasserstoffsäure (48%ig in Wasser) aufgenommen. Es wurden 1,69 g roter Phosphor zugegeben und anschließend 15 Stunden lang bei 85°C gerührt. Nach dem Abkühlen wurde das Reaktionsgemisch in ca. 1 Liter eiskaltes Wasser gegeben und mit Essigsäureethylester extrahiert. Das nach Trocknen und Einengen der Essigsäureethylesterphase erhaltene Rohpropukt wurde säulenchromatographisch gereinigt. Ausbeute: 2,9 g (36%)

¹H-NMR(CDCl₃): δ = 2,61 (s, 3H); 4,73 (s,1H); 7,18 (m, 2H), 12,33 (s,1H).

### 2. Stufe: 3-Acetyl-2,5-bis-(3,3-dichlorprop-2-enyloxy)-1-chlorbenzol

2,9 g 3-Acetyl-1-chlor-2,5-dihydroxybenzol wurden in 29 ml Aceton gelöst und mit 3,39 g 1,1,3-Trichlorpropen, 4,3 g Kaliumcarbonat und 0,7 g Natriumiodid versetzt. Es wurde 5 Stunden lang unter Rückfluss erhitzt (DC-Kontrolle). Anschließend wurde die Reaktionsmischung abkühlen gelassen, am Rotationsverdampfer eingeengt und das so erhaltene Rohprodukt einer chromatographischen Reinigung unterworfen. Ausbeute: 6,1 g (99%)

¹H-NMR (CDCl₃): δ = 2,60 (s, 3H); 4,58 (d, 2H); 4,62 (d, 2H); 6,12 (t,1H); 6,21 (t,1 H); 7,01 (d,1H); 7,09 (d,1H).

### 3. Stufe: 3-Acetyl-1-chlor-5-(3,3-dichlorprop-2-eny)oxy)-2-hydroxybenzol

6,1 g 3-Acetyl-2,5-bis-(3,3-dichlorprop-2-enyloxy)-1-chlorbenzol wurden in 160 ml Toluol gelöst. Zu dieser Lösung wurden 7,79 g Magnesiumdibromid-Diethylether-Komplex gelöst in 25 ml Diethylether gegeben und anschließend 6 Stunden lang bei 85°C gerührt. Ist die Reaktion gemäß DC-Kontrolle beendet wurde der Ansatz abkühlen gelassen, mit etwa 300 ml Wasser versetzt und die organische Phase abgetrennt. Die Wasserphase wurde zweimal mit Essigester nachextrahiert und anschließend wurden die vereinigten organischen Phasen mit gesättigter Kochsalzlösung gewaschen. Nach Trocknen und Einengen der organischen Phase wurde das Rohprodukt chromatographisch gereinigt. Ausbeute: 2,86 g (64%)

¹ H-NMR (CDCl₃): δ = 2,62 (s, 3H); 4,62 (d, 2H); 6,13 (t,1H); 7,17 (d,1H), 7,22 (d, 1 H); 12,40 (s,1H).

### 4. Stufe: 3-Acetyl-1-chlor-5-(3,3-dichlorprop-2-enyloxy)-2-[3-(5-trifluormethylpyrid-2-yloxy)-propyloxy]benzol

1,2 g 3-Acetyl-1-chlor-5-(3,3-dichlorprop-2-enyloxy)-2-hydroxybenzol, 0,9 g 3-(5-Trifluormethylpyrid-2-yloxy)propan-1-ol und 1,12 g Triphenylphosphin wurden unter Schutzgasatmosphäre in 35 ml trockenem Tetrahydrofuran (THF) gelöst. Unter Eisbadkühlung wurden 0,74 g Diethylazodicarboxylat zugetropft. Nach ca. 30 Minuten wurde das Eisbad entfernt und es wird 15 Stunden lang bei Raumtemperatur nachgerührt. Der Ansatz wurde nun im Vakuum eingeengt und der Rückstand in einer Mischung aus Essigsäureethylester und Wasser aufgenommen. Nach Schütteln wurde die organische Phase isoliert, getrocknet und im Vakuum eingeengt. Die Reinigung des Rohproduktes erfolgte chromatographisch. Ausbeute: 1,52 g (74%)

¹H-NMR (CDCl₃): δ = 2,27 (m, 2H); 2,6 (s, 3H); 4,08 (t, 2H); 4,60 (m, 4H); 6,12 (t, 1 H); 6,81 (d,1H); 6,99 (d,1H); 7,08 (d,1H); 7,77 (dd,1H); 8,42 (br. s, 1H).

### Beispiel B: 1-Chlor-5-(3,3-dichlorprop-2-enyloxy)-3-(3-ethyl-1,2,4-oxadiazol-5-yl)-2-[3-(5-trifluormethylpyrid-2-yloxy)-propyloxy]benzol (Nr. 356)

### 1. Stufe: 3-Chlor-2,5-dihydroxybenzoesäure

9,95 g 3-Chlor-2-hydroxy-5-methoxybenzoesäuremethylester wurden in 300 ml Bromwasserstoffsäure (48%ig in Wasser) aufgenommen. Es wurden 1,79 g roter Phosphor zugegeben und anschließend 15 Stunden lang bei 85°C gerührt. Nach dem Abkühlen wurde das Reaktionsgemisch in ca. 1 Liter eiskaltes Wasser gegeben und mit Essigsäureethylester extrahiert. Das nach Trocknen und Einengen der Essigsäureethylesterphase erhaltene Rohpropukt wurde ohne weitere Reinigung in die nächste Stufe eingesetzt.

### 2. Stufe: 3-Chlor-2,5-dihydroxybenzoesäuremethylester

8,65 g des Rohproduktes der vorhergehenden Stufe wurden in 12 ml wasserfreiem Methanol gelöst und mit 0,5 ml konzentrierter Schwefelsäure versetzt. Es wurde 6 Stunden unter Rückfluss erhitzt. Nach dem Abkühlen wurde das Reaktionsgemisch im Vakuum eingeengt, mit tert-Butyl-methylether auf etwa 200 ml verdünnt und mit Wasser und gesättigter Natriumhydrogencarbonatlösung gewaschen. Die Etherphase wurde getrocknet, eingeengt und das Rohprodukt chromatographisch gereinigt. Ausbeute: 4,85 g (52% über 2 Stufen)

¹ H-NMR (CDCl₃): δ = 3, 97 (s, 3H); 4,61 (br. s,1H); 7,15 (d,1H); 7,22 (d,1H); 10,83 (s,1H).

3. Stufe: 3-Chlor-5-(3,3-dichlorprop-2-enyloxy)-2-hydroxybenzoesäuremethylester 1,67 g 3-Chlor-2,5-dihydroxybenzoesäuremethylester, 2 g 1,1,3-Trichlorpropen, 2,27 g Kaliumcarbonat und 0,37 g Natriumiodid wurden in 17 ml Aceton gelöst und 16 Stunden lang bei 50°C gerührt. Nach dem Abkühlen wurde der Ansatz im Vakuum eingeengt, das Rohprodukt in Essigsäureethylester aufgenommen und mit Wasser gewaschen. Trocknen und Einengen der Essigesterphase lieferte ein Rohprodukt, das in 65 ml Toluol gelöst wurde. Es wurden 2,95 g Magnesiumdibromid-Diethylether-Komplex in 10 ml Diethylether gelöst zupipettiert und 20 Stunden lang auf 80°C bis 85°C erhitzt. Der Ansatz wurde abkühlen gelassen, mit etwa 300 ml Wasser versetzt und die organische Phase abgetrennt. Die Wasserphase wurde zweimal mit Essigester nachextrahiert und anschließend wurden die vereinigten organischen Phasen mit gesättigter Kochsalzlösung gewaschen. Nach Trocknen und Einengen der organischen Phase wurde das Rohprodukt chromatographisch gereinigt. Ausbeute: 2,01 g (79%)

¹H-NMR (CDCl₃): δ = 3,99 (s, 3H); 4,60 (d, 2H); 6,12 (t,1H); 7,20 (d,1H), 7,25 (d, 1 H); 10,95 (s,1H).

### 4. Stufe: 3-Chlor-5-(3,3-dichlorprop-2-enyloxy)-2-[3-(5-trifluormethylpyrid-2-yloxy)-propyloxy]benzoesäuremethylester (Nr. 39)

1,85 g 3-Chlor-5-(3,3-dichlorprop-2-enyloxy)-2-hydroxybenzoesäuremethylester, 1,3 g 3-(5-trifluormethylpyrid-2-yloxy)propan-l-ol und 1,64 g Triphenylphosphin wurden unter Schutzgasatmosphäre in 50 ml trockenem Tetrahydrofuran (THF) gelöst. Unter Eisbadkühlung wurden 1,09 g Diethylazodicarboxylat zugetropft. Nach ca. 30 Minuten wurde das Eisbad entfernt und es wurde 15 Stunden lang bei Raumtemperatur nachgerührt. Der Ansatz wurde nun im Vakuum eingeengt und der Rückstand in einer Mischung aus Essigsäureethylester und Wasser aufgenommen. Nach Schütteln wurde die organische Phase isoliert, getrocknet und im Vakuum eingeengt. Die Reinigung des Rohproduktes erfolgte chromatographisch. Ausbeute: 1,79 g (59%)

¹H-NMR (CDCl₃): δ = 2,30 (m, 2H); 3,86 (s, 3H); 4,16 (t, 2H); 4,62 (m, 4H); 6,13 (t, 1 H); 6,80 (d,1H); 7,10 (d,1H); 7,21 (d,1H); 7,77 (dd,1H); 8,42 (br. s, 1H).

### 5. Stufe: 1-Chlor-5-(3,3-dichlorprop-2-enyloxy)-3-(3-ethyl-1,2,4-oxadiazol-5-yl)-2-[3-(5-trifluormethylpyrid-2-yloxy)-propyloxy]benzol

0,05 g 3-Chlor-5-(3,3-dichlorprop-2-enyloxy)-2-[3-(5-trifluormethylpyrid-2-yloxy)-propyloxy]benzoesäuremethylester und 0,028 g Propionsäureamidoxim wurden in 1 ml wasserfreiem Ethanol gelöst. Zu dieser Lösung wurde eine Lösung aus 0,008 g Natrium in 0,5 ml Ethanol gegeben. Es wurde 2 Stunden bei 40°C und 20 Stunden bei Raumtemperatur gerührt. Anschließend wurde der Ansatz eingeengt und mit Diethylether und Wasser versetzt. Nach Schütteln wurde die Etherphase isoliert, getrocknet, im Vakuum eingeengt und das Rohprodukt chromatographisch gereinigt. Ausbeute: 0,017 g (32%)

¹ H-NMR (CDCl₃): δ = 1,40 (t, 3H); 2,38 (m, 2H); 2,82 (q, 2H); 4,19 (t, 2H); 4,66 (m, 4H); 6,15 (t,1H); 6,80 (d,1H); 7,17 (d, 1H); 7,47 (d,1H); 7,76 (dd,1H); 8,43 (br. s,1H).

### Beispiel C: 3-Acetyl-1-chlor-5-(3,3-dichlorprop-2-enyloxy)-2-[4-(5-trifluormethylpyrid-2-yloxy)-butyloxy]benzol (Nr. 59)

0,1 g 3-Acetyl-1-chlor-5-(3,3-dichlorprop-2-enyloxy)-2-hydroxybenzol, 0,08 g 4-(5-Trifluormethylpyrid-2-yloxy)butan-1-ol und 0,093 g Triphenylphosphin wurden unter Schutzgasatmosphäre in 2 ml trockenem Tetrahydrofuran (THF) gelöst. Unter Eisbadkühlung wurden 56 µl Diethylazodicarboxylat zugetropft. Nach ca. 30 Minuten wurde das Eisbad entfernt und es wurde 15 Stunden lang bei Raumtemperatur nachgerührt. Der Ansatz wurde nun im Vakuum eingeengt und der Rückstand in einer Mischung aus Essigsäureethylester und Wasser aufgenommen. Nach Schütteln wurde die organische Phase isoliert, getrocknet und im Vakuum eingeengt. Die Reinigung des Rohproduktes erfolgte chromatographisch. Ausbeute: 0,051 g (35%)

¹H-NMR (CDCl₃): δ = 1,96 (m, 4H); 2,6 (s, 3H); 4,02 (m, 2H); 4,41 (m, 2H); 4,59 (d, 2H); 6,12 (t,1H); 6,80 (d,1H); 7,00 (d,1H); 7,08 (d,1H); 7,78 (dd,1H); 8,42 (br. s,1H).

### Beispiel D: 1-Chlor-3-(2-chlorphenylethinyl)-5-(3,3-dichlorprop-2-enyloxy)2-[3-(5-trifluormethylpyrid-2-yloxy)-propyloxy]benzol (Nr. 226)

### 1. Stufe: 5-Benzoyloxy-3-chlor-2-hydroxy-1-iodbenzol

3 g 5-Benzoyloxy-1-chlor-2-hydroxybenzol wurden in 35 ml DMF gelöst. Es wurden nacheinander 2,17 g Natriumiodid und 3,29 g Chloramin T zugegeben. Nach einstündigem Rühren bei Raumtemperatur wurde der Ansatz mit etwa 500 ml Wasser versetzt und mit Essigsäureethylester extrahiert. Die organische Phase wurde mit 5%iger Natriumthiosulfatlösung gewaschen, getrocknet und am Rotationsverdampfer eingeengt. Das so erhaltene Produkt war sauber genug für die weitere Umsetzung. Ausbeute: 3,81 g (84%)

¹ H-NMR (CDCl₃): δ = 4,70 (br. s,1H); 7,30 (d,1H); 7,54 (m, 3H); 7,63 (m,1H); 8,17 (d, 2H).

### 2. Stufe: 5-Benzoyloxy-3-chlor-1-iod-2-[3-(5-trifluormethylpyrid-2-yloxy)-propyloxy]benzol

3,5 g 5-Benzoyloxy-3-chlor-2-hydroxy-1-iodbenzol, 2,07 g 3-(5-Trifluormethylpyrid-2-yloxy)propan-1-ol und 2,57 g Triphenylphosphin wurden unter Schutzgasatmosphäre in 100 ml trockenem Tetrahydrofuran (THF) gelöst. Unter Eisbadkühlung wurden 1,71 g Diethylazodicarboxylat zugetropft. Nach ca. 30 Minuten wurde das Eisbad entfernt und es wurde 15 Stunden lang bei Raumtemperatur nachgerührt. Der Ansatz wurde nun im Vakuum eingeengt und der Rückstand in einer Mischung aus Essigsäureethylester und Wasser aufgenommen. Nach Schütteln wurde die organische Phase isoliert, getrocknet und im Vakuum eingeengt. Die Reinigung des Rohproduktes erfolgte chromatographisch. Ausbeute: 3,12 g (58%)

¹ H-NMR (CDCl₃): δ = 2,38 (quint., 2H); 4,19 (t, 2H); 4,65 (t, 2H); 6,82 (d,1H); 7,31 (d,1H); 7,53 (m, 2H); 7,58 (d,1H); 7,65 (m,1H); 7,78 (dd,1H); 8,16 (d,1H); 8,43 (br. s,1H).

### 3. Stufe: 3-Chlor-5-hydroxy -1-iod-2-[3-(5-trifluormethylpyrid-2-yloxy)-propyloxy]-benzol

3,12 g 5-Benzoyloxy-3-chlor-1-iod-2-[3-(5-trifluormethylpyrid-2-yloxy)-propyloxy]benzol wurden in 16 ml Tetrahydrofuran und 7,8 ml Methanol gelöst und mit 3,9 ml 2N Natronlauge versetzt. Nach 30-minütigem Rühren bei Raumtemperatur wurden 3,9 ml 2N Chlorwasserstoffsäure zugegeben und das Reaktionsgemisch im Vakuum konzentriert. Der Rückstand wurde mit gesättigter Kochsalzlösung und Essigsäureethylester versetzt und geschüttelt. Die Essigesterphase wurde isoliert, getrocknet und eingeengt. Das so erhaltene Rohprodukt wurde chromatographisch gereinigt. Ausbeute: 2,03 g (80%)

¹ H-NMR (CDCl₃): δ = 2,34 (quint., 2H); 4,09 (t, 2H); 4,62 (t, 2H); 5,13 (s,1H); 6,82 (d,1H); 6,88 (d,1H); 7,17 (d,1H); 7,78 (dd,1H); 8,43 (br. s, 1 H).

### 4. Stufe: 3-Chlor-5-(3,3-dichlorprop-2-enyloxy)-1-iod-2-[3-(5-trifluormethylpyrid-2-yloxy)-propyloxy]benzol

2,23 g 3-Chlor-5-hydroxy -1-iod-2-[3-(5-trifluormethylpyrid-2-yloxy)-propyloxy]benzol wurden in 22 ml Aceton gelöst und mit 0,79 g 1,1,3-Trichlorpropen, 1,3 g Kaliumcarbonat und 0,27 g Natriumiodid versetzt. Es wurde 20 Stunden lang bei Raumtemperatur gerührt (DC-Kontrolle). Anschließend wurde die Reaktionsmischung am Rotationsverdampfer eingeengt und das so erhaltene Rohprodukt einer chromatographischen Reinigung unterworfen. Ausbeute: 2,62 g (95%)

¹H-NMR (CDCl₃): δ = 2,35 (quint., 2H); 4,12 (t, 2H); 4,58 (d, 2H); 4,62 (t, 2H); 6,10 (t, 1H); 6,81 (d,1H); 6,92 (d,1H); 7,20 (d,1H); 7,78 (dd,1H); 8,43(br. s, 1H).

### 5. Stufe: 1-Chlor-3-(2-chlorphenylethinyl)-5-(3,3-dichlorprop-2-enyloxy)-2-[3-(5-trifluormethylpyrid-2-yloxy)-propyloxy]benzol

0,1 g 3-Chlor-5-(3,3-dichlorprop-2-enyloxy)-1-iod-2-[3-(5-trifluormethylpyrid-2-yloxy)-propyloxy]benzol, 0,03 g 1-Chlor-2-ethinylbenzol, 0,003 g Kupfer(I)iodid wurden unter Schutzgasatmosphäre in 1 ml Triethylamin gelöst. Es wurden 0,005 g Bis(triphenylphosphin)palladium(II)chlorid zugegeben und 1 Stunde lang bei 70°C gerührt. Anschließend wurde der Ansatz abkühlen gelassen, im Vakuum eingeengt und der resultierende Rückstand einer chromatographischen Aufreinigung unterworfen. Ausbeute: 0,085 g (84%)

¹H-NMR (CDCl₃): δ = 2,35 (quint., 2H); 4,31 (t, 2H); 4,61 (m, 4H); 6,14 (t,1H); 6,73 (d,1H); 6,95 (m, 2H); 7,2 - 7,3 (m, 2H); 7,40 (m,1H); 7,55 (m, 1 H); 7,70 (dd,1H); 8,36 (br. s,1H).

### Beispiel E: 1-Chlor-5-(3,3-dichlorprop-2-enyloxy)- 3-(2Δ-oxazolin-2-yl)-2-[3-(5-trifluormethylpyrid-2-yloxy)-propyloxy]benzol (Nr. 738)

### 1. Stufe: 3-Chlor-5-(3,3-dichlorprop-2-enyloxy)-2-[3-(5-trifluormethylpyrid-2-yloxy)-propyloxy]benzoesäure

1 g 3-Chlor-5-(3,3-dichlorprop-2-enyloxy)-2-[3-(5-trifluormethylpyrid-2-yloxy)-propyloxy]benzoesäuremethylester wurden in 20 ml Tetrahydrofuran und 10 ml Methanol gelöst und mit 2,6 ml 2N Natronlauge versetzt. Nach einstündigem Rühren bei Raumtemperatur wurden 2,6 ml 2N Chlorwasserstoffsäure zugegeben und das Reaktionsgemisch im Vakuum konzentriert. Der Rückstand wurde mit gesättigter Kochsalzlösung und Essigsäureethylester versetzt und geschüttelt. Die Essigesterphase wurde isoliert, getrocknet und eingeengt. Das so erhaltene Rohprodukt wurde chromatographisch gereinigt. Ausbeute: 0,91 g (94%)

¹H-NMR (CDCl₃): δ = 2,39 (m, 2H); 4,32 (t, 2H); 4,62 (m, 4H); 6,16 (t,1H); 6,83 (d, 1 H); 7,18 (d,1H); 7,52 (d,1H); 7,78 (dd,1H); 8,42 (br. s,1H).

### 2. Stufe: 3-Chlor-5-(3,3-dichlorprop-2-enyloxy)-2-[3-(5-trifluormethylpyrid-2-yloxy)-propyloxy]benzoesäure-N-2-hydroxyethylamid (Nr. 33)

0,2 g 3-Chlor-5-(3,3-dichlorprop-2-enyloxy)-2-[3-(5-trifluormethylpyrid-2-yloxy)-propyloxy]benzoesäure und 0,065 g Carbonyldiimidazol wurden 1,5 Stunden lang bei Raumtemperatur in 4 ml wasserfreiem Toluol reagieren gelassen. Anschließend wurden 0,027 g 2-Aminoethanol zugegeben und es wurde eine weitere Stunde bei Raumtemperatur gerührt. Das Reaktionsgemisch wurde im Vakuum eingeengt und säulenchromatographisch gereinigt. Ausbeute: 0,159 g (73%)

¹ H-NMR (CDCI₃): δ = 2,36 (m, 2H); 3,23 (br. s,1H); 3,60 (m, 2H); 3,82 (m, 2H); 4,13 (t, 2H); 4,60 (t, 4H); 4,63 (d, 2H); 6,15 (t,1H); 6,82 (d, 1 H); 7,06 (d,1H); 7,28 (d,1H); 7,79 (dd,1H); 8,20 (br. m,1H); 8,42 (br. s,1H).

### 3. Stufe: 1-Chlor-5-(3,3-dichlorprop-2-enyloxy)- 3-(2Δ-oxazolin-2-yl)-2-[3-(5-trifluormethylpyrid-2-yloxy)-propyloxy]benzol

0,099 g 3-Chlor-5-(3,3-dichlorprop-2-enyloxy)-2-[3-(5-trifluormethylpyrid-2-yloxy)-propyloxy]benzoesäure-N-2-hydroxyethylamid wurden in 1 ml wasserfreiem Toluol gelöst und mit 0,065 g (Methoxycarbonylsulfamoyl)-triethylammonium-N-betain versetzt. Es wurde 1,5 Stunden lang bei 50°C gerührt. Anschließend wurde das Reaktionsgemisch abkühlen gelassen, mit Essigsäureethylester und Wasser versetzt und geschüttelt. Die Essigesterphase wurde isoliert, getrocknet und im Vakuum eingeengt. Das Rohprodukt wurde chromatographisch gereinigt. Ausbeute: 0,018 g (20%)

¹H-NMR (CDCI₃): δ = 2,28 (m, 2H); 4,04 (t, 2H); 4,15 (t, 2H); 4,40 (t, 2H); 4,60 (m, 4H); 6,14 (t,1H); 6,80 (d,1H); 7,03 (d,1H); 7,20 (d,1H); 7,76 (dd,1H); 8,42 (br. s,1H).

### Beispiel F: 1-Chlor-5-(3,3-dichlorprop-2-enyloxy)- 3-(1-methylpyrazol-3-yl)-2-[3-(5-trifluormethylpyrid-2-yloxy)-propyloxy]benzol (Nr. 950)

0,3 g 3-Acetyl-1-chlor-5-(3,3-dichlorprop-2-enyloxy)-2-[3-(5-trifluormethylpyrid-2-yloxy)-propyloxy]benzol und 128 µl N,N-Dimethylformamiddimethylacetal wurden 5 Stunden lang bei 95 °C gerührt. Anschließend wurde das Reaktionsgemisch im Hochvakuum (< 1 mbar) eingeengt und der Rückstand in 4 ml Ethanol gelöst. Es wurden 0,015 g Methylhydrazin zugegeben und 2 Stunden lang unter Rückfluss erhitzt. Nach dem Abkühlen wurde der Ansatz einkonzentriert und das so erhaltene Rohprodukt chromatographisch gereinigt. Ausbeute: 0,212 g (66%)

¹ H-NMR (CDCl₃): δ = 1,98 (m, 2H); 3,73 (t, 2H); 3,77 (s, 3H); 4,34 (t, 2H); 4,62 (d, 2H); 6,15 (t,1H); 6,27 (m,1H); 6,73 (d,1H); 6,76 (d,1H); 7,01 (d,1H); 7,42 (m,1H); 7,78 (dd,1H); 8,41 (br. s,1H).

Das Produkt war mit weniger als 10% der regioisomeren Struktur 1-Chlor-5-(3,3-dichlorprop-2-enyloxy)- 3-(1-methylpyrazol-5-yl)-2-[3-(5-trifluormethylpyrid-2-yloxy)-propyloxy]benzol verunreinigt.

In analoger Weise können die in den nachfolgenden Tabelle 1 bis 11 aufgeführten Verbindungen hergestellt werden. Dabei bedeuten Me Methyl und Et Ethyl.

**Tabelle 1:**

| Nummer | Grundkörper |
|---|---|
| 1 | 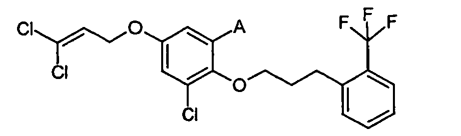 |
| 2 | 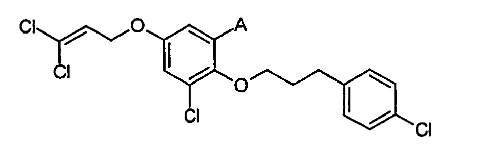 |
| 3 | 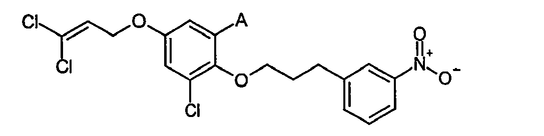 |
| 4 | 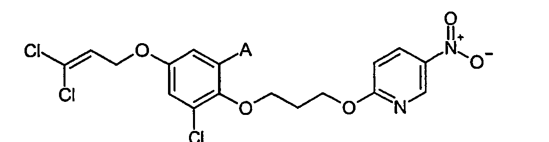 |
| 5 | 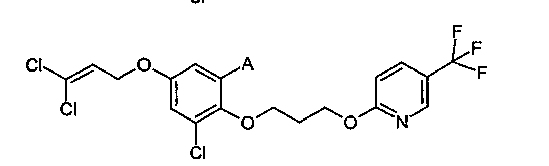 |
| 6 | 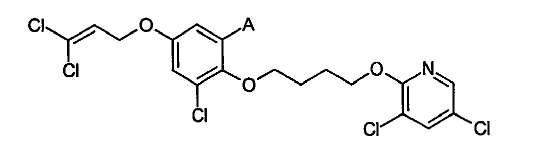 |
| 7 | 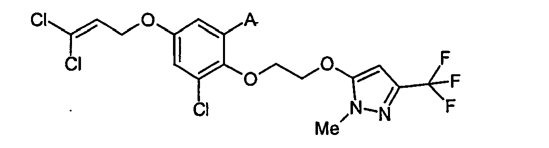 |
| 8 | 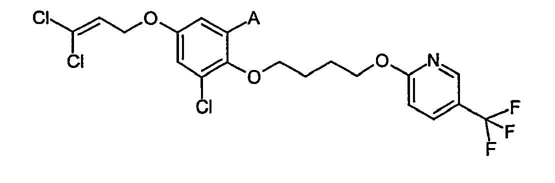 |
| 9 | 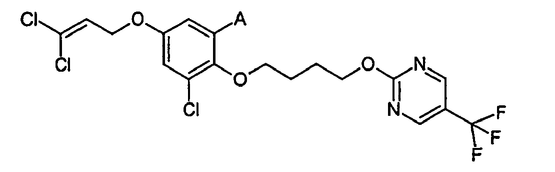 |
| 10 | 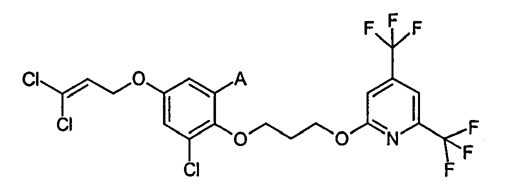 |
| 11 | 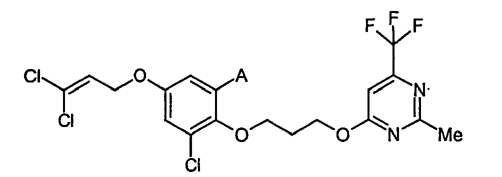 |
| 12 | 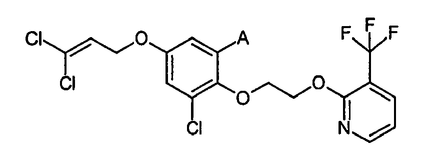 |
| 13 | 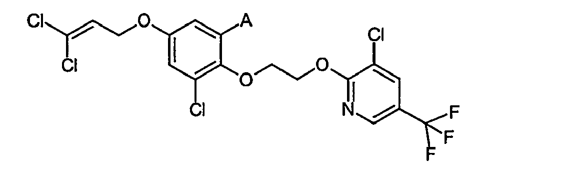 |
| 14 | 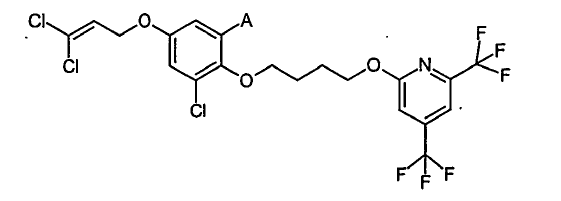 |
| 15 | 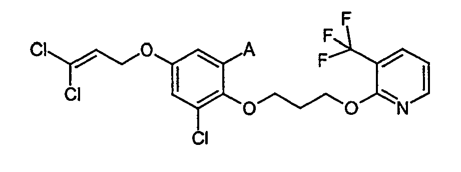 |
| 16 | 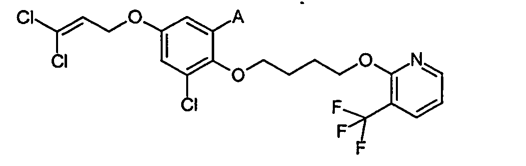 |
| 17 | 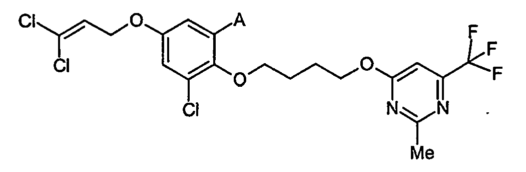 |
| 18 | 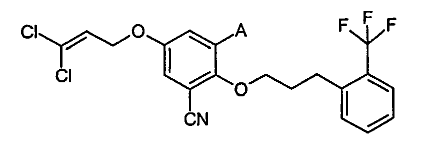 |
| 19 | 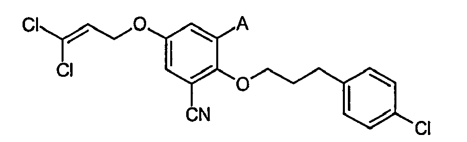 |
| 20 | 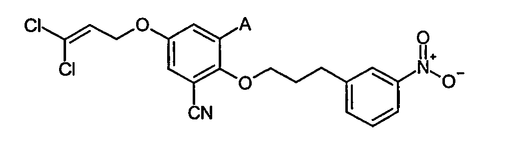 |
| 21 | 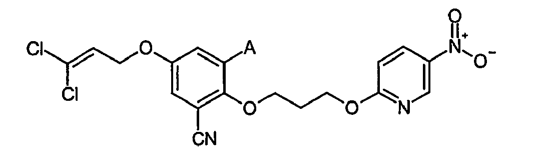 |
| 22 | 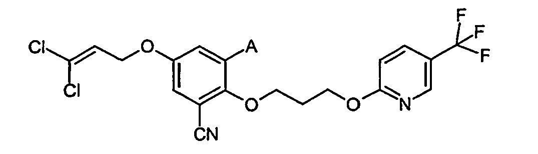 |
| 23 | 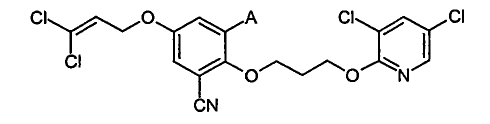 |
| 24 | 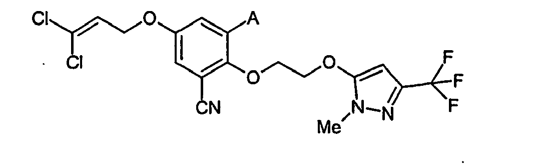 |
| 25 | 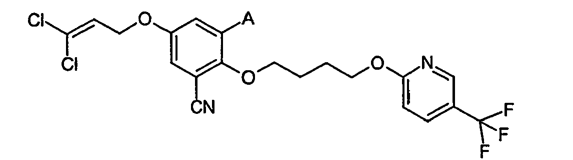 |
| 26 | 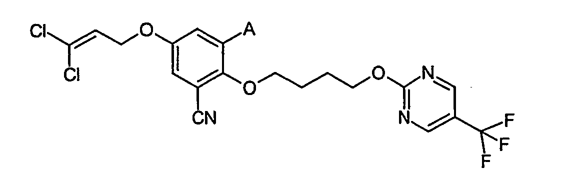 |
| 27 | 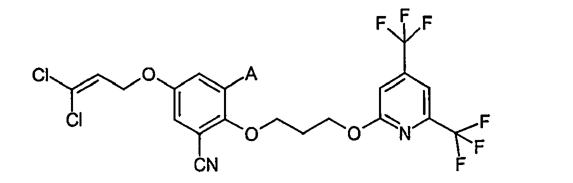 |
| 28 | 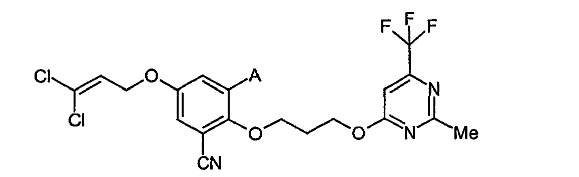 |
| 29 | 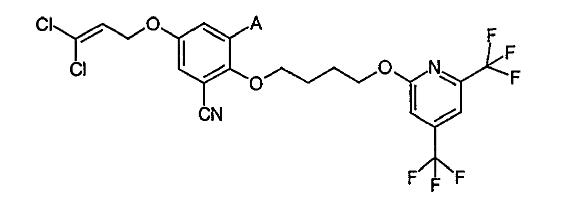 |
| 30 | 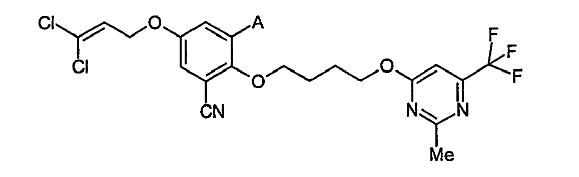 |
| 31 | 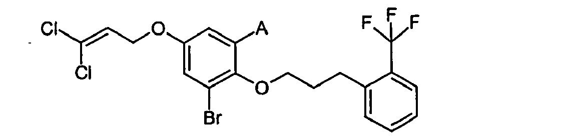 |
| 32 | 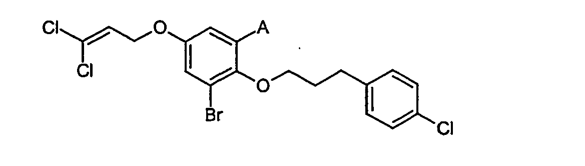 |
| 33 | 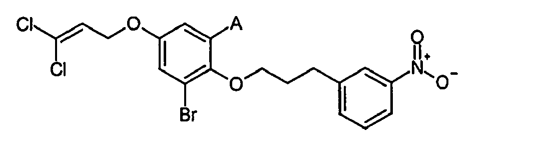 |
| 34 | 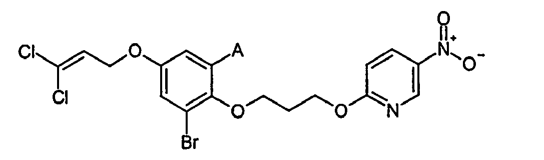 |
| 35 | 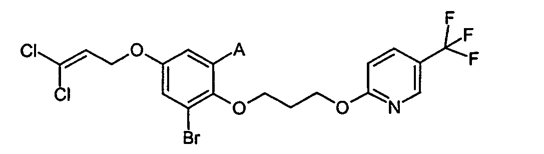 |
| 36 | 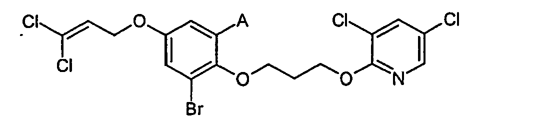 |
| 37 | 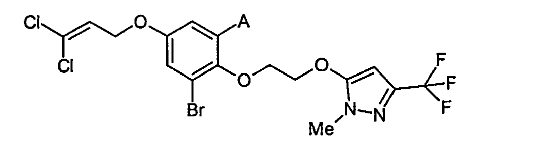 |
| 38 | 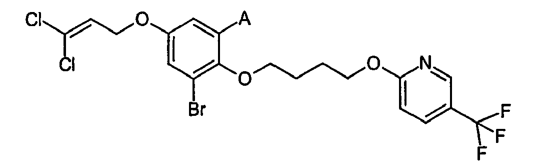 |
| 39 | 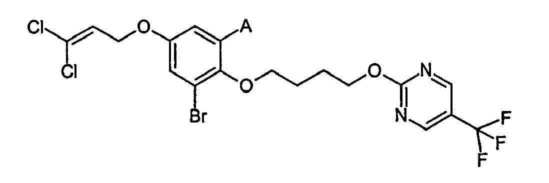 |
| 40 | 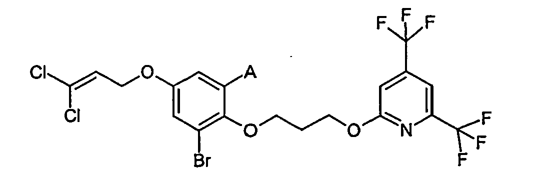 |
| 41 | 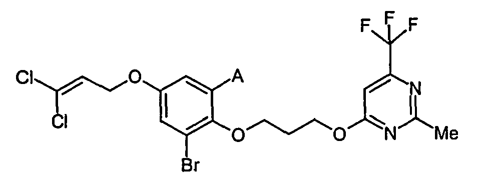 |
| 42 | 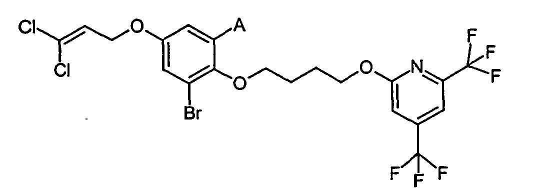 |
| 43 | 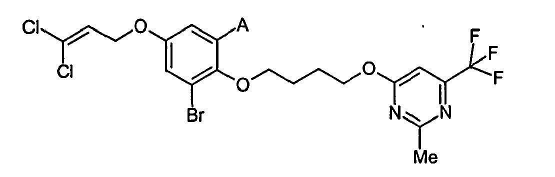 |
| 44 | 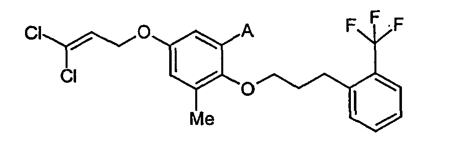 |
| 45 | 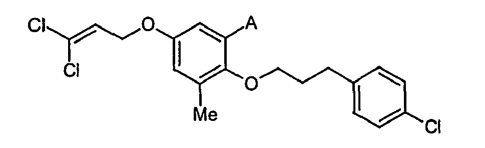 |
| 46 | 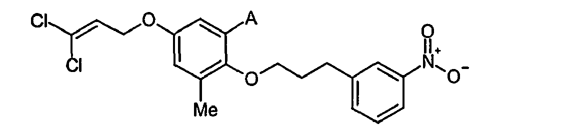 |
| 47 | 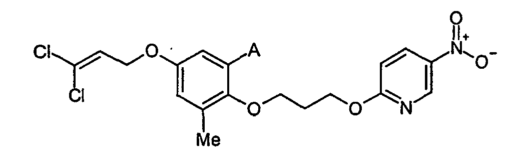 |
| 48 | 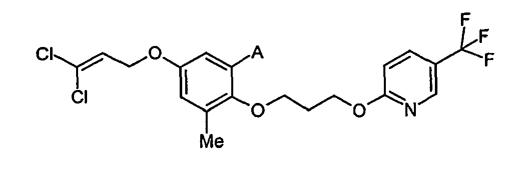 |
| 49 | 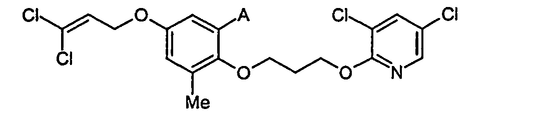 |
| 50 | 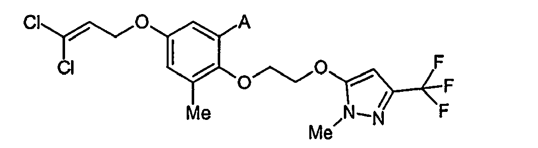 |
| 51 | 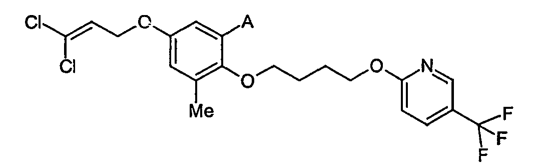 |
| 52 | 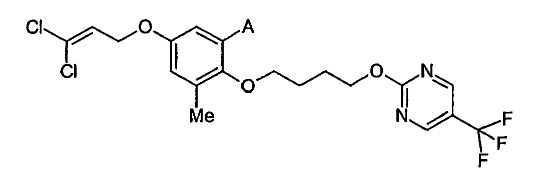 |
| 53 | 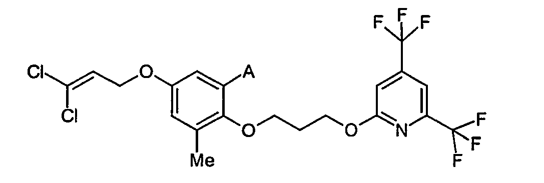 |
| 54 | 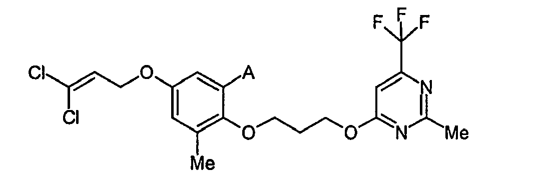 |
| 55 | 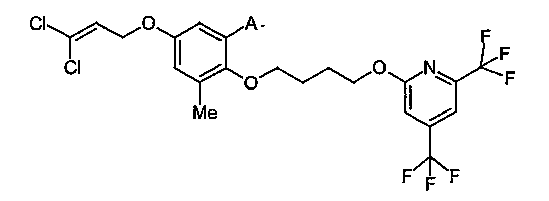 |
| 56 | 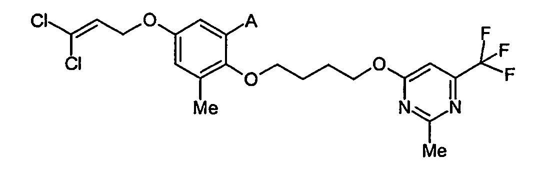 |

**Tabelle 2:**

| | | | | |
|---|---|---|---|---|
| A= 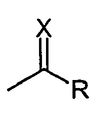 | | | | |

| Nr. | Grundkörper | X | R | Smp. |
|---|---|---|---|---|
| 1 | 1 | O | CH₂CF₃ | |
| 2 | 1 | O | Et | |
| 3 | 1 | O | Me | |
| 4 | 2 | O | CH₂CF₃ | |
| 5 | 2 | O | Et | |
| 6 | 2 | O | Me | Öl |
| 7 | 2 | O | OMe | Öl |
| 8 | 3 | O | CH₂CF₃ | |
| 9 | 3 | O | Et | |
| 10 | 3 | O | Me | |
| 11 | 4 | O | CH₂CF₃ | |
| 12 | 4 | O | Et | |
| 13 | 4 | O | Me | |
| 14 | 5 | O | 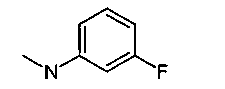 | 68°C |
| 15 | 5 | O | 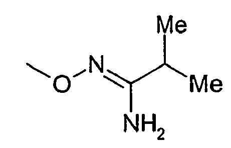 | Öl |
| 16 | 5 | O | 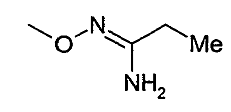 | Öl |
| 17 | 5 | O | 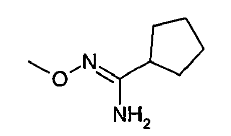 | Öl |
| 18 | 5 | O | 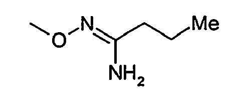 | Öl |
| 19 | 5 | O | 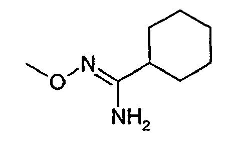 | Öl |
| 20 | 5 | O | 1-lmidazolyl | Öl |
| 21 | 5 | O | CH=CH-NMe₂ | Sirup |
| 22 | 5 | O | CH₂CF₃ | |
| 23 | 5 | O | CH₂CH₂CH₃ | |
| 24 | 5 | O | CHBr₂ | Öl |
| 25 | 5 | O | Et | Öl |
| 26 | 5 | O | H | Öl |
| 27 | 5 | O | Me | Öl |
| 28 | 5 | N-OH | Me | 66°C |
| 29 | 5 | N-OMe | Me | Öl |
| 30 | 5 | O | NH₂ | 91°C |
| 31 | 5 | S | NH₂ | 110°C |
| 32 | 5 | O | NHCH(Me)CH₂OH | 106°C |
| 33 | 5 | O | NHCH₂CH₂OH | 78°C |
| 34 | 5 | O | NHEt | 73°C |
| 35 | 5 | O | NHMe-OMe | Öl |
| 36 | 5 | O | NMe₂ | Öl |
| 37 | 5 | O | O-CH(Me)2 | glasartig |
| 38 | 5 | O | O-CH₂CH₂CH₃ | Öl |
| 39 | 5 | O | OMe | Öl |
| 40 | 5 | O | O-Phenyl | Öl |
| 41 | 6 | O | CH₂CF₃ | |
| 42 | 6 | O | Et | |
| 43 | 6 | O | Me | Öl |
| 44 | 6 | N-OH | Me | 129°C |
| 45 | 6 | N-OMe | Me | Öl |
| 46 | 6 | O | Me | |
| 47 | 7 | O | CH₂CF₃ | |
| 48 | 7 | O | Et | |
| 49 | 7 | O | Me | |
| 50 | 8 | O | 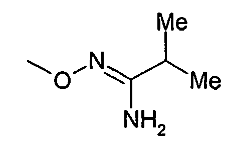 | Öl |
| 51 | 8 | O | 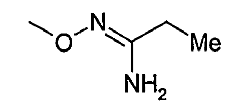 | Öl |
| 52 | 8 | O | 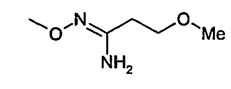 | Öl |
| 53 | 8 | O | 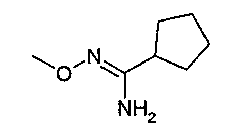 | Öl |
| 54 | 8 | O | 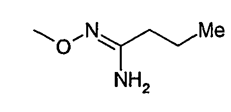 | Öl |
| 55 | 8 | O | 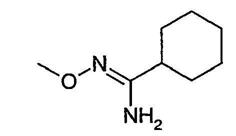 | Öl |
| 56 | 8 | O | CH₂CF₃ | |
| 57 | 8 | O | Et | |
| 58 | 8 | O | H | |
| 59 | 8 | O | Me | Öl |
| 60 | 8 | O | OMe | Öl |
| 61 | 9 | O | CH₂CF₃ | |
| 62 | 9 | O | Et | |
| 63 | 9 | O | Me | |
| 64 | 10 | O | CH₂CF₃ | |
| 65 | 10 | O | Et | |
| 66 | 10 | O | Me | |
| 67 | 11 | O | CH₂CF₃ | |
| 68 | 11 | O | Et | |
| 69 | 11 | O | Me | |
| 70 | 11 | O | OMe | 32°C - 35°C |
| 71 | 12 | O | CH₂CF₃ | |
| 72 | 12 | O | Et | |
| 73 | 12 | O | Me | 82°C |
| 74 | 13 | O | CH₂CF₃ | |
| 75 | 13 | O | Et | |
| 76 | 13 | O | Me | 110°C |
| 77 | 14 | O | CH₂CF₃ | |
| 78 | 14 | O | Et | |
| 79 | 14 | O | Me | |
| 80 | 15 | O | CH₂CF₃ | |
| 81 | 15 | O | Et | |
| 82 | 15 | O | Me | Öl |
| 83 | 15 | N-OH | Me | Öl |
| 84 | 15 | N-OMe | Me | Öl |
| 85 | 15 | N-OCH₂CH=CH₂ | Me | Öl |
| 86 | 15 | O | OMe | Öl |
| 87 | 16 | O | CH₂CF₃ | |
| 88 | 16 | O | Et | |
| 89 | 16 | O | Me | Öl |
| 90 | 16 | N-OH | Me | Öl |
| 91 | 16 | N-OMe | Me | Öl |
| 92 | 16 | N-OCH₂CH=CH₂ | Me | Öl |
| 93 | 16 | O | OMe | Öl |
| 94 | 17 | O | CH₂CF₃ | |
| 95 | 17 | O | Et | |
| 96 | 17 | O | Me | |
| 97 | 17 | O | OMe | Öl |
| 98 | 18 | O | CH₂CF₃ | |
| 99 | 18 | O | Et | |
| 100 | 18 | O | Me | |
| 101 | 19 | O | CH₂CF₃ | |
| 102 | 19 | O | Et | |
| 103 | 19 | O | Me | |
| 104 | 20 | O | CH₂CF₃ | |
| 105 | 20 | O | Et | |
| 106 | 20 | O | Me | |
| 107 | 21 | O | Me | |
| 108 | 22 | O | CH₂CF₃ | |
| 109 | 22 | O | Et | |
| 110 | 22 | O | H | |
| 111 | 22 | O | Me | |
| 112 | 23 | O | CH₂CF₃ | |
| 113 | 23 | O | Et | |
| 114 | 23 | O | Me | |
| 115 | 24 | O | CH₂CF₃ | |
| 116 | 24 | O | Et | |
| 117 | 24 | O | Me | |
| 118 | 25 | O | CH₂CF₃ | |
| 119 | 25 | O | Et | |
| 120 | 25 | O | H | |
| 121 | 25 | O | Me | |
| 122 | 26 | O | CH₂CF₃ | |
| 123 | 26 | O | Et | |
| 124 | 26 | O | Me | |
| 125 | 27 | O | CH₂CF₃ | |
| 126 | 27 | O | Et | |
| 127 | 27 | O | Me | |
| 128 | 28 | O | CH₂CF₃ | |
| 129 | 28 | O | Et | |
| 130 | 28 | O | Me | |
| 131 | 29 | O | CH₂CF₃ | |
| 132 | 29 | O | Et | |
| 133 | 29 | O | Me | |
| 134 | 30 | O | CH₂CF₃ | |
| 135 | 30 | O | Et | |
| 136 | 30 | O | Me | |
| 137 | 31 | O | CH₂CF₃ | |
| 138 | 31 | O | Et | |
| 139 | 31 | O | Me | |
| 140 | 32 | O | CH₂CF₃ | |
| 141 | 32 | O | Et | |
| 142 | 32 | O | Me | |
| 143 | 33 | O | CH₂CF₃ | |
| 144 | 33 | O | Et | |
| 145 | 33 | O | Me | |
| 146 | 34 | O | CH₂CF₃ | |
| 147 | 34 | O | Et | |
| 148 | 34 | O | Me | |
| 149 | 35 | O | CH₂CF₃ | |
| 150 | 35 | O | Et | |
| 151 | 35 | O | Me | |
| 152 | 36 | O | CH₂CF₃ | |
| 153 | 36 | O | Et | |
| 154 | 36 | O | Me | |
| 155 | 37 | O | CH₂CF₃ | |
| 156 | 37 | O | Et | |
| 157 | 37 | O | Me | |
| 158 | 38 | O | CH₂CF₃ | |
| 159 | 38 | O | Et | |
| 160 | 38 | O | Me | |
| 161 | 39 | O | CH₂CF₃ | |
| 162 | 39 | O | Et | |
| 163 | 39 | O | Me | |
| 164 | 40 | O | CH₂CF₃ | |
| 165 | 40 | O | Et | |
| 166 | 40 | O | Me | |
| 167 | 41 | O | CH₂CF₃ | |
| 168 | 41 | O | Et | |
| 169 | 41 | O | Me | |
| 170 | 42 | O | CH₂CF₃ | |
| 171 | 42 | O | Et | |
| 172 | 42 | O | Me | |
| 173 | 43 | O | CH₂CF₃ | |
| 174 | 43 | O | Et | |
| 175 | 43 | O | Me | |
| 176 | 44 | O | CH₂CF₃ | |
| 177 | 44 | O | Et | |
| 178 | 44 | O | Me | |
| 179 | 45 | O | CH₂CF₃ | |
| 180 | 45 | O | Et | |
| 181 | 45 | O | Me | |
| 182 | 46 | O | CH₂CF₃ | |
| 183 | 46 | O | Et | |
| 184 | 46 | O | Me | |
| 185 | 47 | O | CH₂CF₃ | |
| 186 | 47 | O | Et | |
| 187 | 47 | O | Me | |
| 188 | 48 | O | CH₂CF₃ | |
| 189 | 48 | O | Et | |
| 190 | 48 | O | H | |
| 191 | 48 | O | Me | |
| 192 | 49 | O | CH₂CF₃ | |
| 193 | 49 | O | Et | |
| 194 | 49 | O | Me | |
| 195 | 50 | O | CH₂CF₃ | |
| 196 | 50 | O | Et | |
| 197 | 50 | O | Me | |
| 198 | 51 | O | CH₂CF₃ | |
| 199 | 51 | O | Et | |
| 200 | 51 | O | H | |
| 201 | 51 | O | Me | |
| 202 | 52 | O | CH₂CF₃ | |
| 203 | 52 | O | Et | |
| 204 | 52 | O | Me | |
| 205 | 53 | O | CH₂CF₃ | |
| 206 | 53 | O | Et | |
| 207 | 53 | O | Me | |
| 208 | 54 | O | CH₂CF₃ | |
| 209 | 54 | O | Et | |
| 210 | 54 | O | Me | |
| 211 | 55 | O | CH₂CF₃ | |
| 212 | 55 | O | Et | |
| 213 | 55 | O | Me | |
| 214 | 56 | O | CH₂CF₃ | |
| 215 | 56 | O | Et | |
| 216 | 56 | O | Me | |

**Tabelle 3:**

| | | | |
|---|---|---|---|
| A= 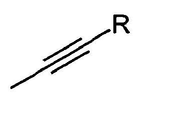 | | | |

| Nr. | Grundkörper | R | Smp. |
|---|---|---|---|
| 217 | 1 | H | |
| 218 | 1 | Me | |
| 219 | 2 | H | |
| 220 | 2 | Me | |
| 221 | 3 | H | |
| 222 | 3 | Me | |
| 223 | 4 | H | |
| 224 | 4 | Me | |
| 225 | 5 | 1-Hexyl | Öl |
| 226 | 5 | 2-Chlorphenyl | Öl |
| 227 | 5 | CH₂OH | 89°C |
| 228 | 5 | CH₂OMe | Öl |
| 229 | 5 | H | Öl |
| 230 | 5 | Me | |
| 231 | 5 | SiMe₃ | Öl |
| 232 | 6 | H | |
| 233 | 6 | Me | |
| 234 | 7 | H | |
| 235 | 7 | Me | |
| 236 | 8 | H | |
| 237 | 8 | Me | |
| 238 | 9 | H | |
| 239 | 9 | Me | |
| 240 | 10 | H | |
| 241 | 10 | Me | |
| 242 | 11 | H | |
| 243 | 11 | Me | |
| 244 | 12 | H | |
| 245 | 12 | Me | |
| 246 | 13 | H | |
| 247 | 13 | Me | |
| 248 | 14 | H | |
| 249 | 14 | Me | |
| 250 | 15 | H | |
| 251 | 15 | Me | |
| 252 | 16 | H | |
| 253 | 16 | Me | |
| 254 | 17 | H | |
| 255 | 17 | Me | |
| 256 | 18 | H | |
| 257 | 18 | Me | |
| 258 | 19 | H | |
| 259 | 19 | Me | |
| 260 | 20 | H | |
| 261 | 20 | Me | |
| 262 | 22 | H | |
| 263 | 22 | Me | |
| 264 | 23 | H | |
| 265 | 23 | Me | |
| 266 | 24 | H | |
| 267 | 24 | Me | |
| 268 | 25 | H | |
| 269 | 25 | Me | |
| 270 | 26 | H | |
| 271 | 26 | Me | |
| 272 | 27 | H | |
| 273 | 27 | Me | |
| 274 | 28 | H | |
| 275 | 28 | Me | |
| 276 | 29 | H | |
| 277 | 29 | Me | |
| 278 | 30 | H | |
| 279 | 30 | Me | |
| 280 | 31 | H | |
| 281 | 31 | Me | |
| 282 | 32 | H | |
| 283 | 32 | Me | |
| 284 | 33 | H | . |
| 285 | 33 | Me | |
| 286 | 34 | H | |
| 287 | 34 | Me | |
| 288 | 35 | H | |
| 289 | 35 | Me | |
| 290 | 36 | H | |
| 291 | 36 | Me | |
| 292 | 37 | H | |
| 293 | 37 | Me | |
| 294 | 38 | H | |
| 295 | 38 | Me | |
| 296 | 39 | H | |
| 297 | 39 | Me | |
| 298 | 40 | H | |
| 299 | 40 | Me | |
| 300 | 41 | H | |
| 301 | 41 | Me | |
| 302 | 42 | H | |
| 303 | 42 | Me | |
| 304 | 43 | H | |
| 305 | 43 | Me | |
| 306 | 44 | H | |
| 307 | 44 | Me | |
| 308 | 45 | H | |
| 309 | 45 | Me | |
| 310 | 46 | H | |
| 311 | 46 | Me | |
| 312 | 47 | H | |
| 313 | 47 | Me | |
| 314 | 48 | H | |
| 315 | 48 | Me | |
| 316 | 49 | H | |
| 317 | 49 | Me | |
| 318 | 50 | H | |
| 319 | 50 | Me | |
| 320 | 51 | H | |
| 321 | 51 | Me | |
| 322 | 52 | H | |
| 323 | 52 | Me | |
| 324 | 53 | H | |
| 325 | 53 | Me | |
| 326 | 54 | H | |
| 327 | 54 | Me | |
| 328 | 55 | H | |
| 329 | 55 | Me | |
| 330 | 56 | H | |
| 331 | 56 | Me | |

**Tabelle 4:**

| | | | |
|---|---|---|---|
| A= 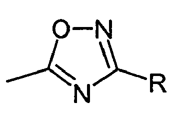 | | | |

| Nr. | Grundkörper | R | Smp. |
|---|---|---|---|
| 332 | 1 | CH₂SMe | |
| 333 | 1 | Et | |
| 334 | 1 | Me | |
| 335 | 2 | CH₂SMe | |
| 336 | 2 | Et | |
| 337 | 2 | Me | |
| 338 | 3 | CH₂SMe | |
| 339 | 3 | Et | |
| 340 | 3 | Me | |
| 341 | 4 | CH₂SMe | |
| 342 | 4 | Et | |
| 343 | 4 | Me | |
| 344 | 5 | CH(Me)₂ | |
| 345 | 5 | CH₂CF₃ | |
| 346 | 5 | CH₂CH₂CH₂OMe | |
| 347 | 5 | CH₂CH₂CH₃ | Öl |
| 348 | 5 | CH₂CH₂OEt | Öl |
| 349 | 5 | CH₂CO₂Me | |
| 350 | 5 | CH₂N(Me)₂ | |
| 351 | 5 | CH₂SMe | Öl |
| 352 | 5 | CH₂SPh | |
| 353 | 5 | CHMe₂ | Öl |
| 354 | 5 | cyclo Hexyl | Öl |
| 355 | 5 | cyclo Pentyl | Öl |
| 356 | 5 | Et | Öl |
| 357 | 5 | Me | Öl |
| 358 | 6 | CH₂SMe | |
| 359 | 6 | Et | |
| 360 | 6 | Me | |
| 361 | 7 | CH(Me)₂ | |
| 362 | 7 | CH₂CF₃ | |
| 363 | 7 | CH₂CH₂CH₂OMe | |
| 364 | 7 | CH₂CO₂Me | |
| 365 | 7 | CH₂N(Me)₂ | |
| 366 | 7 | CH₂SMe | |
| 367 | 7 | CH₂SPh | |
| 368 | 7 | Et | |
| 369 | 7 | Me | |
| 370 | 8 | CH(Me)₂ | |
| 371 | 8 | CH₂CF₃ | |
| 372 | 8 | CH₂CH₂CH₂OMe | |
| 373 | 8 | CH₂CH₂CH₃ | Öl |
| 374 | 8 | CH₂CH₂OEt | Öl |
| 375 | 8 | CH2CH3 | Öl |
| 376 | 8 | CH₂CO₂Me | |
| 377 | 8 | CH₂N(Me)₂ | |
| 378 | 8 | CH₂SMe | |
| 379 | 8 | CH₂SPh | |
| 380 | 8 | CHMe₂ | Öl |
| 381 | 8 | cyclo Hexyl | Öl |
| 382 | 8 | cyclo Pentyl | Öl |
| 383 | 8 | Me | |
| 384 | 9 | CH₂SMe | |
| 385 | 9 | Et | |
| 386 | 9 | Me | |
| 387 | 10 | CH₂SMe | |
| 388 | 10 | Et | |
| 389 | 10 | Me | |
| 390 | 11 | CH₂SMe | |
| 391 | 11 | Et | |
| 392 | 11 | Me | |
| 393 | 12 | CH₂SMe | |
| 394 | 12 | Et | |
| 395 | 12 | Me | |
| 396 | 13 | CH₂SMe | |
| 397 | 13 | Et | |
| 398 | 13 | Me | |
| 399 | 14 | CH₂SMe | |
| 400 | 14 | Et | |
| 401 | 14 | Me | |
| 402 | 15 | CH₂SMe | |
| 403 | 15 | Et | |
| 404 | 15 | Me | |
| 405 | 16 | CH₂SMe | |
| 406 | 16 | Et | |
| 407 | 16 | Me | |
| 408 | 17 | CH₂SMe | |
| 409 | 17 | Et | |
| 410 | 17 | Me | |
| 411 | 18 | CH₂SMe | |
| 412 | 18 | Et | |
| 413 | 18 | Me | |
| 414 | 19 | CH₂SMe | |
| 415 | 19 | Et | |
| 416 | 19 | Me | |
| 417 | 20 | CH₂SMe | |
| 418 | 20 | Et | |
| 419 | 20 | Me | |
| 420 | 21 | CH₂SMe | |
| 421 | 21 | Et | |
| 422 | 21 | Me | |
| 423 | 22 | CH(Me)₂ | |
| 424 | 22 | CH₂CF₃ | |
| 425 | 22 | CH₂CH₂CH₂OMe | |
| 426 | 22 | CH₂CO₂Me | |
| 427 | 22 | CH₂N(Me)₂ | |
| 428 | 22 | CH₂SMe | |
| 429 | 22 | CH₂SPh | |
| 430 | 22 | Et | |
| 431 | 22 | Me | |
| 432 | 23 | CH₂SMe | |
| 433 | 23 | Et | |
| 434 | 23 | Me | |
| 435 | 24 | CH₂SMe | |
| 436 | 24 | Et | |
| 437 | 24 | Me | |
| 438 | 25 | CH(Me)₂ | |
| 439 | 25 | CH₂CF₃ | |
| 440 | 25 | CH₂CH₂CH₂OMe | |
| 441 | 25 | CH₂CO₂Me | |
| 442 | 25 | CH₂N(Me)₂ | |
| 443 | 25 | CH₂SMe | |
| 444 | 25 | CH₂SPh | |
| 445 | 25 | Et | |
| 446 | 25 | Me | |
| 447 | 26 | CH(Me)₂ | |
| 448 | 26 | CH₂CF₃ | |
| 449 | 26 | CH₂CH₂CH₂OMe | |
| 450 | 26 | CH₂CO₂Me | |
| 451 | 26 | CH₂N(Me)₂ | |
| 452 | 26 | CH₂SMe | |
| 453 | 26 | CH₂SPh | |
| 454 | 26 | Et | |
| 455 | 26 | Me | |
| 456 | 27 | CH₂SMe | |
| 457 | 27 | Et | |
| 458 | 27 | Me | |
| 459 | 28 | CH₂SMe | |
| 460 | 28 | Et | |
| 461 | 28 | Me | |
| 462 | 29 | CH₂SMe | |
| 463 | 29 | Et | |
| 464 | 29 | Me | |
| 465 | 30 | CH₂SMe | |
| 466 | 30 | Et | |
| 467 | 30 | Me | |
| 468 | 31 | CH₂SMe | |
| 469 | 31 | Et | |
| 470 | 31 | Me | |
| 471 | 32 | CH₂SMe | |
| 472 | 32 | Et | |
| 473 | 32 | Me | |
| 474 | 33 | CH₂SMe | |
| 475 | 33 | Et | |
| 476 | 33 | Me | |
| 477 | 34 | CH₂SMe | |
| 478 | 34 | Et | |
| 479 | 34 | Me | |
| 480 | 35 | CH(Me)₂ | |
| 481 | 35 | CH₂CF₃ | |
| 482 | 35 | CH₂CH₂CH₂OMe | |
| 483 | 35 | CH₂CO₂Me | |
| 484 | 35 | CH₂N(Me)₂ | |
| 485 | 35 | CH₂SMe | |
| 486 | 35 | CH₂SPh | |
| 487 | 35 | Et | |
| 488 | 35 | Me | |
| 489 | 36 | CH₂SMe | |
| 490 | 36 | Et | |
| 491 | 36 | Me | |
| 492 | 37 | CH₂SMe | |
| 493 | 37 | Et | |
| 494 | 37 | Me | |
| 495 | 38 | CH(Me)₂ | |
| 496 | 38 | CH₂CF₃ | |
| 497 | 38 | CH₂CH₂CH₂OMe | |
| 498 | 38 | CH₂CO₂Me | |
| 499 | 38 | CH₂N(Me)₂ | |
| 500 | 38 | CH₂SMe | |
| 501 | 38 | CH₂SPh | |
| 502 | 38 | Et | |
| 503 | 38 | Me | |
| 504 | 39 | CH₂SMe | |
| 505 | 39 | Et | |
| 506 | 39 | Me | |
| 507 | 40 | CH(Me)₂ | |
| 508 | 40 | CH₂CF₃ | |
| 509 | 40 | CH₂CH₂CH₂OMe | |
| 510 | 40 | CH₂CO₂Me | |
| 511 | 40 | CH₂N(Me)₂ | |
| 512 | 40 | CH₂SMe | |
| 513 | 40 | CH₂SPh | |
| 514 | 40 | Et | |
| 515 | 40 | Me | |
| 516 | 41 | CH₂SMe | |
| 517 | 41 | Et | |
| 518 | 41 | Me | |
| 519 | 42 | CH₂SMe | |
| 520 | 42 | Et | |
| 521 | 42 | Me | |
| 522 | 43 | CH₂SMe | |
| 523 | 43 | Et | |
| 524 | 43 | Me | |
| 525 | 44 | CH₂SMe | |
| 526 | 44 | Et | |
| 527 | 44 | Me | |
| 528 | 45 | CH₂SMe | |
| 529 | 45 | Et | |
| 530 | 45 | Me | |
| 531 | 46 | CH₂SMe | |
| 532 | 46 | Et | |
| 533 | 46 | Me | |
| 534 | 47 | CH₂SMe | |
| 535 | 47 | Et | |
| 536 | 47 | Me | |
| 537 | 48 | CH(Me)₂ | |
| 538 | 48 | CH₂CF₃ | |
| 539 | 48 | CH₂CH₂CH₂OMe | |
| 540 | 48 | CH₂CO₂Me | |
| 541 | 48 | CH₂N(Me)₂ | |
| 542 | 48 | CH₂SMe | |
| 543 | 48 | CH₂SPh | |
| 544 | 48 | Et | |
| 545 | 48 | Me | |
| 546 | 49 | CH₂SMe | |
| 547 | 49 | Et | |
| 548 | 49 | Me | |
| 549 | 50 | CH₂SMe | |
| 550 | 50 | Et | |
| 551 | 50 | Me | |
| 552 | 51 | CH(Me)₂ | |
| 553 | 51 | CH₂CF₃ | |
| 554 | 51 | CH₂CH₂CH₂OMe | |
| 555 | 51 | CH₂CO₂Me | |
| 556 | 51 | CH₂N(Me)₂ | |
| 557 | 51 | CH₂SMe | |
| 558 | 51 | CH₂SPh | |
| 559 | 51 | Et | |
| 560 | 51 | Me | |
| 561 | 52 | CH₂SMe | |
| 562 | 52 | Et | |
| 563 | 52 | Me | |
| 564 | 53 | CH₂SMe | |
| 565 | 53 | Et | |
| 566 | 53 | Me | |
| 567 | 54 | CH₂SMe | |
| 568 | 54 | Et | |
| 569 | 54 | Me | |
| 570 | 55 | CH₂SMe | |
| 571 | 55 | Et | |
| 572 | 55 | Me | |
| 573 | 56 | CH₂SMe | |
| 574 | 56 | Et | |
| 575 | 56 | Me | |

**Tabelle 5:**

| | | | | | |
|---|---|---|---|---|---|
| A= 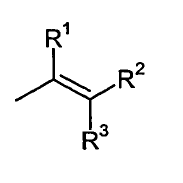 | | | | | |

| Nr. | Grundkörper | R¹ | R² | R³ | Smp. |
|---|---|---|---|---|---|
| 576 | 1 | H | Me | H | |
| 577 | 1 | H | Me | Me | |
| 578 | 2 | H | CH2CF3 | Me | |
| 579 | 2 | H | CO₂Et | H | |
| 580 | 2 | H | CO₂Me | H | |
| 581 | 2 | H | Et | Me | |
| 582 | 2 | H | H | H | |
| 583 | 2 | H | Me | H | |
| 584 | 2 | H | Me | Me | |
| 585 | 3 | H | Me | H | |
| 586 | 3 | H | Me | Me | |
| 587 | 4 | H | Me | H | |
| 588 | 4 | H | Me | Me | |
| 589 | 5 | H | Br | Br | |
| 590 | 5 | H | CH2CF3 | Me | |
| 591 | 5 | H | Cl | Cl | |
| 592 | 5 | H | CO₂Et | H | Öl |
| 593 | 5 | H | CO₂Me | H | Öl |
| 594 | 5 | H | Et | Me | |
| 595 | 5 | H | H | H | Öl |
| 596 | 5 | H | Me | H | Öl |
| 597 | 5 | H | Me | Me | |
| 598 | 5 | Me | CF₃ | H | |
| 599 | 6 | H | Me | H | |
| 600 | 6 | H | Me | Me | |
| 601 | 7 | H | Br | Br | |
| 602 | 7 | H | Cl | Cl | |
| 603 | 7 | H | Me | H | |
| 604 | 7 | H | Me | Me | |
| 605 | 7 | Me | CF₃ | H | |
| 606 | 8 | H | Br | Br | |
| 607 | 8 | H | Cl | Cl | |
| 608 | 8 | H | Me | H | |
| 609 | 8 | H | Me | Me | |
| 610 | 8 | Me | CF₃ | H | |
| 611 | 9 | H | Me | H | |
| 612 | 9 | H | Me | Me | |
| 613 | 10 | H | Me | H | |
| 614 | 10 | H | Me | Me | |
| 615 | 11 | H | Me | H | |
| 616 | 11 | H | Me | Me | |
| 617 | 12 | H | Me | H | |
| 618 | 12 | H | Me | Me | |
| 619 | 13 | H | Me | H | |
| 620 | 13 | H | Me | Me | |
| 621 | 14 | H | Me | H | |
| 622 | 14 | H | Me | Me | |
| 623 | 15 | H | Me | H | |
| 624 | 15 | H | Me | Me | |
| 625 | 16 | H | Me | H | |
| 626 | 16 | H | Me | Me | |
| 627 | 17 | H | Me | H | |
| 628 | 17 | H | Me | Me | |
| 629 | 18 | H | Me | H | |
| 630 | 18 | H | Me | Me | |
| 631 | 19 | H | Me | H | |
| 632 | 19 | H | Me | Me | |
| 633 | 20 | H | Me | H | |
| 634 | 20 | H | Me | Me | |
| 635 | 21 | H | Me | H | |
| 636 | 21 | H | Me | Me | |
| 637 | 22 | H | Br | Br | |
| 638 | 22 | H | Cl | Cl | |
| 639 | 22 | H | Me | H | |
| 640 | 22 | H | Me | Me | |
| 641 | 22 | Me | CF₃ | H | |
| 642 | 23 | H | Me | H | |
| 643 | 23 | H | Me | Me | |
| 644 | 24 | H | Me | H | |
| 645 | 24 | H | Me | Me | |
| 646 | 25 | H | Br | Br | |
| 647 | 25 | H | Cl | Cl | |
| 648 | 25 | H | Me | H | |
| 649 | 25 | H | Me | Me | |
| 650 | 25 | Me | CF₃ | H | |
| 651 | 26 | H | Br | Br | |
| 652 | 26 | H | Cl | Cl | |
| 653 | 26 | H | Me | H | |
| 654 | 26 | H | Me | Me | |
| 655 | 26 | Me | CF₃ | H | |
| 656 | 27 | H | Me | H | |
| 657 | 27 | H | Me | Me | |
| 658 | 28 | H | Me | H | |
| 659 | 28 | H | Me | Me | |
| 660 | 29 | H | Me | H | |
| 661 | 29 | H | Me | Me | |
| 662 | 30 | H | Me | H | |
| 663 | 30 | H | Me | Me | |
| 664 | 31 | H | Me | H | |
| 665 | 31 | H | Me | Me | |
| 666 | 32 | H | Me | H | |
| 667 | 32 | H | Me | Me | |
| 668 | 33 | H | Me | H | |
| 669 | 33 | H | Me | Me | |
| 670 | 34 | H | Me | H | |
| 671 | 34 | H | Me | Me | |
| 672 | 35 | H | Br | Br | |
| 673 | 35 | H | Cl | Cl | |
| 674 | 35 | H | Me | H | |
| 675 | 35 | H | Me | Me | |
| 676 | 35 | Me | CF₃ | H | |
| 677 | 36 | H | Me | H | |
| 678 | 36 | H | Me | Me | |
| 679 | 37 | H | Me | H | |
| 680 | 37 | H | Me | Me | |
| 681 | 38 | H | Br | Br | |
| 682 | 38 | H | Cl | Cl | |
| 683 | 38 | H | Me | H | |
| 684 | 38 | H | Me | Me | |
| 685 | 38 | Me | CF₃ | H | |
| 686 | 39 | H | Me | H | |
| 687 | 39 | H | Me | Me | |
| 688 | 40 | H | Br | Br | |
| 689 | 40 | H | Cl | Cl | |
| 690 | 40 | H | Me | H | |
| 691 | 40 | H | Me | Me | |
| 692 | 40 | Me | CF₃ | H | |
| 693 | 41 | H | Me | H | |
| 694 | 41 | H | Me | Me | |
| 695 | 42 | H | Me | H | |
| 696 | 42 | H | Me | Me | |
| 697 | 43 | H | Me | H | |
| 698 | 43 | H | Me | Me | |
| 699 | 44 | H | Me | H | |
| 700 | 44 | H | Me | Me | |
| 701 | 45 | H | Me | H | |
| 702 | 45 | H | Me | Me | |
| 703 | 46 | H | Me | H | |
| 704 | 46 | H | Me | Me | |
| 705 | 47 | H | Me | H | |
| 706 | 47 | H | Me | Me | |
| 707 | 48 | H | Br | Br | |
| 708 | 48 | H | Cl | Cl | |
| 709 | 48 | H | Me | H | |
| 710 | 48 | H | Me | Me | |
| 711 | 48 | Me | CF₃ | H | |
| 712 | 49 | H | Me | H | |
| 713 | 49 | H | Me | Me | |
| 714 | 50 | H | Me | H | |
| 715 | 50 | H | Me | Me | |
| 716 | 51 | H | Br | Br | |
| 717 | 51 | H | Cl | Cl | |
| 718 | 51 | H | Me | H | |
| 719 | 51 | H | Me | Me | |
| 720 | 51 | Me | CF₃ | H | |
| 721 | 52 | H | Me | H | |
| 722 | 52 | H | Me | Me | |
| 723 | 53 | H | Me | H | |
| 724 | 53 | H | Me | Me | |
| 725 | 54 | H | Me | H | |
| 726 | 54 | H | Me | Me | |
| 727 | 55 | H | Me | H | |
| 728 | 55 | H | Me | Me | |
| 729 | 56 | H | Me | H | |
| 730 | 56 | H | Me | Me | |

**Tabelle 6:**

| | | | | | | |
|---|---|---|---|---|---|---|
| A= 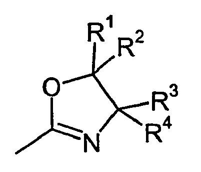 | | | | | | |

| Nr. | Grundkörper | R¹ | R² | R³ | R⁴ | Smp. |
|---|---|---|---|---|---|---|
| 731 | 1 | H | H | H | H | |
| 732 | 1 | H | H | Me | H | |
| 733 | 1 | Me | H | H | H | |
| 734 | 3 | H | H | H | H | |
| 735 | 3 | H | H | Me | H | |
| 736 | 3 | Me | H | H | H | |
| 737 | 5 | 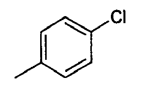 | H | Me | H | |
| 738 | 5 | H | H | H | H | Öl |
| 739 | 5 | H | H | Me | H | Öl |
| 740 | 5 | H | H | 3-Pyridyl | H | |
| 741 | 5 | Me | H | H | H | |
| 742 | 7 | H | H | Me | H | |
| 743 | 7 | H | H | Me | Me | |
| 744 | 7 | Me | H | H | H | |
| 745 | 8 | 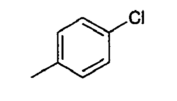 | H | Me | H | |
| 746 | 8 | H | H | H | H | |
| 747 | 8 | H | H | Me | H | |
| 748 | 8 | H | H | 3-Pyridyl | H | |
| 749 | 8 | Me | H | H | H | |
| 750 | 14 | H | H | H | H | |
| 751 | 14 | H | H | Me | H | |
| 752 | 14 | Me | H | H | H | |
| 753 | 16 | H | H | H | H | |
| 754 | 18 | H | H | Me | H | |
| 755 | 22 | 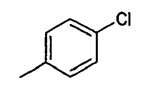 | H | Me | H | |
| 756 | 22 | H | H | Me | H | |
| 757 | 22 | H | H | Me | Me | |
| 758 | 22 | H | H | 3-Pyridyl | H | |
| 759 | 22 | Me | H | H | H | |
| 760 | 25 | 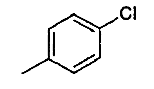 | H | Me | H | |
| 761 | 25 | H | H | Me | H | |
| 762 | 25 | H | H | Me | Me | |
| 763 | 25 | H | H | 3-Pyridyl | H | |
| 764 | 25 | Me | H | H | H | |
| 765 | 26 | H | H | Me | H | |
| 766 | 26 | H | H | Me | Me | |
| 767 | 26 | Me | H | H | H | |
| 768 | 33 | Me | H | H | H | |
| 769 | 35 | 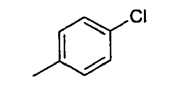 | H | Me | H | |
| 770 | 35 | H | H | Me | H | |
| 771 | 35 | H | H | Me | Me | |
| 772 | 35 | H | H | 3-Pyridyl | H | |
| 773 | 35 | Me | H | H | H | |
| 774 | 38 | 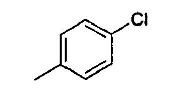 | H | Me | H | |
| 775 | 38 | H | H | Me | H | |
| 776 | 38 | H | H | Me | Me | |
| 777 | 38 | H | H | 3-Pyridyl | H | |
| 778 | 38 | Me | H | H | H | |
| 779 | 40 | H | H | Me | H | |
| 780 | 40 | H | H | Me | Me | |
| 781 | 40 | Me | H | H | H | |
| 782 | 48 | 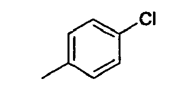 | H | Me | H | |
| 783 | 48 | H | H | Me | H | |
| 784 | 48 | H | H | Me | Me | |
| 785 | 48 | H | H | 3-Pyridyl | H | |
| 786 | 48 | Me | H | H | H | |
| 787 | 51 | 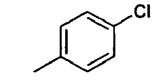 | H | Me | H | |
| 788 | 51 | H | H | Me | H | |
| 789 | 51 | H | H | Me | Me | |
| 790 | 51 | H | H | 3-Pyridyl | H | |
| 791 | 51 | Me | H | H | H | |

**Tabelle 7:**

| | | | | |
|---|---|---|---|---|
| A= 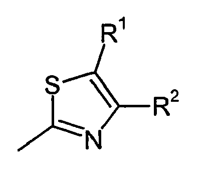 | | | | |

| Nr. | Grundkörper | R¹ | R² | Smp. |
|---|---|---|---|---|
| 792 | 5 | H | CF₃ | |
| 793 | 5 | H | CH₂CH₂OMe | |
| 794 | 5 | H | Me | Öl |
| 795 | 5 | Me | 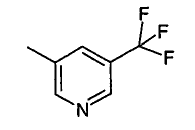 | |
| 796 | 5 | Me | cyclo-Propyl | |
| 797 | 5 | Me | Me | 105°C |
| 798 | 7 | H | CF₃ | |
| 799 | 7 | H | CH₂CH₂OMe | |
| 800 | 7 | H | Me | |
| 801 | 7 | Me | 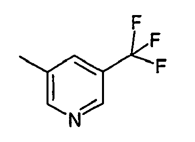 | |
| 802 | 7 | Me | cyclo-Propyl | |
| 803 | 7 | Me | Me | |
| 804 | 8 | H | CF₃ | |
| 805 | 8 | H | CH₂CH₂OMe | |
| 806 | 8 | H | Me | |
| 807 | 8 | Me | 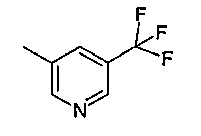 | |
| 808 | 8 | Me | cyclo-Propyl | |
| 809 | 8 | Me | Me | |
| 810 | 22 | H | CF₃ | |
| 811 | 22 | H | CH₂CH₂OMe | |
| 812 | 22 | H | Me | |
| 813 | 22 | Me | 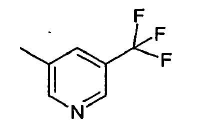 | |
| 814 | 22 | Me | cyclo-Propyl | |
| 815 | 22 | Me | Me | |
| 816 | 25 | H | CF₃ | |
| 817 | 25 | H | CH₂CH₂OMe | |
| 818 | 25 | H | Me | |
| 819 | 25 | Me | 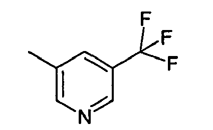 | |
| 820 | 25 | Me | cyclo-Propyl | |
| 821 | 25 | Me | Me | |
| 822 | 26 | H | CF₃ | |
| 823 | 26 | H | CH₂CH₂OMe | |
| 824 | 26 | H | Me | |
| 825 | 26 | Me | 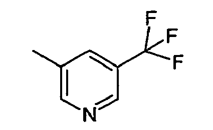 | |
| 826 | 26 | Me | cyclo-Propyl | |
| 827 | 26 | Me | Me | |
| 828 | 35 | H | CF₃ | |
| 829 | 35 | H | CH₂CH₂OMe | |
| 830 | 35 | H | Me | |
| 831 | 35 | Me | 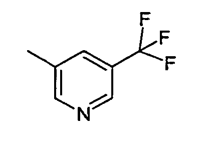 | |
| 832 | 35 | Me | cyclo-Propyl | |
| 833 | 35 | Me | Me | |
| 834 | 38 | H | CF₃ | |
| 835 | 38 | H | CH₂CH₂OMe | |
| 836 | 38 | H | Me | |
| 837 | 38 | Me | 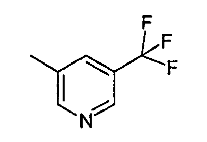 | |
| 838 | 38 | Me | cyclo-Propyl | |
| 839 | 38 | Me | Me | |
| 840 | 40 | H | CF₃ | |
| 841 | 40 | H | CH₂CH₂OMe | |
| 842 | 40 | H | Me | |
| 843 | 40 | Me | 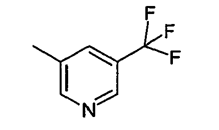 | |
| 844 | 40 | Me | cyclo-Propyl | |
| 845 | 40 | Me | Me | |
| 846 | 48 | H | CF₃ | |
| 847 | 48 | H | CH₂CH₂OMe | |
| 848 | 48 | H | Me | |
| 849 | 48 | Me | 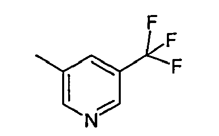 | |
| 850 | 48 | Me | cyclo-Propyl | |
| 851 | 48 | Me | Me | |
| 852 | 51 . | H | CF₃ | |
| 853 | 51 | H | CH₂CH₂OMe | |
| 854 | 51 | H | Me | |
| 855 | 51 | Me | 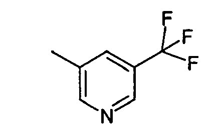 | |
| 856 | 51 | Me | cyclo-Propyl | |
| 857 | 51 | Me | Me | |

**Tabelle 8:**

| | | | | |
|---|---|---|---|---|
| A= 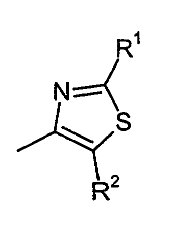 | | | | |

| Nr. | Grundkörper | R¹ | R² | Smp. |
|---|---|---|---|---|
| 858 | 1 | Me | H | |
| 859 | 2 | Me | H | |
| 860 | 3 | Me | H | |
| 861 | 4 | Me | H | |
| 862 | 5 | CF₃ | H | |
| 863 | 5 | CH=CH₂ | Me | |
| 864 | 5 | H | H | Öl |
| 865 | 5 | Me | H | Öl |
| 866 | 6 | Me | H | |
| 867 | 7 | CF₃ | H | |
| 868 | 7 | CH=CH₂ | Me | |
| 869 | 7 | H | H | |
| 870 | 7 | Me | H | |
| 871 | 8 | CF₃ | H | |
| 872 | 8 | CH=CH₂ | Me | |
| 873 | 8 | H | H | |
| 874 | 8 | Me | H | |
| 875 | 9 | Me | H | |
| 876 | 10 | Me | H | |
| 877 | 11 | Me | H | |
| 878 | 12 | Me | H | |
| 879 | 13 | Me | H | |
| 880 | 14 | Me | H | |
| 881 | 15 | Me | H | |
| 882 | 16 | Me | H | |
| 883 | 17 | Me | H | |
| 884 | 18 | Me | H | |
| 885 | 19 | Me | H | |
| 886 | 20 | Me | H | |
| 887 | 21 | Me | H | |
| 888 | 22 | CF₃ | H | |
| 889 | 22 | CH=CH₂ | Me | |
| 890 | 22 | H | H | |
| 891 | 22 | Me | H | |
| 892 | 23 | Me | H | |
| 893 | 24 | Me | H | |
| 894 | 25 | CF₃ | H | |
| 895 | 25 | CH=CH₂ | Me | |
| 896 | 25 | H | H | |
| 897 | 25 | Me | H | |
| 898 | 26 | CF₃ | H | |
| 899 | 26 | CH=CH₂ | Me | |
| 900 | 26 | H | H | |
| 901 | 26 | Me | H | |
| 902 | 27 | Me | H | |
| 903 | 28 | Me | H | |
| 904 | 29 | Me | H | |
| 905 | 30 | Me | H | |
| 906 | 31 | Me | H | |
| 907 | 32 | Me | H | |
| 908 | 33 | Me | H | |
| 909 | 34 | Me | H | |
| 910 | 35 | CF₃ | H | |
| 911 | 35 | CH=CH₂ | Me | |
| 912 | 35 | H | H | |
| 913 | 35 | Me | H | |
| 914 | 36 | Me | H | |
| 915 | 37 | Me | H | |
| 916 | 38 | CF₃ | H | |
| 917 | 38 | CH=CH₂ | Me | |
| 918 | 38 | H | H | |
| 919 | 38 | Me | H | |
| 920 | 39 | Me | H | |
| 921 | 40 | CF₃ | H | |
| 922 | 40 | CH=CH₂ | Me | |
| 923 | 40 | H | H | |
| 924 | 40 | Me | H | |
| 925 | 41 | Me | H | |
| 926 | 42 | Me | H | |
| 927 43 | 43 | Me | H | |
| 928 | 44 | Me | H | |
| 929 | 45 | Me | H | |
| 930 | 46 | Me | H | |
| 931 | 47 | Me | H | |
| 932 | 48 | CF₃ | H | |
| 933 | 48 | CH=CH₂ | Me | |
| 934 | 48 | H | H | |
| 935 | 84 | Me | H | |
| 936 | 49 | Me | H | |
| 937 | 50 | Me | H | |
| 938 | 51 | CF₃ | H | |
| 939 | 51 | CH=CH₂ | Me | |
| 940 | 51 | H | H | |
| 941 | 51 | Me | H | |
| 942 | 52 | Me | H | |
| 943 | 53 | Me | H | |
| 944 | 54 | Me | H | |
| 945 | 55 | Me | H | |
| 946 | 56 | Me | H | |

**Tabelle 9:**

| | | | | | |
|---|---|---|---|---|---|
| A= 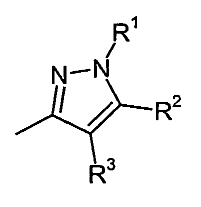 | | | | | |

| Nr. | Grundkörper | _{R}1 | R² | R³ | Smp. |
|---|---|---|---|---|---|
| 947 | 5 | 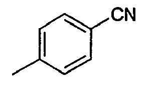 | Me | Me | |
| 948 | 5 | CH(Me)₂ | H | Me | |
| 949 | 5 | H | H | H | Öl |
| 950 | 5 | Me | H | H | Öl |
| 951 | 7 | 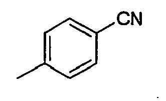 | Me | Me | |
| 952 | 7 | CH(Me)₂ | H | Me | |
| 953 | 7 | H | H | H | |
| 954 | 7 | Me | H | H | |
| 955 | 8 | 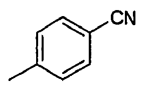 | Me | Me | |
| 956 | 8 | CH(Me)₂ | H | Me | |
| 957 | 8 | H | H | H | |
| 958 | 8 | Me | H | H | |
| 959 | 22 | 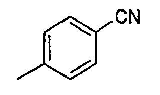 | Me | Me | |
| 960 | 22 | CH(Me)₂ | H | Me | |
| 961 | 22 | H | H | H | |
| 962 | 22 | Me | H | H | |
| 963 | 25 | 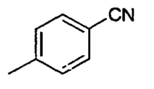 | Me | Me | |
| 964 | 25 | CH(Me)₂ | H | Me | |
| 965 | 25 | H | H | H | |
| 966 | 25 | Me | H | H | |
| 967 | 26 | CN | Me | Me | |
| 968 | 26 | CH(Me)₂ | H | Me | |
| 969 | 26 | H | H | H | |
| 970 | 26 | Me | H | H | |
| 971 | 35 | 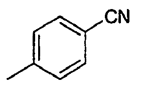 | Me | Me | |
| 972 | 35 | CH(Me)₂ | H | Me | |
| 973 | 35 | H | H | H | |
| 974 | 35 | Me | H | H | |
| 975 | 38 | 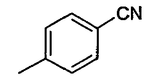 | Me | Me | |
| 976 | 38 | CH(Me)₂ | H | Me | |
| 977 | 38 | H | H | H | |
| 978 | 38 | Me | H | H | |
| 979 | 40 | 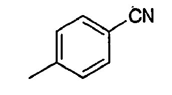 | Me | Me | |
| 980 | 40 | CH(Me)₂ | H | Me | |
| 981 | 40 | H | H | H | |
| 982 | 40 | Me | H | H | |
| 983 | 48 | 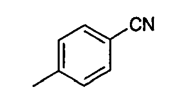 | Me | Me | |
| 984 | 48 | CH(Me)₂ | H | Me | |
| 985 | 48 | H | H | H | |
| 986 | 48 | Me | H | H | |
| 987 | 51 | 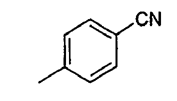 | Me | Me | |
| 988 | 51 | CH(Me)₂ | H | Me | |
| 989 | 51 | H | H | H | |
| 990 | 51 | Me | H | H | |

**Tabelle 10:**

| | | | | |
|---|---|---|---|---|
| A= 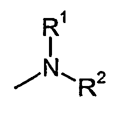 | | | | |

| Nr. | Grundkörper | R¹ | R² | Smp. |
|---|---|---|---|---|
| 991 | 1 | H | C(=O)NMe₂ | |
| 992 | 1 | H | C(=O)OMe | |
| 993 | 2 | H | C(=O)NMe₂ | |
| 994 | 2 | H | C(=O)OMe | |
| 995 | 3 | H | C(=O)NMe₂ | |
| 996 | 3 | H | C(=O)OMe | |
| 997 | 4 | H | C(=O)NMe₂ | |
| 998 | 4 | H | C(=O)OMe | |
| 999 | 5 | H | C(=O)Me | |
| 1000 | 5 | H | C(=O)NMe₂ | 111°C |
| 1001 | 5 | H | C(=O)OMe | Öl |
| 1002 | 5 | Me | C(=O)H | |
| 1003 | 6 | H | C(=O)NMe₂ | |
| 1004 | 6 | H | C(=O)OMe | |
| 1005 | 7 | H | C(=O)Me | |
| 1006 | 7 | H | C(=O)NMe₂ | |
| 1007 | 7 | H | C(=O)OMe | |
| 1008 | 7 | Me | C(=O)Et | |
| 1009 | 8 | H | C(=O)Me | |
| 1010 | 8 | H | C(=O)NMe₂ | |
| 1011 | 8 | H | C(=O)OMe | |
| 1012 | 8 | Me | C(=O)Me | |
| 1013 | 9 | H | C(=O)NMe₂ | |
| 1014 | 9 | H | C(=O)OMe | |
| 1015 | 10 | H | C(=O)NMe₂ | |
| 1016 | 10 | H | C(=O)OMe | |
| 1017 | 11 | H | C(=O)NMe₂ | |
| 1018 | 11 | H | C(=O)OMe | |
| 1019 | 12 | H | C(=O)NMe₂ | |
| 1020 | 12 | H | C(=O)OMe | |
| 1021 | 13 | H | C(=O)NMe₂ | |
| 1022 | 13 | H | C(=O)OMe | |
| 1023 | 14 | H | C(=O)NMe₂ | |
| 1024 | 14 | H | C(=O)OMe | |
| 1025 | 15 | H | C(=O)NMe₂ | |
| 1026 | 15 | H | C(=O)OMe | |
| 1027 | 16 | H | C(=O)NMe₂ | |
| 1028 | 16 | H | C(=O)OMe | |
| 1029 | 17 | H | C(=O)NMe₂ | |
| 1030 | 17 | H | C(=O)OMe | |
| 1031 | 18 | H | C(=O)NMe₂ | |
| 1032 | 18 | H | C(=O)OMe | |
| 1033 | 19 | H | C(=O)NMe₂ | |
| 1034 | 19 | H | C(=O)OMe | |
| 1035 | 20 | H | C(=O)NMe₂ | |
| 1036 | 20 | H | C(=O)OMe | |
| 1037 | 21 | H | C(=O)NMe₂ | |
| 1038 | 21 | H | C(=O)OMe | |
| 1039 | 22 | H | C(=O)Et | |
| 1040 | 22 | H | C(=O)NMe₂ | |
| 1041 | 22 | H | C(=O)OMe | |
| 1042 | 22 | Me | C(=O)CH₂CH₂CH₃ | |
| 1043 | 23 | H | C(=O)NMe₂ | |
| 1044 | 23 | H | C(=O)OMe | |
| 1045 | 24 | H | C(=O)NMe₂ | |
| 1046 | 24 | H | C(=O)OMe | |
| 1047 | 25 | Et | C(=O)CF₃ | |
| 1048 | 25 | H | C(=O)Me | |
| 1049 | 25 | H | C(=O)NMe₂ | |
| 1050 | 25 | H | C(=O)OMe | |
| 1051 | 26 | Et | C(=O)CF₃ | |
| 1052 | 26 | H | C(=O)Et | |
| 1053 | 26 | H | C(=O)NMe₂ | |
| 1054 | 26 | H | C(=O)OMe | |
| 1055 | 27 | H | C(=O)NMe₂ | |
| 1056 | 27 | H | C(=O)OMe | |
| 1057 | 28 | H | C(=O)NMe₂ | |
| 1058 | 28 | H | C(=O)OMe | |
| 1059 | 29 | H | C(=O)NMe₂ | |
| 1060 | 29 | H | C(=O)OMe | |
| 1061 | 30 | H | C(=O)NMe₂ | |
| 1062 | 30 | H | C(=O)OMe | |
| 1063 | 31 | H | C(=O)NMe₂ | |
| 1064 | 31 | H | C(=O)OMe | |
| 1065 | 32 | H | C(=O)NMe₂ | |
| 1066 | 32 | H | C(=O)OMe | |
| 1067 | 33 | H | C(=O)NMe₂ | |
| 1068 | 33 | H | C(=0)OMe | |
| 1069 | 34 | H | C(=O)NMe₂ | |
| 1070 | 34 | H | C(=O)OMe | |
| 1071 | 35 | Et | C(=O)CF₃ | |
| 1072 | 35 | H | C(=O)Me | |
| 1073 | 35 | H | C(=O)NMe₂ | |
| 1074 | 35 | H | C(=O)OMe | |
| 1075 | 36 | H | C(=O)NMe₂ | |
| 1076 | 36 | H | C(=O)OMe | |
| 1077 | 37 | H | C(=O)NMe₂ | |
| 1078 | 37 | H | C(=O)OMe | |
| 1079 | 38 | Et | C(=O)CF₃ | |
| 1080 | 38 | H | C(=O)Me | |
| 1081 | 38 | H | C(=O)NMe₂ | |
| 1082 | 38 | H | C(=O)OMe | |
| 1083 | 39 | H | C(=O)NMe₂ | |
| 1084 | 39 | H | C(=O)OMe | |
| 1085 | 40 | Et | C(=O)CF₃ | |
| 1086 | 40 | H | C(=O)CH(Me₂) | |
| 1087 | 40 | H | C(=O)NMe₂ | |
| 1088 | 40 | H | C(=O)OMe | |
| 1089 | 41 | H | C(=O)NMe₂ | |
| 1090 | 41 | H | C(=O)OMe | |
| 1091 | 42 | H | C(=O)NMe₂ | |
| 1092 | 42 | H | C(=O)OMe. | |
| 1093 | 43 | H | C(=O)NMe₂ | |
| 1094 | 43 | H | C(=O)OMe | |
| 1095 | 44 | H | C(=O)NMe₂ | |
| 1096 | 44 | H | C(=O)OMe | |
| 1097 | 45 | H | C(=O)NMe₂ | |
| 1098 | 45 | H | C(=O)OMe | |
| 1099 | 46 | H | C(=O)NMe₂ | |
| 1100 | 46 | H | C(=O)OMe | |
| 1101 | 47 | H | C(=O)NMe₂ | |
| 1102 | 47 | H | C(=O)OMe | |
| 1103 | 48 | Et | C(=O)CF₃ | |
| 1104 | 48 | H | C(=O)Me | |
| 1105 | 48 | H | C(=O)NMe₂ | |
| 1106 | 48 | H | C(=O)OMe | |
| 1107 | 49 | H | C(=O)NMe₂ | |
| 1108 | 49 | H | C(=O)OMe | |
| 1109 | 50 | H | C(=O)NMe₂ | |
| 1110 | 50 | H | C(=O)OMe | |
| 1111 | 51 | Et | C(=O)CF₃ | |
| 1112 | 51 | H | C(=O)Me | |
| 1113 | 51 | H | C(=O)NMe₂ | |
| 1114 | 51 | H | C(=O)OMe | |
| 1115 | 52 | H | C(=O)NMe₂ | |
| 1116 | 52 | H | C(=O)OMe | |
| 1117 | 53 | H | C(=O)NMe₂ | |
| 1118 | 53 | H | C(=O)OMe | |
| 1119 | 54 | H | C(=O)NMe₂ | |
| 1120 | 54 | H | C(=O)OMe | |
| 1121 | 55 | H | C(=O)NMe₂ | |
| 1122 | 55 | H | C(=O)OMe | |
| 1123 | 56 | H | C(=O)NMe₂ | |
| 1124 | 56 | H | C(=O)OMe | |

**Tabelle 11:**

| | | | | | |
|---|---|---|---|---|---|
| A= 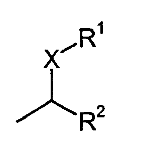 | | | | | |

| Nr. | Grundkörper | X | R¹ | R² | Smp. |
|---|---|---|---|---|---|
| 1125 | 5 | NMe | Me | Me | |
| 1126 | 5 | O | H | Me | Öl |
| 1127 | 5 | O | H | H | Öl |
| 1128 | 5 | O | H | Et | Öl |
| 1129 | 5 | O | C(=O)Me | H | Öl |
| 1130 | 5 | O | -CH₂CH(OMe)CH₂O- | | Öl |
| 1131 | 5 | O | -CH(Me)CH(Me)O- | | Öl |
| 1132 | 5 | O | -CH(Et)CH₂O- | | Öl |
| 1133 | 5 | O | -CH(CH₂SEt)CH₂O- | | Öl |
| 1134 | 5 | O | -CH2CH2O- | | |
| 1135 | 5 | S | -CH₂CH₂CH₂S- | | Öl |
| 1136 | 7 | O | -CH₂CH(OMe)CH₂O- | | |
| 1137 | 7 | O | H | Me | |
| 1138 | 7 | O | H | Et | |
| 1139 | 7 | O | -CH(Me)CH(Me)O- | | |
| 1140 | 7 | O | -CH(Et)CH₂O- | | |
| 1141 | 7 | S | -CH₂CH₂CH₂S- | | |
| 1142 | 8 | NMe | Me | Me | |
| 1143 | 8 | O | H | Me | Öl |
| 1144 | 8 | O | -CH₂CH(OMe)CH₂O- | | |
| 1145 | 8 | O | H | Me | |
| 1146 | 8 | O | H | Et | |
| 1147 | 8 | O | H | H | |
| 1148 | 8 | O | C(=O)Me | H | |
| 1149 | 8 | O | -CH2CH2O- | | |
| 1150 | 8 | O | -CH(Me)CH(Me)O- | | |
| 1151 | 8 | O | -CH(Et)CH₂O- | | |
| 1152 | 8 | S | -CH₂CH₂CH₂S- | | |
| 1153 | 22 | O | -CH₂CH(OMe)CH₂O- | | |
| 1154 | 22 | O | H | Me | |
| 1155 | 22 | O | H | Et | |
| 1156 | 22 | O | -CH(Me)CH(Me)O- | | |
| 1157 | 22 | O | -CH(Et)CH₂O- | | |
| 1158 | 22 | S | -CH₂CH₂CH₂S- | | |
| 1159 | 25 | O | -CH₂CH(OMe)CH₂O- | | |
| 1160 | 25 | O | H | Me | |
| 1161 | 25 | O | H | Et | |
| 1162 | 25 | O | -CH(Me)CH(Me)O- | | |
| 1163 | 25 | O | -CH(Et)CH₂O- | | |
| 1164 | 25 | S | -CH₂CH₂CH₂S- | | |
| 1165 | 26 | O | -CH₂CH(OMe)CH₂O- | | |
| 1166 | 26 | O | H | Me | |
| 1167 | 26 | O | H | Et | |
| 1168 | 26 | O | -CH(Me)CH(Me)O- | | |
| 1169 | 26 | O | -CH(Et)CH₂O- | | |
| 1170 | 26 | S | -CH_{Z}CH₂CH₂S- | | |
| 1171 | 35 | NMe | Me | Me | |
| 1172 | 35 | O | -CH₂CH(OMe)CH₂O- | | |
| 1173 | 35 | O | H | Me | |
| 1174 | 35 | O | H | Et | |
| 1175 | 35 | O | H | H | |
| 1176 | 35 | O | C(=O)Me | H | |
| 1177 | 35 | O | -CH2CH2O- | | |
| 1178 | 35 | O | -CH(Me)CH(Me)O- | | |
| 1179 | 35 | O | -CH(Et)CH₂O- | | |
| 1180 | 35 | S | -CH₂CH₂CH₂S- | | |
| 1181 | 38 | NMe | Me | Me | |
| 1182 | 38 | O | -CH₂CH(OMe)CH₂O- | | |
| 1183 | 38 | O | H | Me | |
| 1184 | 38 | O | H | Et | |
| 1185 | 38 | O | H | H | |
| 1186 | 38 | O | C(=O)Me | H | |
| 1187 | 38 | O | -CH2CH2O- | | |
| 1188 | 38 | O | -CH(Me)CH(Me)O- | | |
| 1189 | 38 | O | -CH(Et)CH₂O- | | |
| 1190 | 38 | S | -CH₂CH₂CH₂S- | | |
| 1191 | 40 | O | -CH₂CH(OMe)CH₂O- | | |
| 1192 | 40 | O | H | Me | |
| 1193 | 40 | O | H | Et | |
| 1194 | 40 | O | -CH(Me)CH(Me)O- | | |
| 1195 | 40 | O | -CH(Et)CH₂O- | | |
| 1196 | 40 | S | -CH₂CH₂CH₂S- | | |
| 1197 | 48 | O | -CH₂CH(OMe)CH₂O- | | |
| 1198 | 48 | O | H | Me | |
| 1199 | 48 | O | H | Et | |
| 1200 | 48 | O | -CH(Me)CH(Me)O- | | |
| 1201 | 48 | O | -CH(Et)CH₂O- | | |
| 1202 | 48 | S | -CH₂CH₂CH₂S- | | |
| 1203 | 51 | O | -CH₂CH(OMe)CH₂O- | | |
| 1204 | 51 | O | H | Me | |
| 1205 | 51 | O | H | Et | |
| 1206 | 51 | O | -CH(Me)CH(Me)O- | | |
| 1207 | 51 | O | -CH(Et)CH₂O- | | |
| 1208 | 51 | S | -CH₂CH₂CH₂S- | | |

### B. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

In den folgenden Beispielen A bis L werden Verbindungen als aktiv angesehen, wenn sie bei einer Konzentration von 500 ppm oder weniger eine Wirkung auf die Schadorganismen von 50% oder mehr aufweisen.

### Beispiel A

Abgeschnittene Stengel mit einem Blatt von Bohnenpflanzen (Phaseolus vulgaris) wurden in mit Leitungswasser gefüllte Braunglasfläschen übertragen und anschließend mit ca. 100 Spinnmilben (Tetranychus urticae) belegt. Pflanzenblatt und Spinnmilben wurden dann für 5 Sekunden in eine wäßrige Lösung des zu prüfenden und formulierten Präparates getaucht. Nach dem Abtropfen wurden Pflanze und Tiere in einer Klimakammer gelagert (16 Stunden Licht/Tag, 25 °C, 40-60 % RF). Nach 6 Tagen Lagerung wurde die Mortalität des Präparates auf alle Stadien der Spinnmilben festgestellt. Die folgenden Beispiele waren aktiv: Nr. 43, 45,97,1132.

### Beispiel B

Angekeimte Ackerbohnen-Samen (Vicia faba) mit Keimwurzeln wurden in mit Leitungswasser gefüllte Braunglasfläschen übertragen und anschließend mit ca. 100 schwarzen Bohnenblattläusen (Aphis fabae) belegt. Pflanzen und Blattläuse wurden dann für 5 Sekunden in eine wäßrige Lösung des zu prüfenden und formulierten Präparates getaucht. Nach dem Abtropfen wurden Pflanze und Tiere in einer Klimakammer gelagert (16 Stunden Licht/Tag, 25 °C, 40-60 % RF). Nach 3 und 6 Tagen Lagerung wurde die Wirkung des Präparates auf die Blattläuse festgestellt (Mortalität). Die folgenden Beispiele waren aktiv: Nr. 33, 36, 357, 1126.

### Beispiel C

Eine Petrischale, deren Boden mit Filterpapier belegt war und ca. 5 ml Nährmedium enthielt, wurde vorbereitet. Filterpapierstücke mit ca. 30 einen Tag alten Eiern der amerikanischen Tabakknospeneule (Heliothis virescens) wurden für 5 Sekunden in einer wäßrigen Lösung des zu prüfenden und formulierten Präparates getaucht und anschließend in der Petrischale ausgelegt. Weitere 200 µl der wäßrigen Lösung wurden über das Nährmedium verteilt. Nach dem Verschließen der Petrischale wurde diese bei ca. 25 °C in einer Klimakammer gelagert. Nach 6 Tagen Lagerung wurde die Mortalität des Präparates auf die Eier und die evtl. hieraus geschlüpften Larven festgestellt. Die folgenden Beispiele waren aktiv: Nr. 6, 27, 28, 29, 31, 35, 39, 43, 45, 59, 97, 226, 227, 228, 229, 351, 356, 357, 592, 593, 595, 949, 1001, 1126, 1129, 1132.

### Beispiel D

Eine Petrischale, deren Boden mit Filterpapier belegt war und ca. 5 ml Nährmedium enthielt, wurde vorbereitet. Fünf L2-Larven des ägyptischen Baumwollwurms (Spodoptera littoralis) wurden in einen kleinen Becher eingezählt. 200 µl einer wäßrigen Lösung des zu prüfenden und formulierten Präparates wurde in den Becher pipettiert. Danach wurden die behandelten Larven in die Petrischale ausgegossen und weitere 200 µl der wäßrigen Lösung über das Nährmedium verteilt. Nach dem Verschließen der Petrischale wurde diese bei ca. 25 °C in einer Klimakammer gelagert. Nach 6 Tagen Lagerung wurde die Wirkung des Präparates auf die Larven festgestellt (Mortalität). Die folgenden Beispiele waren aktiv: Nr. 27, 28,29,39,43,45,59.

### Beispiel E

Eine Petrischale, deren Boden zur Hälfte mit Filterpapier belegt war und ein angekeimtes Maiskorn auf einem feuchten Wattetupfer enthielt, wurde vorbereitet. Auf das Filterpapier wurden ca. 50, 4-5 Tage alte Eier des Maiswurzelwurms (Diabrotica undecimpunctata) übertragen. Drei Tropfen von 200 µl einer wäßrigen Lösung des zu prüfenden und formulierten Präparates wurde auf die Eier und der Rest auf das Maiskorn pipettiert. Nach dem Verschließen der Petrischale wurde diese bei ca. 25 °C in einer Klimakammer gelagert. Nach 6 Tagen Lagerung wurde die Mortalität des Präparates auf die Eier und die evtl. hieraus geschlüpften Larven festgestellt. Die folgenden Beispiele waren aktiv: Nr. 7.

### Beispiel F

Prüfungsteil A (Kontaktwirkung): In einem Glasgefäß wurde zu ca. 5000 frischgeschlüpften, aktiven (mobilen) Larven (2. Entwicklungsstadium) des Wurzelgallennematoden (Meloidogyne incognita) eine wäßrige Lösung des zu prüfenden und formulierten Präparates zugesetzt (Endvolumen 20 ml). Nach 6-tägiger Dauerexposition der Nematodenlarven wurde der prozentuale Anteil der durch die Einwirkung des Präparates bewegungslos (immobil) gewordenen Individuen im Vergleich zu den unbehandelten Kontrollen bestimmt (Prozent nematizide Kontaktwirkung).

Prüfungsteil B (Boden-Drench-Wirkung): Hierzu wurde die gesamte Lösung aus Prüfungsteil A (Wirkstoff und vorbehandelte Nematodenlarven) in einen mit 60 ml Erde gefüllten und mit drei 9 Tage alten Gurkenpflanzen (Cucumis sativus) bepflanzten Topf gegossen. Durch diese Drenchapplikation reduzierte sich der Wirkstoffgehalt bezogen auf das Bodenvolumen auf ein Drittel des Wirkstoffgehalts aus Prüfungsteil A. Nach zwei Wochen im Gewächshaus bei ca. 26 °C (zweimal tägliches Gießen) wurden die Wurzelballen der Gurkenpflanzen aus dem mit Nematoden verseuchten Erdgemisch vorsichtig ausgewaschen. Die Anzahl der Wurzelgallen pro Pflanze wurde ausgezählt und mit dem Befall unbehandelter Kontrollpflanzen verglichen. Die Berechnung der prozentualen Befallsminderung als Kriterium für die Wirkungsbeurteilung wurde nach der Abbott-Formel vorgenommen (Prozent nematizide Boden-Drench-Wirkung).
Die folgenden Beispiele waren aktiv: Nr. 6, 7, 29, 32, 34, 44, 356.

### Beispiel G

Angekeimte Ackerbohnen-Samen (Vicia faba) mit Keimwurzeln wurden in mit Leitungswasser gefüllte Braunglasfläschen übertragen. Vier Milliliter einer wäßrigen Lösung des zu prüfenden und formulierten Präparates wurden in das Braunglasfläschen hineinpipettiert. Anschließend wurde die Ackerbohne mit ca. 100 schwarzen Bohnenblattläusen (Aphis fabae) stark belegt. Pflanze und Tiere wurden dann in einer Klimakammer gelagert (16 Stunden Licht/Tag, 25 °C, 40-60 % RF). Nach 3 und 6 Tagen Lagerung wurde die wurzelsystemische Wirkung des Präparates auf die Blattläuse festgestellt (Mortalität). Die folgenden Beispiele waren aktiv: Nr. 1126.

### Beispiel H

Eine Petrischale, deren Boden mit Filterpapier belegt war und ca. 5 ml Nährmedium enthielt, wurde vorbereitet. Fünf L2-Larven der Zuckerrübeneule (Spodoptera exigua) wurden in einen kleinen Becher eingezählt. 200 µl einer wäßrigen Lösung des zu prüfenden und formulierten Präparates wurden in den Becher pipettiert. Danach wurden die behandelten Larven in die Petrischale ausgegossen und weitere 200 µl der wäßrigen Lösung wurden über das Nährmedium verteilt. Nach dem Verschließen der Petrischale wurde diese bei ca. 25 °C in einer Klimakammer gelagert. Nach 6 Tagen Lagerung wurde die Wirkung des Präparates auf die Larven festgestellt (Mortalität). Die folgenden Beispiele waren aktiv: Nr. 31, 97, 226, 227, 228, 229, 351, 356, 357, 592, 593, 595, 949, 1001, 1126, 1129, 1132.

### Beispiel I

Baumwollpflanzen wurden mit einer wäßrigen Lösung des zu prüfenden und formulierten Präparates besprüht. Nach dem Abtrocknen wurden Blätter abgeschnitten, in eine Petrischale gelegt und mit 5 L2-Larven der Zuckerrübeneule (Spodoptera exigua) besetzt. Nach 4 Tagen Lagerung bei ca. 23 °C wurde die Wirkung des Präparates auf die Larven festgestellt (Mortalität). Die folgenden Beispiele waren aktiv: Nr. 27, 28, 45, 59.

### Beispiel J

Nährmedium wurde mit einer wäßrigen Lösung des zu prüfenden und formulierten Präparates vermischt und mit 10 L1-Larven des Apfelwicklers (Carpocapsa pomonella) besetzt. Nach 14 Tagen Lagerung bei ca. 23 °C wurde die Wirkung des Präparates auf die Larven festgestellt (Mortalität). Die folgenden Beispiele waren aktiv: Nr. 28, 59.

### Beispiel K

Baumwollblätter wurden in eine Petrischale gelegt, mit jeweils 5L1-, L2-, L3- und L4-Larven der amerikanischen Tabakknospeneule (Heliothis virescens) besetzt und mit einer wäßrigen Lösung des zu prüfenden und formulierten Präparates besprüht. Nach 4 Tagen Lagerung bei ca. 25 °C wurde die Wirkung des Präparates auf die Larven festgestellt (Mortalität). Die folgenden Beispiele waren aktiv: Nr. 27

### Beispiel L

Ein Kohlblatt wurde mit einer wäßrigen Lösung des zu prüfenden und formulierten Präparates besprüht. Nach dem Abtrocknen wurde das behandelte Blatt mit Larven der Kohlmotte (Plutella xylostella) besetzt. Nach 4 Tagen Lagerung bei ca. 25 °C wurde die Wirkung des Präparates auf die Larven festgestellt (Mortalität). Die folgenden Beispiele waren aktiv: Nr. 27, 28, 45.

## Patentansprüche

1. Verbindungen der Formel (I), 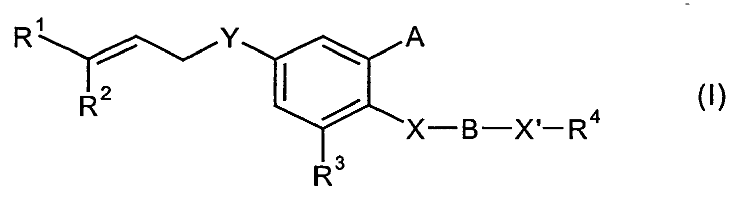 worin
R¹ und R² unabhängig voneinander Halogen bedeuten,
Y -O-, -S- oder -NH- ist,
X -O-, -S(O)ᵣ- oder -NR⁵- ist, worin r = 0, 1 oder 2 und R⁵ Wasserstoff oder C₁-C₈Alkyl bedeuten,
X' eine direkte C-C-Bindung, -O-, -S(O)ᵣ- oder -NR⁵- sind, worin r und R⁵ die oben definierte Bedeutung besitzen,
R³ Wasserstoff, Halogen, Nitro, Cyano, C₁-C₈Alkyl, C₁-C₈Haloalkyl, C₁-C₈Alkoxy, C₁-C₈Haloalkoxy oder C₃-C₁₀Cycloalkyl ist oder einer der für A definierten Bedeutungen besitzt,
A eine der Gruppen -OR⁶, -SR⁶, -NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -C(=Z)-O-N=C(NH₂)-R⁶, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)-NR⁷R⁸, -NR⁹-C(=Z)-R⁶, -NR⁹-C(=Z)-OR⁶, -NR⁹-C(=Z)-SR⁶ oder-NR⁹-C(=Z)- NR⁷R⁸ bedeutet, oder
A C₂-C₈Alkenyl ist, das gegebenenfalls mit ein oder mehreren Resten substituiert ist, wobei diese Reste Halogen, Cyano, Nitro, Hydroxy, C₁-C₈Alkyl, C₁-C₈Haloalkyl, C₁-C₈Alkoxy, C₁-C₈Haloalkoxy, Tri-(C₁-C₈-alkyl)silyl, Aryl-(C₁-C₈)-Dialkylsilyl, Diaryl-(C₁-C₈)-Alkylsilyl, Triarylsilyl, -COOR⁶, -CO-NR⁷R⁸, C₆-C₁₄Aryl und/oder Heteroaryl mit ein bis drei Ringheteroatomen sind, wobei diese Reste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A C₂-C₈Alkinyl ist, das gegebenenfalls mit ein oder mehreren Resten substituiert ist, wobei diese Reste Halogen, Cyano, Nitro, Hydroxy, C₁-C₈Alkyl, C₁-C₈Haloalkyl, C₁-C₈A1koxy, C₁-C₈Haloalkoxy, Tri-(C₁-C₈-alkyl)silyl, Aryl-(C₁-C₈)-Dialkylsilyl, Diaryl-(C₁-C₈)-Alkylsilyl, Triarylsilyl, -COOR⁶, -CO-NR⁷R⁸, C₆-C₁₄Aryl und/oder Heteroaryl mit ein bis drei Ringheteroatomen sind, wobei diese Reste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A C₆-C₁₄Aryl ist, das gegebenenfalls mit ein oder mehreren Resten substituiert ist, wobei diese Reste Halogen, Cyano, Hydroxy, Nitro, C₁-C₈Alkyl, C₁-C₈Haloalkyl, C₁-C₈Alkoxy, C₁-C₈Haloalkoxy, C₁-C₈Alkylthio, C₁-C₈Haloalkylthio, C₂-C₈Alkenyl, C₂-C₈Haloalkenyl, C₂-C₈Alkinyl, C₂-C₈Haloalkinyl, C₂-C₈-Alkyloxyalkylen, C₂-C₈-Alkylthioalkylen, C₃-C₈-Alkanoyloxyalkylen, C₁-C₈-Aminoalkylen, Phenyloxyalkylen, Phenylthioalkylen, C₃-C₈Cycloalkyl, C₆-C₁₄Aryl, Heteroaryl mit ein bis drei Ringheteroatomen, -COOR⁶, -CO-NR⁷R⁸, und/oder -S(O)ᵣR³ sind, worin r und R³ die weiter oben definierte Bedeutung besitzen, wobei die Aryl- und/oder die Heteroarylreste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A ein heterocyclischer Rest mit ein bis drei Ringheteroatomen ist, der gegebenenfalls mit ein oder mehreren Resten substituiert ist, wobei diese Reste Halogen, Cyano, Hydroxy, Nitro, C₁-C₈Alkyl, C₁-C₈Haloalkyl, C₁-C₈Alkoxy, C₁-C₈Haloalkoxy, C₁-C₈Alkylthio, C₁-C₈Haloalkylthio, C₂-C₈Alkenyl, C₂-C₈Haloalkenyl, C₂-C₈Alkinyl, C₂-C₈Haloalkinyl, C₂-C₈-Alkyloxyalkylen, C₂-C₈-Alkylthioalkylen, C₃-C₈-Alkanoyloxyalkylen, C₁-C₈-Aminoalkylen, Phenyloxyalkylen, Phenylthioalkylen, C₃-C₈Cycloalkyl, C₆-C₁₄Aryl, Heteroaryl mit ein bis drei Ringheteroatomen, -COOR⁶, - CO-NR⁷R⁸ und/oder -S(O)ᵣ-R³ sind, worin r und R³ die weiter oben definierte Bedeutung besitzen, wobei die Aryl- und/oder die Heteroarylreste wiederum mit ein oder mehreren Resten ausgewählt aus der Gruppe bestehend aus Halogen, Cyano, Nitro, Hydroxy, C₁-C₈Alkyl, C₁-C₈Haloalkyl, C₁-C₈Alkoxy und C₁-C₈Haloalkoxy substituiert sein können; oder
A ein durch ein bis sechs Gruppen substituiertes C₁-C₈Alkyl ist, dessen Substituenten ausgewählt werden aus der Gruppe bestehend aus Hydroxy, Amino, N-(C₁-C₈-Alkyl)amino, N,N-Bis-(C₁-C₈-Alkyl)amino, C₁-C₈Alkoxy, C₁-C₈Haloalkoxy, C₁-C₈Acyloxy und C₁-C₈Haloacyloxy, oder wobei zwei Substituenten zusammen auch eine gegebenenfalls ein oder mehrere Sauerstoff-, Stickstoff- und/oder Schwefelatome enthaltende Alkylenkette ausbilden können, beispielsweise ein Acetal-, Lacton- oder Lactam-Ringsystem bilden,
Z =O, =S, =N-R³³, =N-O-R³³ oder =N-NR³³R³⁴ darstellt,
R⁶, R⁷, R⁸, R⁹, R³³ und R³⁴ unabhängig voneinander Wasserstoff, C₁-C₈Alkyl, C₂-C₈Alkenyl, C₂-C₈Alkinyl, C₃-C₁₀Cycloalkyl, C₄-C₁₀Cyclalkenyl, C₄-C₁₀Cycloalkinyl, C₆-C₁₄Aryl oder Heteroaryl mit ein bis drei Ringheteroatomen bedeuten, die ihrerseits durch Halogen, Hydroxy, Cyano, Nitro, C₁-C₈Alkyl, C₂-C₈Alkenyl, C₂-C₈Alkinyl, C₃-C₁₀Cycloalkyl, C₄-C₁₀Cycloalkenyl, C₄-C₁₀Cycloalkinyl, C₆-C₁₄Aryl, halogensubstituiertes C₆-C₁₄Aryl, Heteroaryl mit ein bis drei Ringheteroatomen oder halogensubstituiertes Heteroaryl mit ein bis drei Ringheteroatomen, Amino, N-(C₁-C₈-Alkyl)amino, N,N-Bis-(C₁-C₈-Alkyl)amino, Tri-(C₁-C₈-alkyl)silyl, Aryl-(C₁-C₈)-Dialkylsilyl, Diaryl-(C₁-C₈)-Alkylsilyl, Triarylsilyl, C₁-C₈Alkoxy und/oder C₁-C₈Haloalkoxy substituiert sein können,
B eine zweiwertige Brückengruppe darstellt und Alkylen mit ein bis zwölf Kohlenstoffatomen, Cycloalkylen mit drei bis vierzehn Kohlenstoffatomen, Alkylen-Cycloalkylen mit vier bis sechsundzwanzig Kohlenstoffatomen, Alkylen-Cycloalkylen-Alkylen mit fünf bis achtunddreizig Kohlenstoffatomen darstellt, wobei diese Brückengruppen ein bis drei ethylenisch-ungesättigte Bindungen aufweisen können und/oder durch -O-, -S-, -C(=O)O- oder -NR⁵- Gruppen unterbrochen sein können,
wobei R⁵ die oben definierte Bedeutung aufweist, und wobei die Brückengruppe gegebenenfalls ein bis zehn Substituenten aufweist, die ausgewählt werden aus der Gruppe bestehend aus C₁-C₃Alkyl, Trifluormethyl oder Trichlormethyl, und
R⁴ einen einwertigen C₆-C₁₄Arylrest oder stickstoffhaltigen Heteroarylrest mit mindestens einem Ringheteroatomen darstellt, der gegebenenfalls mit ein bis vier Resten substituiert ist, die ausgewählt werden aus der Gruppe bestehend aus Halogen, Cyano, Nitro, Thiocyanato, Isocyanato, C₁-C₈Alkyl, C₂-C₈Alkenyl, C₂-C₈Alkinyl, C₃-C₁₀Cycloalkyl, C₄-C₁₀Cycloalkenyl, C₄-C₁₀Cycloalkinyl, C₆-C₁₄Aryl, stickstoffhaltigem Heteroaryl mit ein bis drei Ringheteroatomen, wobei diese Substituenten wiederum substituiert sein können mit Resten ausgewählt aus der Gruppe bestehend aus Halogen, Cyano, Nitro, C₁-C₈Alkyl, C₁-C₈Haloalkyl, C₁-C₈Alkoxy und C₁-C₈Haloalkoxy, -C(=Z)-R⁶, -C(=Z)-OR⁶, -C(=Z)-SR⁶, -C(=Z)-NR⁷R⁸, -C(=Z)-O-N=C(NH₂)-R⁶, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)- NR⁷R⁸, -NR⁹-C(=Z)-R⁶, -NR⁹-C(=Z)-OR⁶, -NR⁹-C(=Z)-SR⁶, -NR⁹-C(=Z)- NR⁷R⁸, -OR⁶, -SR⁶,-S(=O)R⁶, -S(=O)₂R⁶, -NR⁷R⁸, worin Z, R⁶, R⁷, R⁸ und R⁹ die oben definierten Bedeutungen besitzen.

2. Verbindungen der Formel (I) nach Anspruch 1, **dadurch gekennzeichnet, dass** R¹ und R² jeweils Chlor oder Brom bedeuten.

3. Verbindungen der Formel (I) nach Anspruch 1, **dadurch gekennzeichnet, dass** Y -O- ist.

4. Verbindungen der Formel (I) nach Anspruch 1, **dadurch gekennzeichnet, dass** R¹ und R² jeweils Chlor sind und Y -O- ist.

5. Verbindungen der Formel (I) nach Anspruch 1, **dadurch gekennzeichnet, dass** X -O-, -S- oder -NH- ist und X' eine direkte C-C-Bindung, -O-, -S- oder -NH- ist.

6. Verbindungen der Formel (I) nach Anspruch 5, **dadurch gekennzeichnet, dass** X und X' jeweils -O- oder X -O- und X' eine direkte C-C-Bindung bedeuten.

7. Verbindungen der Formel (I) nach Anspruch 1, **dadurch gekennzeichnet, dass** R³ Wasserstoff, Fluor, Chlor, Brom, Methyl, Trifluormethyl, Monochlormethyl, Dichlormethyl, Trichlormethyl, Methoxy, Trifluormethoxy, Monochlormethoxy, Dichlormethoxy, Trichlormethoxy, Nitro, Cyano, Cyclohexyl, Phenyl oder ein einwertiger Rest abgeleitet von Thiophen, Furan, Pyrrol, Thiazol, Oxazol, Imidazol, Isothiazol, Isoxazol, Pyrazol, 1,3,4-Oxadia-zol, 1,3,4-Thiadiazol, 1,3,4-Triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Pyrazin, Pyrimidin, Pyridazin, 1,3,5-Triazin, 1,2,4-Triazin, 1,2,4,5-Tetrazin, Chinolin, lsochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin, 4H-Chinolizin, Piperidin, Pyrrolidin, Oxazolin, Tetrahydrofuran, Tetrahydropyran, Isoxazolidin oder Thiazolidin bedeutet.

8. Verbindungen der Formel (I) nach Anspruch 1, **dadurch gekennzeichnet, dass** R³ Methyl, Trifluormethyl, Cyano, Chlor oder Brom bedeutet.

9. Verbindungen der Formel (I) nach Anspruch 1, **dadurch gekennzeichnet, dass** Z =O, =N-OH, =N-OCH₃ oder =N-CH₂-CH=CH₂ bedeutet.

10. Verbindungen der Formel (I) nach Anspruch 1, **dadurch gekennzeichnet, dass** A eine Gruppe der Formel -C(=O)-CH₃, -C(=N-OCH₃)-CH₃, -C(=N-OH)-CH₃, - C(=O)-OCH₃, -C(=O)-CH=CH-N(CH₃)₂, -C(=O)-NH-C₂H₅, -C(=O)-NH₂, -C(=O)-N(CH₃)₂, -C(=O)-NH-CH₂-CH₂-OH, -C(=O)-NH-CH(CH₃)-CH₂-OH, -C(=O)-CHBr₂, - C(=O)-N(CH₃)-O-CH₃, -C(=N-O-CH₂-CH=CH₂)-CH₃, -C(=S)-NH₂, -C(=O)-O-C₆H₅, -C(=O)-O-C₃H₇, -C(=O)-imidazolyl, -C(=O)-3-ethyl-1,2,4-oxadiazol-5-yl, -C(=O)-2-Δ -oxazolin-2-yl, -C(=O)-1-methyl-pyrazol-3-yl, -C(=O)-ethin-2-yl, -C(=O)-1-hydroxyethin-2-yl, -C(=O)-1-trimethylsilyl-ethin-2-yl, -C(=O)-1-hexyl-ethin-2-yl, -C(=O)-1-(2-chlorphenyl)-ethin-2-yl, -C(=O)-1-methoxy-ethin-2-yl und -C(=O)-ethen-2-yl bedeutet.

11. Verbindungen der Formel (I) nach Anspruch 1, **dadurch gekennzeichnet,dass** A C₂-C₈Alkenyl, C₂-C₈Alkinyl, Phenyl, Pyridyl oder Pyrimidyl bedeutet, die gegebenenfalls substituiert sind durch ein oder mehrere Halogenatome, Cyanogruppen, Nitrogruppen, Hydroxylgruppen, C₁-C₃-Alkoxyreste oder C₁-C₃Haloalkoxyreste, C₁-C₃Alkoxyreste, C₁-C₃Haloalkoxyreste, die ihrerseits gegebenenfalls substituiert sind durch ein oder mehrere Halogenatome, Cyanogruppen, Nitrogruppen, Hydroxylgruppen, C₁-C₃Alkoxyreste oder C₁-C₃ Haloalkoxyreste, oder die -OR⁶, -SR⁶, -NR⁷R⁸, -S(=O)R⁶, -S(=O)₂R⁶, -C(=Z)-R⁶,-C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)- NR⁷R⁸, -S-C(=Z)-R⁶, -S-C(=Z)-OR⁶, -S-C(=Z)-SR⁶, -S-C(=Z)- NR⁷R⁸, -NR⁹-C(=Z)-R⁶, -NR⁹-C(=Z)-OR⁶, -NR⁹-C(=Z)-SR⁶ oder -NR⁹-C(=Z)-NR⁷R⁸, bedeutet, wobei Z, R⁶, R⁷, R⁸ und R⁹ die in Anspruch 1 definierten Bedeutungen besitzen, oder die durch ein oder mehrere Hydroxylgruppen, C₁-C₃-Alkoxyreste, C₁-C₃Haloalkoxyreste, C₁-C₃Acyloxyreste oder C₁-C₃Haloacyloxyreste substituiertes C₁-C₈-Alkyl bedeutet, oder die eine Gruppe der Formeln Q¹ - Q¹⁰ bedeutet 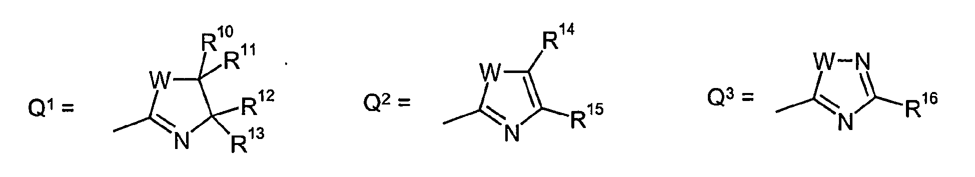 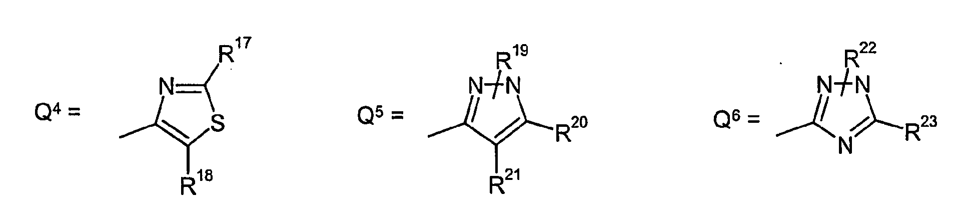 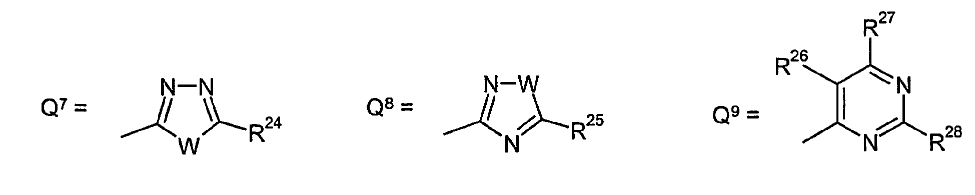 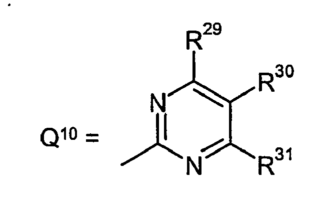 worin W Sauerstoff oder Schwefel ist, R¹⁰ bis R¹⁸, R²⁰ und R²¹, R²³ bis R³¹ Wasserstoff, C₁-C₆Alkyl, C₃-C₇Cycloalkyl, C₂-C₆Alkenyl, C₄-C₇Cycloalkenyl, Pyridyl oder Phenyl bedeuten, wobei die C₁-C₆Alkyl-, C₃-C₇Cycloalkyl-, C₂-C₆Alkenyl- oder die C₄-C₇Cycloalkenylreste gegebenenfalls ein- oder mehrfach, vorzugsweise ein-, zwei- oder dreifach substitiert sind durch Halogen, Cyano, -OR⁶, -SR⁶, -S(O)R⁶, - S(O)₂R⁶ und/oder -NR⁷R⁸, wobei die Reste R⁶, R⁷ und R⁸ die oben definierte Bedeutung aufweisen, und wobei Phenyl oder Pyridyl gegebenenfalls ein- oder mehrfach, vorzugsweise ein-, zwei- oder dreifach substitiert sind durch Halogen, Cyano, Nitro, -OR⁶, -SR⁶, -S(O)R⁶, -S(O)₂R⁶,-NR⁷R⁸, C₁-C₄Alkyl, C₁-C₄Haloalkyl, C₂-C₄Alkenyl und/oder C₂-C₄Haloalkyl.

12. Verbindungen der Formel (1) nach Anspruch 1, **dadurch gekennzeichnet, dass** B eine Gruppe der Formeln P¹ bis P⁶ bedeutet 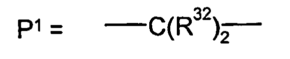 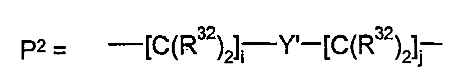 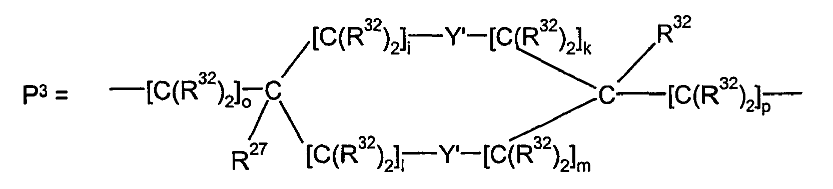 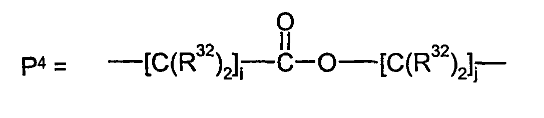 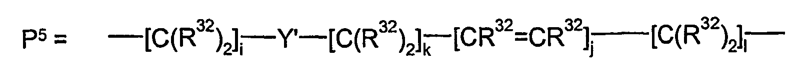 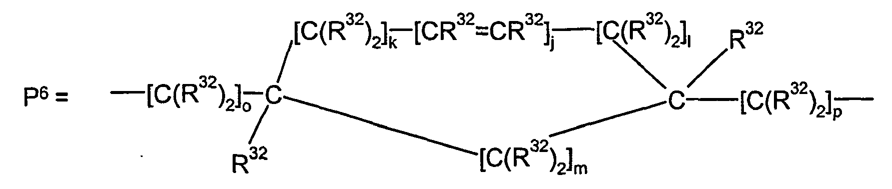 worin R³² jeweils unabhängig voneinander Wasserstoff, C₁-C₃Alkyl oder Trifluormethyl bedeuten,
Y' jeweils unabhängig voneinander eine direkte C-C-Bindung, -O-, -S- oder -NHbedeuten,
i eine ganze Zahl von 1 bis 6 ist,
j eine ganze Zahl von 1 bis 6 ist,
k eine ganze Zahl von 0 bis 2 ist,
l eine ganze Zahl von 0 bis 2 ist,
m eine ganze Zahl von 0 bis 2 ist,
o eine ganze Zahl von 0 bis 1 ist, und
p eine ganze Zahl von 0 bis 1 ist.

13. Verbindungen der Formel (I) nach Anspruch 1, **dadurch gekennzeichnet, dass** B eine Gruppe der Formel -C_{q}H_{2q-} ist, und q eine ganze Zahl von 2 bis 4 ist, insbesondere -(CH₂)₂-, -(CH₂)₃- und -(CH₂)₄-.

14. Verbindungen der Formel (I) nach Anspruch 1, **dadurch gekennzeichnet, dass** R⁴ C₆-C₁₄Aryl bedeutet, das gegebenenfalls substituiert ist durch ein bis drei Reste ausgewählt aus der Gruppe bestehend aus Halogen, Cyano, Nitro, Thiocyanato, Isocyanato, C₁-C₆-Alkyl, C₃-C₈-Cycloalkyl, C₂-C₈-Alkenyl, C₄-C₈-Cycloalkenyl und C₂-C₈-Alkinyl, wobei ein oder mehrere Wasserstoffatome der C₁-C₆-Alkyl-, C₃-C₈-Cycloalkyl-, C₂-C₈-Alkenyl-, C₄-C₈-Cycloalkenyl- und C₂-C₈-Alkinylreste durch Halogen und/oder Cyano substituiert sein können.

15. Verbindungen der Formel (I) nach Anspruch 1, **dadurch gekennzeichnet, dass** R⁴ ausgewählt wird aus der Gruppe bestehend aus Phenyl, Pyridyl, Pyrimidyl, Pyrazinyl, Pyrazolyl oder Naphthyl, die gegebenenfalls substituiert sind durch einen oder mehrere Reste ausgewählt aus der Gruppe bestehend aus Halogen, Cyano, Nitro, C₁-C₃-Alkyl, C₁-C₃-Haloalkyl, C₁-C₃-Alkoxy, C₁-C₃-Haloalkoxy, -C(=Z)-R⁶, -C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, - S-C(=Z)-SR⁶, -S-C(=Z)-NR⁷R⁸, -NR⁹-C(=Z)-R⁶, -NR⁹-C(=Z)-OR⁶, -NR⁹-C(=Z)-SR⁶, -NR⁹-C(=Z)- NR⁷R⁸, -OR⁶, -SR⁶, -S(=O)R⁶ und -S(=O)₂R⁶ bedeuten, wobei Z, R⁶, R⁷, R⁸ und R⁹ die in Anspruch 1 definierten Bedeutungen besitzen.

16. Verbindungen der Formel (I) nach Anspruch 1, **dadurch gekennzeichnet, dass** R⁴ Phenyl oder Pyridyl ist, das ein bis drei Substituenten trägt aus der Gruppe Halogen, Cyano, Nitro, C₁-C₃-Alkyl und C₁-C₃-Haloalkyl oder von Kombinationen dieser Substituenten.

17. Verbindungen der Formel (I) nach Anspruch 1, **dadurch gekennzeichnet, dass** R⁴ Trifluoromethylphenyl, Chlorophenyl, Nitrophenyl, Nitropyridyl, Trifluoromethylpyridyl, Di-trifluoromethyl-pyridyl, Chloropyridyl, Dichloropyridyl, Chloro-trifluoromethyl-pyridyl, Trifluoromethyl-pyrimidyl, Di-trifluoromethyl-pyrimidyl, Methyl-trifluoromethyl-pyrimidyl oder Trifluoromethyl-pyrazolyl bedeutet.

18. Verfahren zur Herstellung von Verbindungen der allgemeinen Formel (I) nach Anspruch 1 umfassend die Schritte: 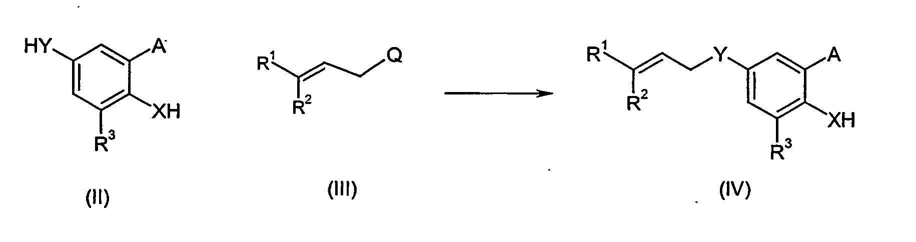 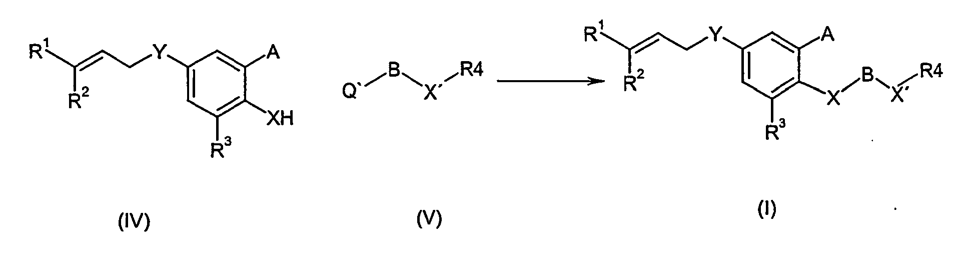
a) Umsetzung der Verbindungen der Formeln II und III zur Verbindung der Formel IV und
b) Umsetzung der Verbindungen der Formeln IV und V zur Verbindung der Formel 1,
worin R¹, R², R³, R⁴, A, Y, X und X'die in Anspruch 1 definierten Bedeutungen besitzen und Q und Q' Abgangsgruppen darstellen.

19. Mittel mit insektizider, akarizider, ixodizider, nematizider, molluskizider und/oder fungizider Wirkung **gekennzeichnet durch** einen Gehalt an mindestens einer Verbindung der Formel (I) oder eines ihrer Salze nach Anspruch 1.

20. Mittel nach Anspruch 19 in Mischung mit Träger- und/oder oberflächenaktiven Stoffen.

21. Verwendung der Verbindungen der Formel (I) nach Anspruch 1 zur Bekämpfung von tierischen Schädlingen.

22. Verwendung der Verbindungen der Formel (I) nach Anspruch 1 zur Bekämpfung von Schadorganismen in transgenen Kulturpflanzen.

23. Verwendung der Verbindungen der Formel (I) nach Anspruch 1 zur Abwehr oder Vertreibung von Schädlingen und/oder Lästlingen von Pflanzen.

24. Verwendung der Verbindungen der Formel (I) nach Anspruch 1 zur Herstellung eines Arzneimittels, insbesondere eines Tierarzneimittels.

25. Verwendung der Verbindungen der Formel (I) nach Anspruch 1 zur Herstellung eines Arzneimittels zur Bekämpfung von Endo- und Ektoparasiten.

26. Verfahren zur Bekämpfung von tierischen Schädlingen, wobei man die tierischen Schädlinge direkt oder indirekt in Kontakt mit einer Verbindung der Formel (I) nach Anspruch 1 bringt.

27. Verfahren zur Bekämpfung von Schadorgansimen in transgenen Kulturpflanzen, wobei man die Schadorganismen direkt oder indirekt in Kontakt mit einer Verbindung der Formel (I) nach Anspruch 1 bringt.

## Claims

1. Compounds of the formula (I) 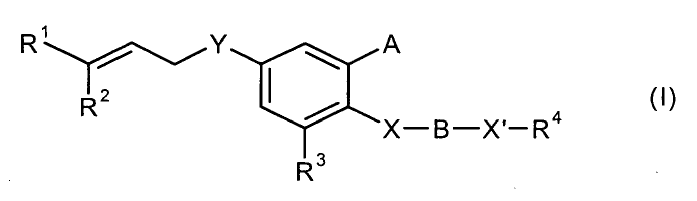 in which
R¹ and R² independently of one another are halogen,
Y is -O-, -S- or -NH-,
X is -O-, -S(O)ᵣ- or -NR⁵-, where r = 0, 1 or 2 and R⁵ is hydrogen or C₁-C₈-alkyl,
X' is a direct C-C bond, -O-, -S(O)ᵣ- or -NR⁵-, where r and R⁵ are as defined above,
R³ is hydrogen, halogen, nitro, cyano, C₁-C₈-alkyl, C₁-C₈-haloalkyl, C₁-C₈-alkoxy, C₁-C₈-haloalkoxy or C₃-C₁₀-cycloalkyl or has one of the meanings defined for A,
A is one of the groups -OR⁶, -SR⁶, -NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -C(=Z)-O-N=C(NH₂)-R⁶, -O-C(=Z)-R⁶, -O-C(=Z)-OR⁶, -O-C(=Z)-SR⁶, -O-C(=Z)- NR⁷R⁸, -S-C(=Z)-R⁶, -S-C(=Z)-OR⁶, -S-C(=Z)-SR⁶, -S-C(=Z)- NR⁷R⁸, NR⁹-C(=Z)-R⁶, -NR⁹-C(=Z)-OR⁶, -NR⁹-C(=Z)-SR⁶ or -NR⁹-C(=Z)- NR⁷R⁸, or
A is C₂-C₈-alkenyl which is unsubstituted or substituted by one or more radicals, these radicals being halogen, cyano, nitro, hydroxyl, C₁-C₈-alkyl, C₁-C₈-haloalkyl, C₁-C₈-alkoxy, C₁-C₈-haloalkoxy, tri-(C₁-C₈-alkyl)silyl, aryl-(C₁-C₈)-dialkylsilyl, diaryl-(C₁-C₈)-alkylsilyl, triarylsilyl, -COOR⁶, -CO-NR⁷R⁸, C₆-C₁₄-aryl and/or heteroaryl having one to three ring heteroatoms, where these radicals for their part may be substituted by one or more radicals selected from the group consisting of halogen, cyano, nitro, hydroxyl, C₁-C₈-alkyl, C₁-C₈-haloalkyl, C₁-C₈-alkoxy and C₁-C₈-haloalkoxy; or
A is C₂-C₈-alkynyl which is unsubstituted or substituted by one or more radicals, these radicals being halogen, cyano, nitro, hydroxyl, C₁-C₈-alkyl, C₁-C₈-haloalkyl, C₁-C₈-alkoxy, C₁-C₈-haloalkoxy, tri-(C₁-C₈-alkyl)silyl, aryl-(C₁-C₈)-dialkylsilyl, diaryl-(C₁-C₈)-alkylsilyl, triarylsilyl, -COOR⁶, -CO-NR⁷R⁸, C₆-C₁₄-aryl and/or heteroaryl having one to three ring heteroatoms, where these radicals for their part may be substituted by one or more radicals selected from the group consisting of halogen, cyano, nitro, hydroxyl, C₁-C₈-alkyl, C₁-C₈-haloalkyl, C₁-C₈-alkoxy and C₁-C₈-haloalkoxy; or
A is C₆-C₁₄-aryl which is unsubstituted or substituted by one or more radicals, these radicals being halogen, cyano, hydroxyl, nitro, C₁-C₈-alkyl, C₁-C₈-haloalkyl, C₁-C₈-alkoxy, C₁-C₈-haloalkoxy, C₁-C₈-alkylthio, C₁-C₈-haloalkylthio, C₂-C₈-alkenyl, C₂-C₈-haloalkenyl, C₂-C₈-alkynyl, C₂-C₈-haloalkynyl, C₂-C₈-alkyloxyalkylene, C₂-C₈-alkylthioalkylene, C₃-C₈-alkanoyloxyalkylene, C₁-C₈-aminoalkylene, phenyloxyalkylene, phenylthioalkylene, C₃-C₈-cycloalkyl, C₆-C₁₄-aryl, heteroaryl having one to three ring heteroatoms, -COOR⁶, -CO-NR⁷R⁸ and/or -S(O)ᵣ R³, where r and R³ are as defined above, where the aryl and/or the heteroaryl radicals for their part may be substituted by one or more radicals selected from the group consisting of halogen, cyano, nitro, hydroxyl, C₁-C₈-alkyl, C₁-C₈-haloalkyl, C₁-C₈-alkoxy and C₁-C₈-haloalkoxy; or
A is a heterocyclic radical having one to three ring heteroatoms which is unsubstituted or substituted by one or more radicals, these radicals being halogen, cyano, hydroxyl, nitro, C₁-C₈-alkyl, C₁-C₈-haloalkyl, C₁-C₈-alkoxy, C₁-C₈-haloalkoxy, C₁-C₈-alkylthio, C₁-C₈-haloalkylthio, C₂-C₈-alkenyl, C₂-C₈-haloalkenyl, C₂-C₈-alkynyl, C₂-C₈-haloalkynyl, C₂-C₈-alkyloxyalkylene, C₂-C₈-alkylthioalkylene, C₃-C₈-alkanoyloxyalkylene, C₁-C₈-aminoalkylene, phenyloxyalkylene, phenylthioalkylene, C₃-C₈-cycloalkyl, C₆-C₁₄-aryl, heteroaryl having one to three ring heteroatoms, -COOR⁶, -CO-NR⁷R⁸ and/or -S(O)ᵣ-R³, where r and R³ are as defined above, where the aryl and/or the heteroaryl radicals for their part may be substituted by one or more radicals selected from the group consisting of halogen, cyano, nitro, hydroxyl, C₁-C₈-alkyl, C₁-C₈-haloalkyl, C₁-C₈-alkoxy and C₁-C₈-haloalkoxy; or
A is C₁-C₈-alkyl which is substituted by one to six groups, its substituents being selected from the group consisting of hydroxyl, amino, N-(C₁-C₈-alkyl)amino, N,N-bis-(C₁-C₈-alkyl)amino, C₁-C₈-alkoxy, C₁-C₈-haloalkoxy, C₁-C₈-acyloxy and C₁-C₈-haloacyloxy, or where two substituents together may also form an alkylene chain which may contain one or more oxygen, nitrogen and/or sulfur atoms, for example an acetal, lactone or lactam ring system,
Z is =O, =S, =N-R³³, =N-O-R³³ or =N-NR³³R³⁴,
R⁶, R⁷, R⁸, R⁹, R³³ and R³⁴ independently of one another are hydrogen, C₁-C₈-alkyl, C₂-C₈-alkenyl, C₂-C₈-alkynyl, C₃-C₁₀-cycloalkyl, C₄-C₁₀-cycloalkenyl, C₄-C₁₀-cycloalkynyl, C₆-C₁₄-aryl or heteroaryl having one to three ring heteroatoms which for their part may be substituted by halogen, hydroxyl, cyano, nitro, C₁-C₈-alkyl, C₂-C₈-alkenyl, C₂-C₈-alkynyl, C₃-C₁₀-cycloalkyl, C₄-C₁₀-cycloalkenyl, C₄-C₁₀-cycloalkynyl, C₆-C₁₄-aryl, halogen-substituted C₆-C₁₄-aryl, heteroaryl having one to three ring heteroatoms or halogen-substituted heteroaryl having one to three ring heteroatoms, amino, N-(C₁-C₈-alkyl)amino, N,N-bis-(C₁-C₈-alkyl)amino, tri-(C₁-C₈-alkyl)silyl, aryl-(C₁-C₈)-dialkylsilyl, diaryl-(C₁-C₈)-alkylsilyl, triarylsilyl, C₁-C₈-alkoxy and/or C₁-C₈-haloalkoxy,
B is a divalent bridge and is alkylene having one to twelve carbon atoms, cycloalkylene having three to fourteen carbon atoms, alkylene-cycloalkylene having four to twenty-six carbon atoms, alkylene-cycloalkylene-alkylene having five to thirty-eight carbon atoms, where these bridges may have one to three ethylenically unsaturated bonds and/or may be interrupted by -O-, -S-, -C(=O)O- or -NR⁵-groups, where R⁵ is as defined above and where the bridge may be unsubstituted or substituted by one to ten substituents selected from the group consisting of C₁-C₃-alkyl, trifluoromethyl and trichloromethyl, and
R⁴ is a monovalent C₆-C₁₄-aryl radical or nitrogen-containing heteroaryl radical having at least one ring heteroatom which is unsubstituted or substituted by one to four radicals selected from the group consisting of halogen, cyano, nitro, thiocyanato, isocyanato, C₁-C₈-alkyl, C₂-C₈-alkenyl, C₂-C₈-alkynyl, C₃-C₁₀-cycloalkyl, C₄-C₁₀-cycloalkenyl, C₄-C₁₀-cyaoalkynyl, C₆-C₁₄-aryl, nitrogen-containing heteroaryl having one to three ring heteroatoms, where these substituents for their part may be substituted by radicals selected from the group consisting of halogen, cyano, nitro, C₁-C₈-alkyl, C₁-C₈-haloalkyl, C₁-C₈-alkoxy, C₁-C₈-haloalkoxy, -C(=Z)-R⁶, -C(=Z)-OR⁶, -C(=Z)-SR⁶, -C(=Z)-NR⁷R⁸, -C(=Z)-O-N=C(NH₂)-R⁶, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)- NR⁷R⁸, -NR⁹-C(=Z)-R⁶, -NR⁹-C(=Z)-OR⁶, -NR⁹-C(=Z)-SR⁶, -NR⁹-C(=Z)- NR⁷R⁸, -OR⁶, -SR⁶, -S(=O)R⁶, -S(=O)₂R⁶, -NR⁷R⁸, where Z, R⁶, R⁷, R⁸ and R⁹ are as defined above.

2. Compounds of the formula (I) according to Claim 1, **characterized in that** R¹ and R² are each chlorine or bromine.

3. Compounds of the formula (I) according to Claim 1, **characterized in that** Y is -O-.

4. Compounds of the formula (I) according to Claim 1, **characterized in that** R¹ and R² are each chlorine and Y is -O-.

5. Compounds of the formula (I) according to Claim 1, **characterized in that** X is -O-, -S- or -NH- and X' is a direct C-C bond, -O-, -S- or -NH-.

6. Compounds of the formula (I) according to Claim 5, **characterized in that** X and X' are each -O- or X is -O- and X' is a direct C-C bond.

7. Compounds of the formula (I) according to Claim 1, **characterized in that** R³ is hydrogen, fluorine, chlorine, bromine, methyl, trifluoromethyl, monochloromethyl, dichloromethyl, trichloromethyl, methoxy, trifluoromethoxy, monochloromethoxy, dichloromethoxy, trichloromethoxy, nitro, cyano, cyclohexyl, phenyl or a monovalent radical derived from thiophene, furan, pyrrole, thiazole, oxazole, imidazole, isothiazole, isoxazole, pyrazole, 1,3,4-oxadiazole, 1,3,4-thiadiazole, 1,3,4-triazole, 1,2,4-oxadiazole, 1,2,4-thiadiazole, 1,2,4-triazole, 1,2,3-triazole, 1,2,3,4-tetrazole, benzo[b]thiophene, benzo[b]furan, indole, benzo[c]thiophene, benzo[c]furan, isoindole, benzoxazole, benzothiazole, benzimidazole, benzisoxazole, benzisothiazole, benzopyrazole, benzothiadiazole, benzotriazole, dibenzofuran, dibenzothiophene, carbazole, pyridine, pyrazine, pyrimidine, pyridazine, 1,3,5-triazine, 1,2,4-triazine, 1,2,4,5-tetrazine, quinoline, isoquinoline, quinoxaline, quinazoline, cinnoline, 1,8-naphthyridine, 1,5-naphthyridine, 1,6-naphthyridine, 1,7-naphthyridine, phthalazine, pyridopyrimidine, purine, pteridine, 4H-quinolizine, piperidine, pyrrolidine, oxazoline, tetrahydrofuran, tetrahydropyran, isoxazolidine or thiazolidine.

8. Compounds of the formula (I) according to Claim 1, **characterized in that** R³ is methyl, trifluoromethyl, cyano, chlorine or bromine.

9. Compounds of the formula (I) according to Claim 1, **characterized in that** Z is =O, =N-OH, =N-OCH₃ or =N-CH₂-CH=CH₂.

10. Compounds of the formula (I) according to Claim 1, **characterized in that** A is a group of the formula -C(=O)-CH₃, -C(=N-OCH₃)-CH₃, -C(=N-OH)-CH₃, -C(=O)-OCH₃, -C(=O)-CH=CH-N(CH₃)₂, -C(=O)-NH-C₂H₅, -C(=O)-NH₂, -C(=O)-N(CH₃)₂, -C(=O)-NH-CH₂-CH₂-OH, -C(=O)-NH-CH(CH₃)-CH₂-OH, -C(=O)-CHBr₂, -C(=O)-N(CH₃)-O-CH₃, -C(=N-O-CH₂-CH=CH₂)-CH₃, -C(=S)-NH₂, -C(=O)-O-C₆H₅, -C(=O)-O-C₃H₇, -C(=O)-imidazolyl, -C(=O)-3-ethyl-1,2,4-oxadiazol-5-yl, -C(=O)-2-Δ -oxazolin-2-yl, -C(=O)-l-methylpyrazol-3-yl, -C(=O)-ethyn-2-yl, -C(=O)-l-hydroxyethyn-2-yl, -C(=O)-1-trimethylsilylethyn-2-yl, -C(=O)-1-hexylethyn-2-yl, -C(=O)-1-(2-chlorophenyl)ethyn-2-yl, -C(=O)-l-methoxyethyn-2-yl and -C(=O)-ethen-2-yl.

11. Compounds of the formula (I) according to Claim 1, **characterized in that** A is C₂-Cₛ-alkenyl, C₂-Cₛ-alkynyl, phenyl, pyridyl or pyrimidyl which are unsubstituted or substituted by one or more halogen atoms, cyano groups, nitro groups, hydroxyl groups, C₁-C₃-alkoxy radicals or C₁-C₃-haloalkoxy radicals, C₁-C₃-alkoxy radicals, C₁-C₃-haloalkoxy radicals which for their part are unsubstituted or substituted by one or more halogen atoms, cyano groups, nitro groups, hydroxyl groups, C₁-C₃-alkoxy radicals or C₁-C₃ haloalkoxy radicals, or is -OR⁶, -SR⁶, -NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)- NR⁷R⁸, -S-C(=Z)-R⁶, -S-C(=Z)-OR⁶, -S-C(=Z)-SR⁶, -S-C(=Z)- NR⁷R⁸, -NR⁹-C(=Z)-R⁶, -NR⁹-C(=Z)-OR⁶, -NR⁹-C(=Z)-SR⁶ or -NR⁹-C(=Z)-NR⁷R⁸, where Z, R⁶, R⁷, R⁸ and R⁹ are as defined in Claim 1, or is C₁-C₈-alkyl which is substituted by one or more hydroxyl groups, C₁-C₃-alkoxy radicals, C₁-C₃-haloalkoxy radicals , C₁-C₃-acyloxy radicals or C₁-C₃-haloacyloxy radicals, or is a group of the formulae Q¹ - Q¹⁰ 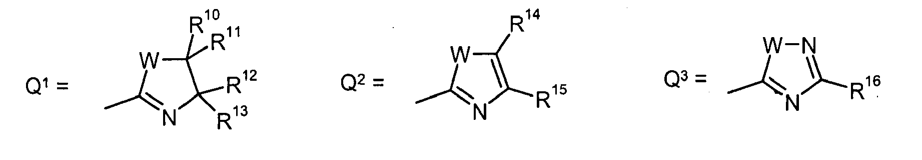 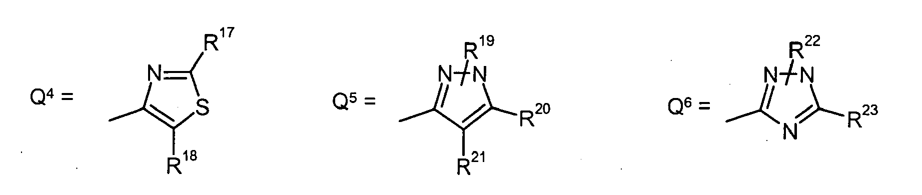 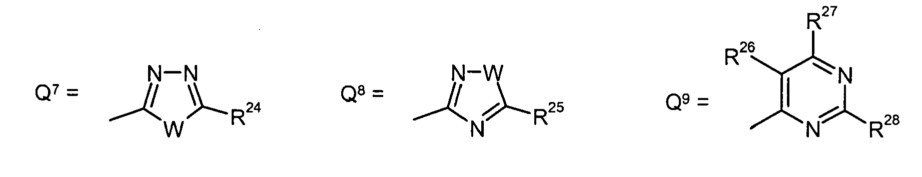 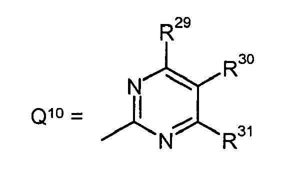 in which W is oxygen or sulfur, R¹⁰ to R¹⁸, R²⁰ and R²¹, R²³ to R³¹ are hydrogen, C₁-C₆-alkyl, C₃-C₇-cycloalkyl, C₂-C₆-alkenyl, C₄-C₇-cycloalkenyl, pyridyl or phenyl, where the C₁-C₆-alkyl, C₃-C₇-cycloalkyl, C₂-C₆-alkenyl and C₄-C₇-cycloalkenyl radicals are unsubstituted or mono- or polysubstituted, preferably mono-, di- or trisubstituted by halogen, cyano, -OR⁶, -SR⁶, -S(O)R⁶, -S(O)_{2R}⁶ and/or -NR⁷R⁸, where the radicals R⁶, R⁷ and R⁸ are as defined above, and where phenyl and pyridyl are unsubstituted or mono- or polysubstituted, preferably mono-, di- or trisubstituted by halogen, cyano, nitro, -OR⁶, -SR⁶, -S(O)R⁶, -S(O)₂R⁶,-NR⁷R⁸, C₁-C₄-alkyl, C₁-C₄-haloalkyl, C₂-C₄-alkenyl and/or C₂-C₄-haloalkyl.

12. Compounds of the formula (I) according to Claim 1, **characterized in that** B is a group of the formulae P¹ to P⁶ 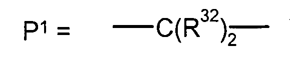 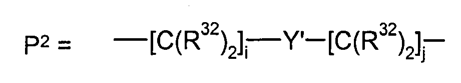 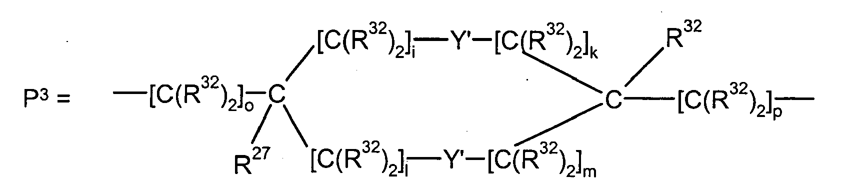 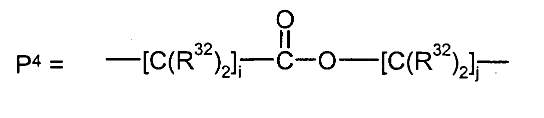 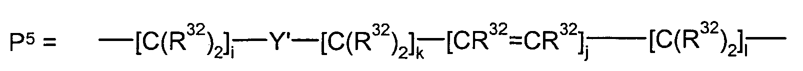 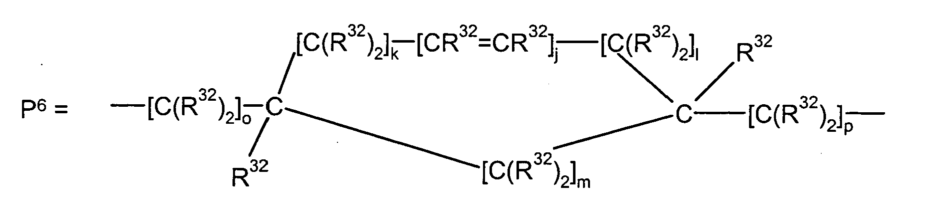 in which R³² in each case independently of the others is hydrogen, C₁-C₃-alkyl or trifluoromethyl,
Y' in each case independently of the others is a direct C-C bond, -O-, -S- or -NH-,
i is an integer from 1 to 6,
j is an integer from 1 to 6,
k is an integer from 0 to 2,
l is an integer from 0 to 2,
m is an integer from 0 to 2,
o is an integer from 0 to 1 and
p is an integer from 0 to 1.

13. Compounds of the formula (I) according to Claim 1, **characterized in that** B is a group of the formula -C_{q}H_{2q}- and q is an integer from 2 to 4, in particular -(CH₂)₂-, -(CH₂)₃- or -(CH₂)₄-.

14. Compounds of the formula (I) according to Claim 1, **characterized in that** R⁴ is C₆-C₁₄-aryl which is unsubstituted or substituted by one to three radicals selected from the group consisting of halogen, cyano, nitro, thiocyanato, isocyanato, C₁-C₆-alkyl, C₃-C₈-cycloalkyl, C₂-C₈-alkenyl, C₄-C₈-cycloalkenyl and C₂-C₈-alkynyl, where one or more hydrogen atoms of the C₁-C₆-alkyl, C₃-C₈-cycloalkyl, C₂-C₈-alkenyl, C₄-C₈-cycloalkenyl and C₂-C₈-alkynyl radicals may be substituted by halogen and/or cyano.

15. Compounds of the formula (I) according to Claim 1, **characterized in that** R⁴ is selected from the group consisting of phenyl, pyridyl, pyrimidyl, pyrazynyl, pyrazolyl and naphthyl which are unsubstituted or substituted by one or more radicals selected from the group consisting of halogen, cyano, nitro, C₁-C₃-alkyl, C₁-C₃-haloalkyl, C₁-C₃-alkoxy, C₁-C₃-haloalkoxy, -C(=Z)-R⁶, -C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)-NR⁷R⁸, -NR⁹-C(=Z)-R⁶, -NR⁹-C(=Z)-OR⁶, -NR⁹-C(=Z)-SR⁶, -NR⁹-C(=Z)- NR⁷R⁸, -OR⁶, -SR⁶, -S(=O)R⁶ and -S(=O)₂R⁶, where Z, R⁶, R⁷, R⁸ and R⁹ are as defined in Claim 1.

16. Compounds of the formula (I) according to Claim 1, **characterized in that** R⁴ is phenyl or pyridyl which carries one to three substituents from the group consisting of halogen, cyano, nitro, C₁-C₃-alkyl and C₁-C₃-haloalkyl or of combinations of these substituents.

17. Compounds of the formula (I) according to Claim 1, **characterized in that** R⁴ is trifluoromethylphenyl, chlorophenyl, nitrophenyl, nitropyridyl, trifluoromethylpyridyl, di(trifluoromethyl)pyridyl, chloropyridyl, dichloropyridyl, chlorotrifluoromethylpyridyl, trifluoromethylpyrimidyl, di(trifluoromethyl)-pyrimidyl, methyltrifluoromethylpyrimidyl or trifluoromethylpyrazolyl.

18. Process for preparing compounds of the formula (I) according to Claim 1, which process comprises the following steps: 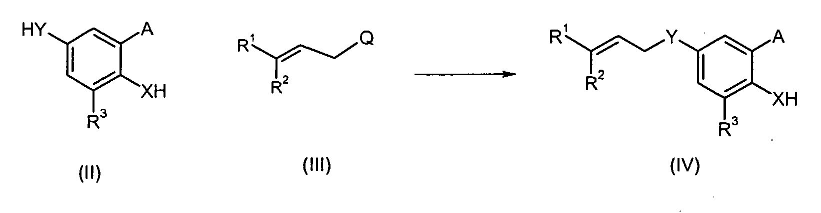 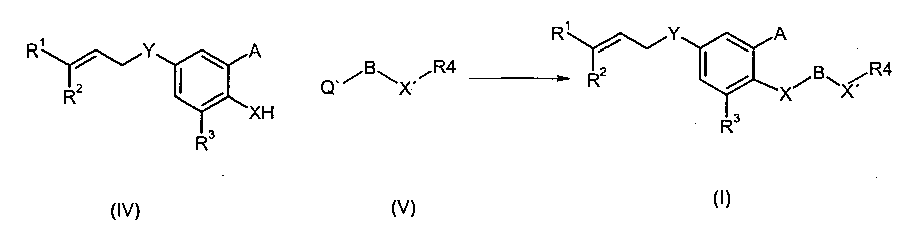
a) a reaction of the compounds of the formulae II and III to give the compound of the formula IV and
b) a reaction of the compounds of the formulae IV and V to give the compound of the formula I,
where R¹, R², R³, R⁴, A, Y, X and X' are as defined in claim 1 and Q and Q' are leaving groups.

19. Composition having insecticidal, acaricidal, ixodicidal, nematicidal, molluscicidal and/or fungicidal action, **characterized by** a content of at least one compound of the formula (I) or a salt thereof according to Claim 1.

20. Composition according to Claim 19 in a mixture with carriers and/or surfactants.

21. Use of the compounds of the formula (I) according to Claim 1 for controlling animal pests.

22. Use of the compounds of the formula (I) according to Claim 1 for controlling harmful organisms in transgenic crop plants.

23. Use of the compounds of the formula (I) according to Claim 1 for fending-off or warding-off pests, including nuisance pests, of plants.

24. Use of the compounds of the formula (I) according to Claim 1 for preparing a medicament, in particular a veterinary medicament.

25. Use of the compounds of the formula (I) according to Claim 1 for preparing a medicament for controlling endo- and ectoparasites.

26. Method for controlling animal pests wherein the animal pests are brought into direct or indirect contact with a compound of the formula (I) according to Claim 1.

27. Method for controlling harmful organisms in transgenic crop plants wherein the harmful organisms are brought into direct or indirect contact with a compound of the formula (I) according to Claim 1.

## Revendications

1. Composés de formule (I) : 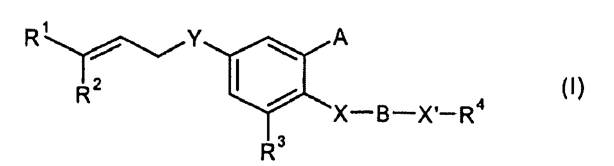 dans laquelle
R¹ et R² représentent, indépendamment l'un de l'autre, un halogène,
Y représente -O-, -S- ou -NH-,
X représente -O-, -S(O)ᵣ- ou -NR⁵-, où r a la valeur 0, 1 ou 2, et R⁵ représente l'hydrogène ou un reste alkyle en C₁ à C₈,
X' représente une liaison directe C-C, -O-, -S(O)ᵣ- ou -NR⁵-, où r et R⁵ ont la définition indiquée ci-dessus,
R³ représente l'hydrogène, un halogène, un groupe nitro, cyano, un reste alkyle en C₁ à C₈, halogénalkyle en C₁ à C₈, alkoxy en C₁ à C₈, halogénalkoxy en C₁ à C₈ ou cycloalkyle en C₃ à C₁₀ ou possède l'une des définitions données pour A,
A représente l'un des groupes -OR⁶, -SR⁶, NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -C(=Z) -O-N=C(NH₂) -R⁶, -O-C(=Z)-R⁶, -O-C(=Z)-OR⁶, -O-C(=Z) -SR⁶, -O-C(=Z)-NR⁷R⁸, -S-C(=Z)-R⁶, -S-C(=Z)-OR⁶, -S-C(=Z)-SR⁶, -S-C(=Z)-NR⁷R⁸, -NR⁹-C(=Z)-R⁶, -NR⁹-C(=Z) -OR⁶, -NR⁹-C(=Z) -SR⁶ ou -NR⁹-C(=Z)-NR⁷R⁸, ou bien
A est un reste alcényle en C₂ à C₈ qui est éventuellement substitué avec un ou plusieurs restes, ces restes étant des restes halogéno, cyano, nitro, hydroxy, alkyle en C₁ à C₈, halogénalkyle en C₁ à C₈, alkoxy en C₁ à C₈, halogénalkoxy en C₁ à C₈, tri (alkyle en C₁ à C₈) silyle, aryl-di(alkyle en C₁ à C₈)silyle, diaryl-(alkyle en C₁ à C₈)silyle, triarylsilyle, -COOR⁶, -CO-NR⁷R⁸, aryle en C₆ à C₁₄ et/ou hétéroaryle ayant un à trois hétéroatomes dans le noyau, ces restes pouvant eux-mêmes être substitués avec un ou plusieurs restes choisis dans le groupe constitué d'halogéno, cyano, nitro, hydroxy, alkyle en C₁ à C₈, halogénalkyle en C₁ à C₈, alkoxy en C₁ à C₈ et halogénalkoxy en C₁ à C₈; ou bien
A représente un reste alcynyle en C₂ à C₈, qui est éventuellement substitué avec un ou plusieurs restes, ces restes étant des restes halogéno, cyano, nitro, hydroxy, alkyle en C₁ à C₈, halogénalkyle en C₁ à C₈, alkoxy en C₁ à C₈, halogénalkoxy en C₁ à C₈, tri(alkyle en C₁ à C₈)silyle, aryl-di (alkyle en C₁ à C₈)silyle, diaryl-(alkyle en C₁ à C₈) - silyle, triarylsilyle, -COOR⁶, -CO-NR⁷R⁸, aryle en C₆ à C₁₄ et/ou hétéroaryle ayant un à trois hétéroatomes dans le noyau, ces restes pouvant eux-mêmes être substitués avec ou plusieurs restes choisis dans le groupe constitué d'halogéno, cyano, nitro, hydroxy, alkyle en C₁ à C₈, halogénalkyle en C₁ à C₈, alkoxy en C₁ à C₈ et halogénalkoxy en C₁ à C₈ ; ou bien
A est un reste aryle en C₆ à C₁₄, qui est éventuellement substitué avec un ou plusieurs restes, ces restes étant des restes halogéno, cyano, hydroxy, nitro, alkyle en C₁ à C₈, halogénalkyle en C₁ à C₈, alkoxy en C₁ à C₈, halogénalkoxy en C₁ à C₈, alkylthio en C₁ à C₈, halogénalkylthio en C₁ à C₈, alcényle en C₂ à C₈, halogénalcényle en C₂ à C₈, alcynyle en C₂ à C₈, halogénalcynyle en C₂ à C₈, alkyloxyalkylène en C₂ à C₈, alkylthioalkylène en C₂ à C₈, alcanoyloxyalkylène en C₃ à C₈, aminoalkylène en C₁ à C₈, phényloxyalkylène, phénylthioalkylène, cycloalkyle en C₃ à C₈, aryle en C₆ à C₁₄, hétéroaryle avec un à trois hétéroatomes dans le noyau, -COOR⁶, -CO-NR⁷R⁸ et/ou -S(O)ᵣ-R³, r et R³ possédant la définition indiquée plus haut, les restes aryle et/ou hétéroaryle pouvant eux-mêmes être substitués avec un ou plusieurs restes choisis dans le groupe constitué d'halogéno, cyano, nitro, hydroxy, alkyle en C₁ à C₈, halogénalkyle en C₁ à C₈, alkoxy en C₁ à C₈ et halogénalkoxy en C₁ à C₈; ou bien
A représente un reste hétérocyclique ayant un à trois hétéroatomes dans le noyau, qui est éventuellement substitué avec un ou plusieurs restes, ces restes étant des restes halogéno, cyano, hydroxy, nitro, alkyle en C₁ à C₈, halogénalkyle en C₁ à C₈, alkoxy en C₁ à C₈, halogénalkoxy en C₁ à C₈, alkylthio en C₁ à C₈, halogénalkylthio en C₁ à C₈, alcényle en C₂ à C₈, halogénalcényle en C₂ à C₈, alcynyle en C₂ à C₈, halogénalcynyle en C₂ à C₈, alkyloxyalkylène en C₂ à C₈, alkylthioalkylène en C₂ à C₈, alcanoyloxyalkylène en C₃ à C₈, aminoalkylène en C₁ à C₈, phényloxyalkylène, phénylthioalkylène, cycloalkyle en C₃ à C₈, aryle en C₈ à C₁₄, hétéroaryle ayant un à trois hétéroatomes dans le noyau, -COOR⁶, -CO-NR⁷R⁸ et/ou -S(O)ᵣ-R³, r et R³ possédant la définition indiquée plus haut, les restes aryle et/ou hétéroaryle pouvant eux-mêmes être substitués avec un ou plusieurs restes choisis dans le groupe constitué d'halogéno, cyano, nitro, hydroxy, alkyle en C₁ à C₈, halogénalkyle en C₁ à C₈, alkoxy en C₁ à C₈ et halogénalkoxy en C₁ à C₈ ; ou bien
A est un reste alkyle en C₁ à C₈ substitué par un à six groupes, dont les substituants sont choisis dans le groupe constitué d'hydroxy, amino, N- (alkyle en C₁ à C₈)amino, N, N-bis- (alkyle en C₁ à C₈) amino, alkoxy en C₁ à C₈, halogénalkoxy en C₁ à C₈, acyloxy en C₁ à C₈ et halogénacyloxy en C₁ à C₈, ou bien deux substituants pouvant alors former aussi conjointement une chaîne alkylène contenant un ou plusieurs atomes d'oxygène, d'azote et/ou de soufre, par exemple ils forment un système de noyau acétal, lactone ou lactame,
Z représente =O, =S, =N-R³³, =N-O-R³³ ou =N-NR³³R³⁴,
R⁶, R⁷, R⁸, R⁹, R³³ et R³⁴ représentent, indépendamment les uns des autres, l'hydrogène, un reste alkyle en C₁ à C₈, alcényle en C₂ à C₈, alcynyle en C₂ à C₈, cycloalkyle en C₃ à C₁₀, cycloalcényle en C₄ à C₁₀, cycloalcynyle en C₄ à C₁₀, aryle en C₆ à C₁₄ ou hétéroaryle avec un à trois hétéroatomes dans le noyau, qui peuvent eux-mêmes être substitués par un reste halogéno, hydroxy, cyano, nitro, alkyle en C₁ à C₈, alcényle en C₂ à C₈, alcynyle en C₂ à C₈, cycloalkyle en C₃ à C₁₀, cycloalcényle en C₄ à C₁₀, cycloalcynyle en C₄ à C₁₀, aryle en C₆ à C₁₄, aryle en C₆ à C₁₄ substitué par un halogène, hétéroaryle ayant un à trois hétéroatomes dans le noyau ou hétéroaryle substitué par un halogène ayant un à trois hétéroatomes dans le noyau, amino, N(alkyle en C₁ à C₈)amino, N,N-bis(alkyle en C₁ à C₈) amino, tri (alkyle en C₁ à C₈)silyle, aryl-di (alkyle en C₁ à C₈) silyle, diaryl- (alkyle en C₁ à C₈)silyle, triarylsilyle, alkoxy en C₁ à C₈ et/ou halogénalkoxy en C₁ à C₈,
B constitue un groupe de pontage divalent et représente un groupe alkylène ayant un à douze atomes de carbone, cycloalkylène ayant trois à quatorze atomes de carbone, alkylène-cycloalkylène ayant quatre à vingt-six atomes de carbone, alkylène-cycloalkylène-alkylène ayant cinq à trente-huit atomes de carbone, ces groupes de pontage pouvant présenter une à trois liaisons d'insaturation éthylénique et/ou pouvant être interrompus par des groupes -O-, -S-, -C(=O)O- ou -NR⁵-, R⁵ ayant la définition indiquée ci-dessus, et le groupe de pontage présentant éventuellement un à dix substituants qui sont choisis dans le groupe constitué d'alkyle en C₁ à C₃, trifluorométhyle ou trichlorométhyle, et
R⁴ représente un reste aryle monovalent en C₆ à C₁₄ ou un reste hétéroaryle contenant de l'azote ayant au moins un hétéroatome dans le noyau, qui est éventuellement substitué avec un à quatre restes choisis dans le groupe constitué d'halogéno, cyano, nitro, thiocyanato, isocyanato, alkyle en C₁ à C₈, alcényle en C₂ à C₈, alcynyle en C₂ à C₈, cycloalkyle en C₃ à C₁₀, cycloalcényle en C₄ à C₁₀, cycloalcynyle en C₄ à C₁₀, aryle en C₆ à C₁₄, hétéroaryle contenant de l'azote ayant un à trois hétéroatomes dans le noyau, ces substituants pouvant être eux-mêmes substitués avec des restes choisis dans le groupe constitué d'halogéno, cyano, nitro, alkyle en C₁ à C₈, halogénalkyle en C₁ à C₈, alkoxy en C₁ à C₈ et halogénalkoxy en C₁ à C₈, -C(=Z)-R⁶, -C (=Z) -OR⁶, -C(=Z)-SR⁶, -C(=Z)-NR⁷R⁸, -C(=Z) -O-N=C(NH₂) -R⁶, -O-C(Z=)-R⁶, -O-C(Z=)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z)-NR⁷R⁸, -NR⁹-C(=Z)-R⁶, -NR⁹-C(=Z)-OR⁶, -NR⁹-C(=Z)-SR⁶, -NR⁹-C(=Z)-NR⁷R⁸, -OR⁶, -SR⁶, -S(=O)R⁶, S(=O)₂R⁶, -NR⁷R⁸, où Z, R⁶, R⁷, R⁸ et R⁹ possèdent les définitions indiquées ci-dessus.

2. Composés de formule (I) suivant la revendication 1, **caractérisés en ce que** R¹ et R² représentent chacun le chlore ou le brome.

3. Composés de formule (I) suivant la revendication 1, **caractérisés en ce que** Y représente -O-.

4. Composés de formule (I) suivant la revendication 1, **caractérisés en ce que** R¹ et R² représentent chacun le chlore et Y représente -O-.

5. Composés de formule (I) suivant la revendication 1, **caractérisés en ce que** X représente -O-, -S- ou -NH- et X' représente une liaison C-C directe, -O-, -S- ou -NH-.

6. Composés de formule (I) suivant la revendication 5, **caractérisés en ce que** X et X' représentent chacun -O- ou bien X représente -O- et X' est une liaison C-C directe.

7. Composés de formule (I) suivant la revendication 1, **caractérisés en ce que** R³ représente l'hydrogène, le fluor, le chlore, le brome, un reste méthyle, trifluorométhyle, monochlorométhyle, dichlorométhyle, trichlorométhyle, méthoxy, trifluorométhoxy, monochlorométhoxy, dichlorométhoxy, trichlorométhoxy, nitro, cyano, cyclohexyle, phényle ou un reste monovalent dérivé de thiophène, furanne, pyrrole, thiazole, oxazole, imidazole, isothiazole, isoxazole, pyrazole, 1,3,4-oxadiazole, 1,3,4-thiadiazole, 1,3,4-triazole, 1,2,4-oxadiazole, 1,2,4-thiadiazole, 1,2,4-triazole, 1,2,3-triazole, 1,2,3,4-tétrazole, benzo [b] thiophène, benzo [b] furanne, indole, benzo [c] thiophène, benzo [c] furanne, isoindole, benzoxazole, benzothiazole, benzimidazole, benzisoxazole, benzisothiazole, benzopyrazole, benzothiadiazole, benzotriazole, dibenzofuranne, dibenzothiophène, carbazole, pyridine, pyrazine, pyrimidine, pyridazine, 1,3,5-triazine, 1,2,4-triazine, 1,2,4,5-tétrazine, quinoléine, isoquinoléine, quinoxaline, quinazoline, cinnoline, 1,8-naphtyridine, 1,5-naphtyridine, 1,6-naphtyridine, 1,7-naphtyridine, phtalazine, pyridopyrimidine, purine, ptéridine, 4H-quinolizine, pipéridine, pyrrolidine, oxazoline, tétrahydrofuranne, tétrahydropyranne, isoxazolidine ou thiazolidine.

8. Composés de formule (I) suivant la revendication 1, **caractérisés en ce que** R⁸ représente un reste méthyle, trifluorométhyle, cyano, chloro ou bromo.

9. Composés de formule (I) suivant la revendication 1, **caractérisés en ce que** Z représente =O, =N-OH, =N-OCH₃ ou =N-CH₂-CH=CH₂.

10. Composés de formule (I) suivant la revendication 1, **caractérisés en ce que** A représente un groupe de formule -C(=O)-CH₃, -C(=N-OCH₃) -CH₃, -C (=N-OH) -CH₃, -C (=O) -OCH₃, -C(=O)-CH=CH-N(CH₃)₂, -C(=O)-NH-C₂H₅, -C(=O)-NH₂, -C(=O)-N(CH₃)₂, -C(=O)-NH-CH₂-CH₂-OH, -C(=O)-NH-CH (CH₃)-CH₂-OH, -C(=O)-CHBr₂, -C(=O)-N(CH₃)-O-CH₃, -C(=N-O-CH₂-CH=CH₂)-CH₃, -C(=S)-NH₂, -C(=O)-O-C₆H₅, -C(=O)-O-C₃H₇, -C(=O)-imidazolyle, -C(=O)-3-éthyl-1,2,4-oxadiazole-5-yle, -C(=O)-2-Δ-oxazoline-2-yle, -C(=O)-1-méthyl-pyrazole-3-yle, -C(=O)-éthyn-2-yle, -C(=O)-1-hydroxy-éthyn-2-yle, -C(=O)-1-triméthylsilyl-éthyn-2-yle, -C(=O)-1-hexyl-éthyn-2-yle, -C(=O)-1-(2-chlorophényl)-éthyn-2-le, -C(=O)-1-méthoxy-éthyn-2-yle et -C(=O)-éthén-2-yle.

11. Composés de formule (I) suivant la revendication 1, **caractérisés en ce que** A représente des restes alcényle en C₂ à C₈, alcynyle en C₂ à C₈, phényle, pyridyle ou pyrimidyle, qui sont éventuellement substitués par un ou plusieurs atomes d'halogène, groupes cyano, groupes nitro, groupes hydroxyle, restes alkoxy en C₁ à C₃ ou restes halogénalkoxy en C₁ à C₃, restes alkoxy en C₁ à C₃, restes halogénalkoxy en C₁ à C₃, qui sont eux-mêmes éventuellement substitués par un ou plusieurs atomes d'halogènes, groupes cyano, groupes nitro, groupes hydroxyle, restes alkoxy en C1 à C3 ou restes halogénalkoxy en C₁ à C₃, ou bien il représente un groupe de formule -OR⁶, -SR⁶, -NR⁷R⁸, -S(=O)R⁶, -S(=O)₂R⁶, -C(=Z)-R⁶, -C(=Z)-OR⁶, -C(=Z)-SR⁶, -C(=Z)-NR⁷R⁸, -O-C(=Z)-R⁶, -O-C(=Z)-OR⁶, -O-C(=Z)-SR⁶, -O-C(=Z)-NR⁷R⁸, -S-C(=Z)-R⁶, -S-C(=Z)-OR⁶, -S-C(=Z)-SR⁶, -S-C(=Z) -NR⁷R⁸, -NR⁹-C(=Z) -R⁶, -NR⁹-C(=Z)-OR⁶, -NR⁹-C(=Z)-SR⁶ ou -NR⁹-C(=Z)-NR⁷R⁸, où Z, R⁶, R⁷, R⁸ et R⁹ ont les définitions indiquées dans la revendication 1, ou un reste alkyle en C₁ à C₈ substitué par un ou plusieurs groupes hydroxyle, restes alkoxy en C₁ à C₃, restes halogénalkoxy en C₁ à C₃, restes acyloxy en C₁ à C₃ ou restes halogénacyloxy en C₁ à C₃, ou représente un groupe parmi les formules Q¹-Q¹⁰ 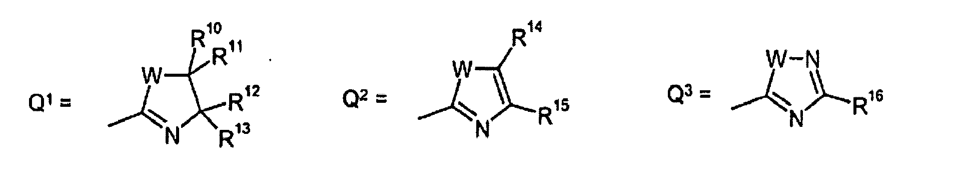 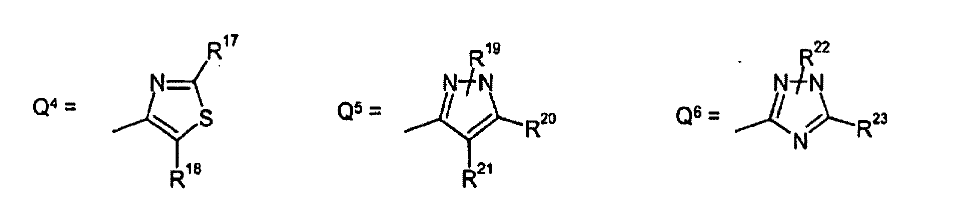 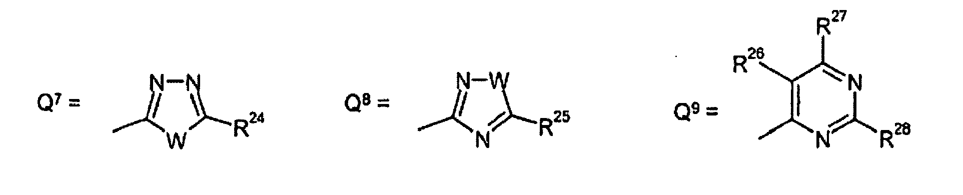 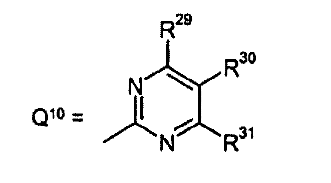 où W représente l'oxygène ou le soufre, R¹⁰ à R¹⁸, R²⁰ et R²¹, R²³ à R³¹ représentent l'hydrogène, un reste alkyle en C₁ à C₆, cycloalkyle en C₃ à C₇, alcényle en C₂ à C₆, cycloalcényle en C₄ à C₇, pyridyle ou phényle, les restes alkyle en C₁ à C₆, cycloalkyle en C₃ à C₇, alcényle en C₂ à C₆ ou cycloalcényle en C₄ à C₇ étant éventuellement substitués une ou plusieurs fois, avantageusement une, deux ou trois fois par des substituants halogéno, cyano, -OR⁶, -SR⁶, -S(O)R⁶, -S(O)₂R⁶ et/ou -NR⁷R⁸, les restes R⁶, R⁷ et R⁸ ayant la définition indiquée ci-dessus, et les restes phényle ou pyridiyle étant éventuellement substitués une ou plusieurs fois, avantageusement une, deux ou trois fois par des substituants halogéno, cyano, nitro, -OR⁶, -SR⁶, -S(O)R⁶, -S(O)₂R⁶, -NR⁷R⁸, alkyle en C₁ à C₄, halogénalkyle en C₁ à C₄, alcényle en C₂ à C₄ et/ou halogénalkyle en C₂ à C₄.

12. Composés de formule (I) suivant la revendication 1, **caractérisés en ce que** B est un groupe de formules P¹ à P⁶ 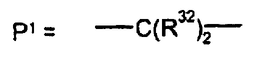 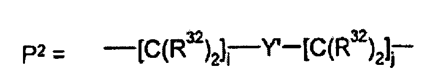 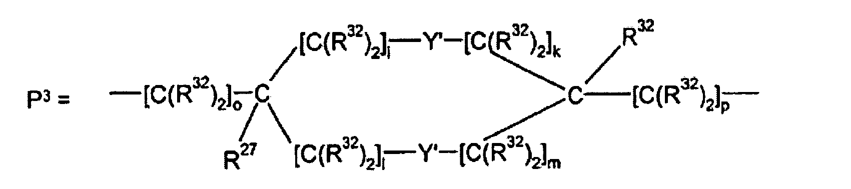 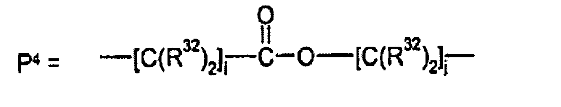 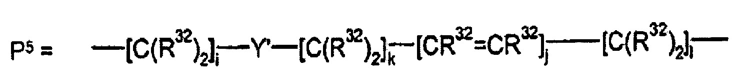 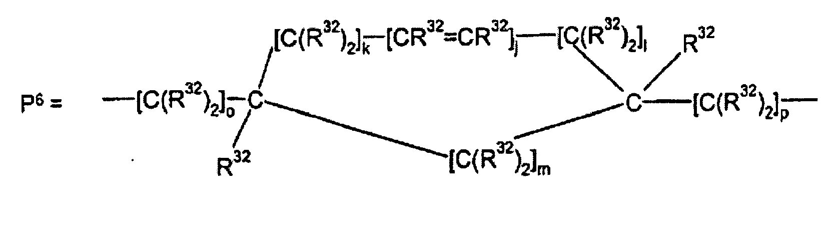 où R³² désigne, indépendamment dans chaque cas, l'hydrogène, un reste alkyle en C₁ à C₃ ou trifluorométhyle,
Y' désigne, indépendamment dans chaque cas, une liaison C-C directe, -O-, -S- ou -NH-,
i est un nombre entier de 1 à 6,
j est un nombre entier de 1 à 6,
k est un nombre entier de 0 à 2,
l est un nombre entier de 0 à 2,
m est un nombre entier de 0 à 2,
o est un nombre entier de 0 à 1, et
p est un nombre entier de 0 à 1.

13. Composés de formule (I) suivant la revendication 1, **caractérisés en ce que** B est un groupe de formule -C_{q}H_{2q}-, et q est un nombre entier de 2 à 4, en particulier un groupe - (CH₂)₂-, - (CH₂)₃- et -(CH₂)₄-.

14. Composés de formule (I) suivant la revendication 1, **caractérisés en ce que** R⁴ représente un reste aryle en C₆ à C₁₄ qui est éventuellement substitué par un à trois restes choisis dans le groupe constitué des restes halogéno, cyano, nitro, thiocyanato, isocyanato, alkyle en C₁ à C₆, cycloalkyle en C₃ à C₈, alcényle en C₂ à C₈, cycloalcényle en C₄ à C₈ et alcynyle en C₂ à C₈, un ou plusieurs atomes d'hydrogène des restes alkyle en C₁ à C₆, cycloalkyle en C₃ à C₈, alcényle en C₂ à C₈, cycloalcényle en C₄ à C₈ et alcynyle en C₂ à C₈ pouvant être substitués par un halogène et/ou un radical cyano.

15. Composés de formule (I) suivant la revendication 1, **caractérisés en ce que** R⁴ est choisi dans le groupe constitué des restes phényle, pyridyle, pyrimidyle, pyrazinyle, pyrazolyle ou naphtyle, qui sont éventuellement substitués par un ou plusieurs restes choisis dans le groupe constitué d'halogéno, cyano, nitro, alkyle en C₁ à C₃, halogénalkyle en C₁ à C₃, alkoxy en C₁ à C₃, halogénalkoxy en C₁ à C₃, -C (=Z) -R⁶, -C (=Z) -OR⁶, -C (=Z) -SR⁶, -C (=Z) -NR⁷R⁸, -O-C(=Z) -R⁶, -O-C(=Z)-OR⁶ , -O-C(=Z)-SR⁶, -O-C(=Z)-NR⁷R⁸, -S-C(=Z)-R⁶, -S-C(=Z)-OR⁶, -S-C(=Z)-SR⁶,-S-C(=Z)-NR⁷R⁸, -NR⁹-C(=Z)-R⁶, -NR⁹-C(=Z)-OR⁶, -NR⁹- C(=Z)-SR⁶, -NR⁹-C(=Z)-NR⁷R⁸, -OR⁶, -SR⁶, -S(=O)R⁶ et -S(=O)₂R⁶, Z , R⁶, R⁷, R⁸ et R⁹ ayant les définitions indiquées dans la revendication 1.

16. Composés de formule (I) suivant la revendication 1, **caractérisés en ce que** R⁴ est un reste phényle ou pyridyle qui porte un à trois substituants du groupe des substituants halogéno, cyano, nitro, alkyle en C₁ à C₃ et halogénalkyle en C₁ à C₃ ou des combinaisons de ces substituants.

17. Composés de formule (I) suivant la revendication 1, **caractérisés en ce que** R⁴ désigne un reste trifluorométhylphényle, chlorophényle, nitrophényle, nitropyridyle, trifluorométhylpyridyle, di-trifluorométhylpyridyle, chloropyridyle, dichloropyridyle, chlorotrifluorométhylpyridyle, trifluorométhylpyridimidyle, di-trifluorométhylpyrimidyle, méthyltrifluorométhylpyrimidyle ou trifluorométhylpyrazolyle.

18. Procédé de production de composés de formule générale (I) suivant la revendication 1, comprenant les étapes: 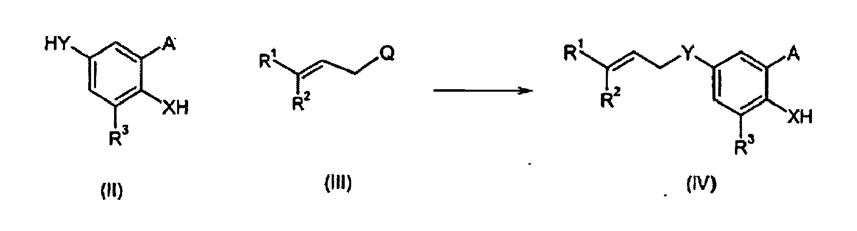 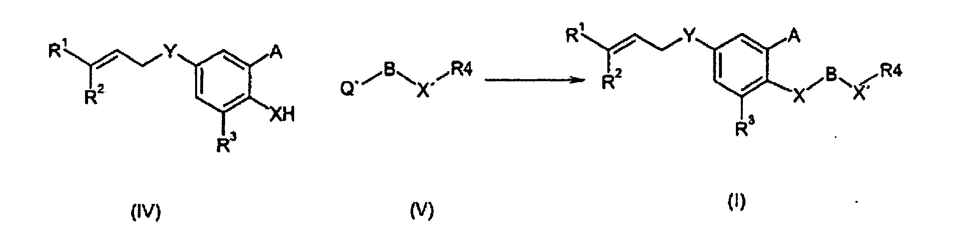
a) réaction des composés de formules II et III pour former le composé de formule IV, et
b) réaction des composés de formules IV et V pour former le composé de formule I,
R¹, R², R³, R⁴, A, Y, X et X' ayant les définitions indiquées dans la revendication 1 et Q et Q' représentant des groupes partants.

19. Composition douée d'action insecticide, acaricide, ixodicide, nématicide, mollusquicide et/ou fongicide, **caractérisée par** une teneur en au moins un composé de formule (I) ou en un de ses sels selon la revendication 1.

20. Composition suivant la revendication 19, en mélange avec des supports et/ou des substances tensioactives.

21. Utilisation des composés de formule (I) suivant la revendication 1, pour la lutte contre des parasites animaux.

22. Utilisation des composés de formule (I) suivant la revendication 1, pour la lutte contre des organismes nuisibles dans des plantes cultivées transgéniques.

23. Utilisation des composés de formule (I) suivant la revendication 1, pour protéger des plantes contre des parasites et/ou des nuisances ou pour les en débarrasser.

24. Utilisation des composés de formule (I) suivant la revendication 1, pour la préparation d'un médicament, en particulier d'un médicament à usage vétérinaire.

25. Utilisation des composés de formule (I) suivant la revendication 1, pour la préparation d'un médicament destiné à combattre des endoparasites et des ectoparasites.

26. Procédé de lutte contre des parasites animaux, dans lequel on met les parasites animaux directement ou indirectement en contact avec un composé de formule (I) suivant la revendication 1.

27. Procédé pour combattre des organismes nuisibles dans des plantes cultivées transgéniques, dans lequel on met les organismes nuisibles directement ou indirectement en contact avec un composé de formule (I) suivant la revendication 1.
